# EUROPEAN PATENT APPLICATION

(11) **EP 4 474 910 A1**
(43) Date of publication of application: **11.12.2024**
(21) Application number: 24179697.8
(22) Date of filing: 03.06.2024
(51) Int. Cl.: G03F 7/004, C08F 220/24, C08F 220/38, G03F 7/038

(54) **MONOMER, POLYMER, CHEMICALLY AMPLIFIED NEGATIVE RESIST COMPOSITION, AND PATTERN FORMING PROCESS**

(30) Priority: 06.06.2023 JP 2023093234
(71) Applicant: SHIN-ETSU CHEMICAL CO., LTD., Tokyo 1000005 (JP)
(72) Inventor: FUKUSHIMA, Masahiro, NIIGATA-KEN (JP); WATANABE, Satoshi, NIIGATA-KEN (JP); MASUNAGA, Keiichi, NIIGATA-KEN (JP); FUNATSU, Kenji, NIIGATA-KEN (JP); KOTAKE, Masaaki, NIIGATA-KEN (JP); MATSUZAWA, Yuta, NIIGATA-KEN (JP)
(74) Representative: Ter Meer Steinmeister & Partner

(57) **Abstract**

A chemically amplified negative resist composition comprising a polymer comprising repeat units derived from a monomer having a dehydrating functional group is provided. The resist composition exhibits a high resolution during pattern formation and forms a pattern with improved LER, fidelity, and dose margin.

## Description

### TECHNICAL FIELD

This invention relates to a monomer, polymer, chemically amplified negative resist composition, and pattern forming process.

### BACKGROUND ART

To meet the demand for higher integration density and operating speed of LSIs, the effort to reduce the pattern rule is in rapid progress. Acid-catalyzed chemically amplified resist compositions are most often used in forming resist patterns with a feature size of 0.2 µm or less. High-energy radiation such as UV, deep-UV or EB is used as the light source for exposure of these resist compositions. In particular, the EB lithography, which is utilized as the ultra-fine microfabrication technique, is also indispensable in processing photomask blanks to form photomasks for use in semiconductor device fabrication.

Polymers comprising a major proportion of aromatic structure having an acidic side chain, for example, polyhydroxystyrene are useful in resist materials for the KrF excimer laser lithography. These polymers are not used in resist materials for the ArF excimer laser lithography because they exhibit strong absorption at a wavelength of around 200 nm. These polymers, however, are expected to form useful resist materials for the EB and EUV lithography for forming patterns of smaller size than the processing limit of ArF excimer laser because they offer high etching resistance.

Resist compositions for photolithography include positive ones in which exposed areas are dissolved away and negative ones in which exposed areas are left as a pattern. A viable composition is selected among them depending on the desired resist pattern. In general, the chemically amplified negative resist composition comprises a polymer which is normally soluble in an aqueous alkaline developer, an acid generator which is decomposed to generate an acid upon exposure to light, and a crosslinker which causes the polymer to crosslink in the presence of the acid serving as a catalyst, thus rendering the polymer insoluble in the developer (sometimes, the crosslinker is incorporated in the polymer). Most often a quencher is added for controlling the diffusion of the acid generated upon light exposure.

Typical of the alkali-soluble units to constitute polymers which dissolve in aqueous alkaline developer are units derived from phenols. A number of negative resist compositions of such type were developed, especially as adapted for exposure to KrF excimer laser light. These compositions have not been used in the ArF excimer laser lithography because the phenolic units are not transmissive to exposure light having a wavelength of 150 to 220 nm. Recently, these compositions are recognized attractive again as the negative resist composition for the short wavelength (e.g., EB or EUV) lithography capable of forming smaller size patterns. Exemplary compositions are described in Patent Documents 1 to 3.

Besides the above-mentioned compositions, many other chemically amplified negative resist compositions have been developed. These negative working resist compositions use a crosslinker for insolubilizing the alkali-soluble polymer under the action of an acid generated upon exposure to high-energy radiation. Many crosslinkers including those disclosed in Patent Documents 4 and 5 have been developed. On the other hand, an attempt has been made to endow the polymer with the function of crosslinker. For example, it was proposed to introduce styrene units having an alkoxymethoxy group substituted thereon (Patent Document 6), repeat units having an alkoxymethylamino group (Patent Document 7), repeat units having an epoxy group (Patent Document 8), repeat units of styrene having an acid-eliminatable group (Patent Document 9), repeat units of adamantyl having an acid-eliminatable hydroxy group (Patent Document 10), and repeat units of aliphatic hydrocarbon and alicyclic hydrocarbon having an acid-eliminatable hydroxy group (Patent Documents 11 to 15).

Although these methods are successful in improving resist properties to some extent, satisfactory results are not yet obtained. In view of the current requirement for further miniaturization, it is desired to have a chemically amplified negative resist composition having a high sensitivity, satisfactory maximum resolution, and high contrast.

### Citation List

Patent Document 1: JP 4396849
Patent Document 2: JP 4478589
Patent Document 3: JP 4678383
Patent Document 4: JP 4955732
Patent Document 5: JP 4801190
Patent Document 6: JP-A H05-232702
Patent Document 7: JP-A H08-202037
Patent Document 8: JP 3914363
Patent Document 9: JP-A 2003-337414
Patent Document 10: JP 4648526
Patent Document 11: USP 7,300,739
Patent Document 12: USP 7,393,624
Patent Document 13: USP 7,563,558
Patent Document 14: JP-A 2013-164588 (USP 9,244,348, EP 2626743)
Patent Document 15: JP-A 2019-203103

### DISCLOSURE OF INVENTION

An object of the invention is to provide a chemically amplified negative resist composition which is improved in resolution during pattern formation and forms a resist pattern having improved LER, fidelity, and dose margin.

The inventors have found that when a chemically amplified negative resist composition having added thereto a polymer comprising repeat units derived from a monomer having a dehydrating functional group is processed by photolithography, a pattern of good profile having a satisfactory resolution, reduced LER, fidelity, and improved dose margin can be formed.

Accordingly, the invention provides a monomer, polymer, chemically amplified negative resist composition, and pattern forming process, as defined below.
1. A monomer having the formula (A1): 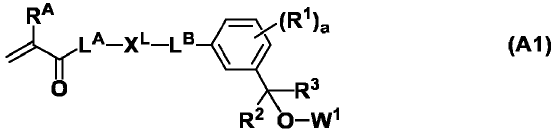 wherein a is an integer of 0 to 4,
   R^{A} is hydrogen, fluorine, methyl or trifluoromethyl,
   L^{A} is -O- or -NH-,
   L^{B} is a single bond, ether bond, ester bond or amide bond,
   X^{L} is a single bond or a C₁-C₄₀ hydrocarbylene group which may contain a heteroatom,
   R¹ is halogen or a C₁-C₂₀ hydrocarbyl group which may contain a heteroatom,
   R² and R³ are each independently hydrogen, a C₁-C₁₅ saturated hydrocarbyl group or C₆-C₁₅ aryl group, the hydrocarbyl group may be substituted with a hydroxy moiety or C₁-C₆ saturated hydrocarbyloxy moiety, the aryl group may have a substituent, with the proviso that R² and R³ are not hydrogen at the same time, and R² and R³ may bond together to form a ring with the carbon atom to which they are attached, some constituent -CH₂- in the ring may be replaced by -O- or -S-, and
   W¹ is hydrogen, a C₁-C₁₀ aliphatic hydrocarbyl group, C₂-C₁₀ aliphatic hydrocarbylcarbonyl group, or C₆-C₁₅ aryl group, the aryl group may have a substituent.
2. The monomer of 1 having the formula (A1-1): 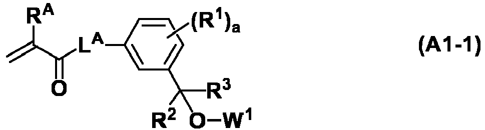 wherein a, R^{A}, L^{A}, R¹, R², R³, and W¹ are as defined above.
3. The monomer of 2 having the formula (A1-2): 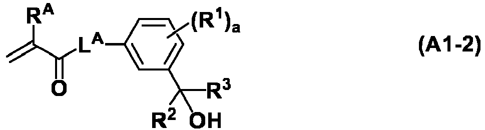 wherein a, R^{A}, L^{A}, R¹, R², and R³ are as defined above.
4. A polymer comprising repeat units derived from the monomer of any one of 1 to 3.
5. The polymer of 4, further comprising repeat units having the formula (A2): 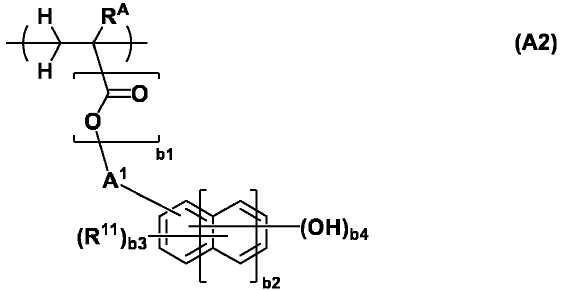 wherein b1 is 0 or 1, b2 is an integer of 0 to 2, b3 is an integer satisfying 0 ≤ b3 ≤ 5+2(b2)-b4, b4 is an integer of 1 to 3,
   R^{A} is hydrogen, fluorine, methyl or trifluoromethyl,
   R¹¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, and
   A¹ is a single bond or C₁-C₁₀ saturated hydrocarbylene group in which some constituent -CH₂- may be replaced by -O-.
6. The polymer of 4 or 5, further comprising repeat units of at least one type selected from repeat units having the formula (A3), repeat units having the formula (A4), and repeat units having the formula (AS): 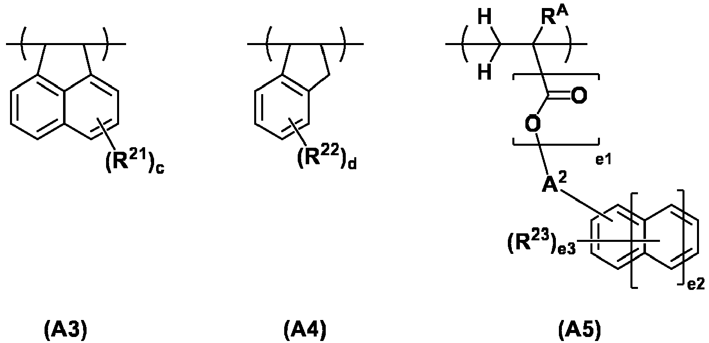 wherein c and d are each independently an integer of 0 to 4, e1 is 0 or 1, e2 is an integer of 0 to 2, e3 is an integer of 0 to 5,
   R^{A} is hydrogen, fluorine, methyl or trifluoromethyl,
   R²¹ and R²² are each independently hydroxy, halogen, an optionally halogenated C₁-C₈ saturated hydrocarbyl group, optionally halogenated C₁-C₈ saturated hydrocarbyloxy group, or optionally halogenated C₂-C₈ saturated hydrocarbylcarbonyloxy group,
   R²³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro, cyano, C₁-C₂₀ saturated hydrocarbylsulfinyl group, or C₁-C₂₀ saturated hydrocarbylsulfonyl group,
   A² is a single bond or C₁-C₁₀ saturated hydrocarbylene group in which some constituent -CH₂- may be replaced by -O-.
7. The polymer of any one of 4 to 6, further comprising repeat units of at least one type selected from repeat units having the formulae (A6) to (A13): 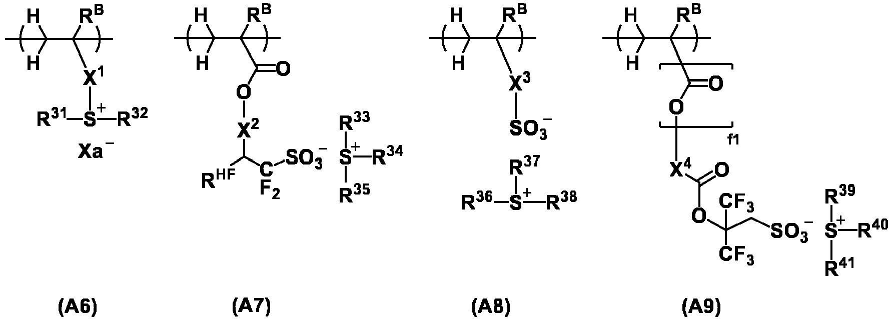 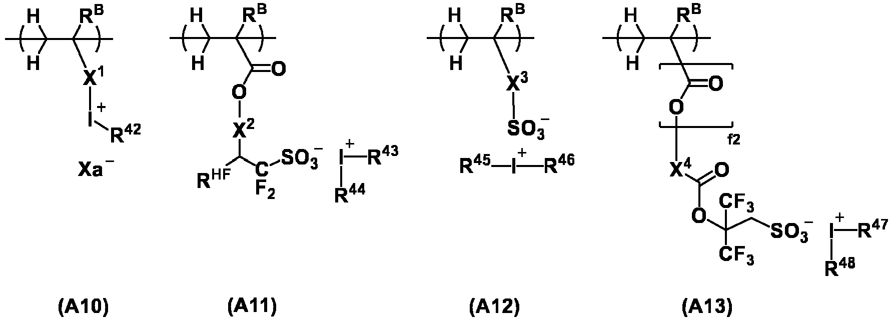 wherein R^{B} is hydrogen or methyl,
   X¹ is each independently a single bond, C₁-C₆ aliphatic hydrocarbylene group, phenylene, naphthylene, or C₇-C₁₈ group obtained by combining the foregoing, -O-X¹¹-, -C(=O)-O-X¹¹-, or -C(=O)-NH-X¹¹-, X¹¹ is a C₁-C₆ aliphatic hydrocarbylene group, phenylene, naphthylene, or C₇-C₁₈ group obtained by combining the foregoing, which may contain a carbonyl moiety, ester bond, ether bond or hydroxy moiety,
   X² is each independently a single bond or -X²¹-C(=O)-O-, X²¹ is a C₁-C₂₀ hydrocarbylene group which may contain a heteroatom,
   X³ is each independently a single bond, methylene, ethylene, phenylene, fluorinated phenylene, trifluoromethyl-substituted phenylene, -O-X³¹-, -C(=O)-O-X³¹-, or -C(=O)-NH-X³¹-, X³¹ is a C₁-C₆ aliphatic hydrocarbylene group, phenylene, fluorinated phenylene, trifluoromethyl-substituted phenylene, or C₇-C₂₀ group obtained by combining the foregoing, which may contain a carbonyl moiety, ester bond, ether bond or hydroxy moiety,
   X⁴ is each independently a single bond or C₁-C₃₀ hydrocarbylene group which may contain a heteroatom,
   f1 and f2 are each independently 0 or 1, f1 and f2 are 0 when X⁴ is a single bond,
   R³¹ to R⁴⁸ are each independently halogen, or C₁-C₂₀ hydrocarbyl group which may contain a heteroatom, R³¹ and R³² may bond together to form a ring with the sulfur atom to which they are attached, R³³ and R³⁴, R³⁶ and R³⁷, or R³⁹ and R⁴⁰ may bond together to form a ring with the sulfur atom to which they are attached,
   R^{HF} is hydrogen or trifluoromethyl, and
   Xa⁻ is a non-nucleophilic counter ion.
8. A chemically amplified negative resist composition comprising (A) a base polymer containing the polymer of any one of 4 to 7.
9. The negative resist composition of 8 wherein repeat units having an aromatic skeleton account for at least 60 mol% of the overall repeat units of the polymer in the base polymer.
10. The negative resist composition of 8 or 9, further comprising (B) an acid generator.
11. The negative resist composition of any one of 8 to 10, further comprising (C) a crosslinker.
12. The negative resist composition of any one of 8 to 10, which is free of a crosslinker.
13. The negative resist composition of any one of 8 to 12, further comprising (D) a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6): 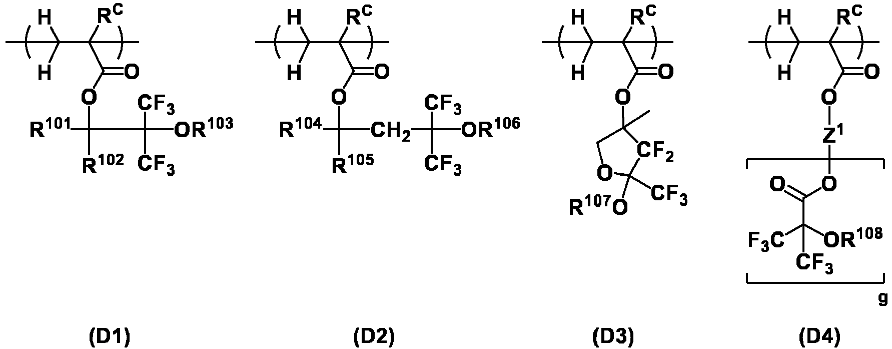 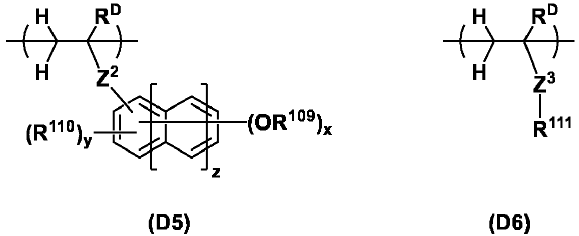 wherein x is an integer of 1 to 3, y is an integer satisfying 0 ≤ y ≤ 5+2z-x, z is 0 or 1, g is an integer of 1 to 3,
   R^{C} is each independently hydrogen, fluorine, methyl or trifluoromethyl,
   R^{D} is each independently hydrogen or methyl,
   R¹⁰¹, R¹⁰², R¹⁰⁴ and R¹⁰⁵ are each independently hydrogen or a C₁-C₁₀ saturated hydrocarbyl group,
   R¹⁰³, R¹⁰⁶, R¹⁰⁷ and R¹⁰⁸ are each independently hydrogen, a C₁-C₁₅ hydrocarbyl group, C₁-C₁₅ fluorinated hydrocarbyl group, or acid labile group, and when R¹⁰³, R¹⁰⁶, R¹⁰⁷ and R¹⁰⁸ each are a hydrocarbyl or fluorinated hydrocarbyl group, an ether bond or carbonyl moiety may intervene in a carbon-carbon bond,
   R¹⁰⁹ is hydrogen or a C₁-C₅ straight or branched hydrocarbyl group in which a heteroatom-containing moiety may intervene in a carbon-carbon bond,
   R¹¹⁰ is a C₁-C₅ straight or branched hydrocarbyl group in which a heteroatom-containing moiety may intervene in a carbon-carbon bond,
   R¹¹¹ is a C₁-C₂₀ saturated hydrocarbyl group in which at least one hydrogen is substituted by fluorine, and in which some constituent -CH₂- may be replaced by an ester bond or ether bond,
   Z¹ is a C₁-C₂₀ (g+1)-valent hydrocarbon group or C₁-C₂₀ (g+1)-valent fluorinated hydrocarbon group,
   Z² is a single bond, *-C(=O)-O- or *-C(=O)-NH-,
   Z³ is a single bond, -O-, *-C(=O)-O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.
14. The negative resist composition of any one of 8 to 13, further comprising (E) a quencher.
15. The negative resist composition of any one of 8 to 14, further comprising (F) an organic solvent.
16. A resist pattern forming process comprising the steps of:
   applying the chemically amplified negative resist composition of any one of 8 to 15, onto a substrate to form a resist film thereon,
   exposing the resist film patternwise to high-energy radiation, and
   developing the exposed resist film in an alkaline developer.
17. The process of 16 wherein the high-energy radiation is EUV or EB.
18. The process of 16 or 17 wherein the substrate has the outermost surface of a material containing at least one element selected from chromium, silicon, tantalum, molybdenum, cobalt, nickel, tungsten, and tin.
19. The process of any one of 16 to 18, wherein the substrate is a mask blank of transmission or reflection type.
20. A mask blank of transmission or reflection type which is coated with the chemically amplified negative resist composition of any one of 8 to 15.

### ADVANTAGEOUS EFFECTS OF INVENTION

When a chemically amplified negative resist composition having added thereto a polymer comprising repeat units derived from the inventive monomer is processed by photolithography, a polarity switch accompanying dehydrating reaction takes place at a satisfactory sensitivity, whereby a pattern of good profile having a high resolution, satisfactory fidelity, reduced LER, and improved dose margin can be formed. The resist composition and the pattern forming process are advantageously applicable to the micropatterning technology, especially EUV and EB lithography processes.

### DESCRIPTION OF EMBODIMENTS

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group. In chemical formulae, Me stands for methyl, Et for ethyl, and Ac for acetyl. Both the broken line (---) and the asterisk (*) designate a point of attachment or valence bond. As used herein, the term "fluorinated" refers to a fluorine-substituted or fluorine-containing compound or group. The terms "group" and "moiety" are interchangeable.

The abbreviations and acronyms have the following meaning.
EB: electron beam
EUV: extreme ultraviolet
Mw: weight average molecular weight
Mn: number average molecular weight
Mw/Mn: molecular weight distribution or dispersity
GPC: gel permeation chromatography
PEB: post-exposure bake
PAG: photoacid generator
LER: line edge roughness

It is understood that for some structures represented by chemical formulae, there can exist enantiomers and diastereomers because of the presence of asymmetric carbon atoms. In such a case, a single formula collectively represents all such isomers. The isomers may be used alone or in admixture.

### Monomer

One embodiment of the invention is a monomer having the formula (A1).

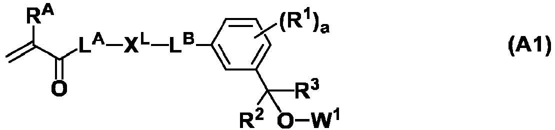

In formula (A1), "a" is an integer of 0 to 4, preferably 0 or 1.

In formula (A1), R^{A} is hydrogen, fluorine, methyl or trifluoromethyl, with hydrogen and methyl being preferred.

In formula (A1), L^{A} is -O- or -NH-, with -O-being preferred.

In formula (A1), L^{B} is a single bond, ether bond, ester bond or amide bond, with a single bond, ether bond or ester bond being preferred.

In formula (A1), X^{L} is a single bond or a C₁-C₄₀ hydrocarbylene group which may contain a heteroatom. The hydrocarbylene group may be straight, branched or cyclic and examples thereof include C₁-C₄₀ alkanediyl groups and C₃-C₄₀ cyclic saturated hydrocarbylene groups. Suitable heteroatoms include oxygen, nitrogen and sulfur atoms.

Examples of the optionally heteroatom-containing C₁-C₄₀ hydrocarbylene group X^{L} are shown below, but not limited thereto. Herein * designates a point of attachment to L^{A} or L^{B}.

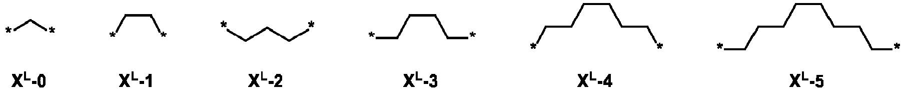

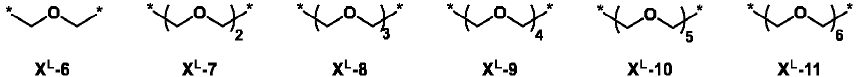

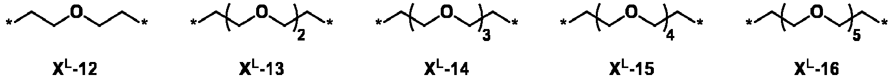

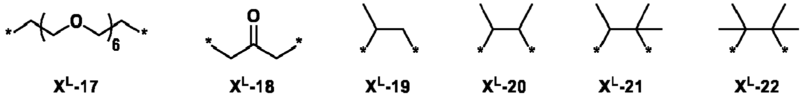

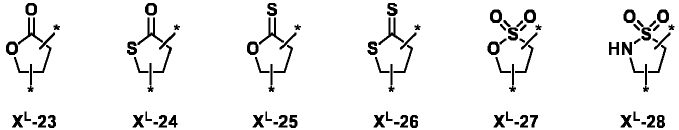

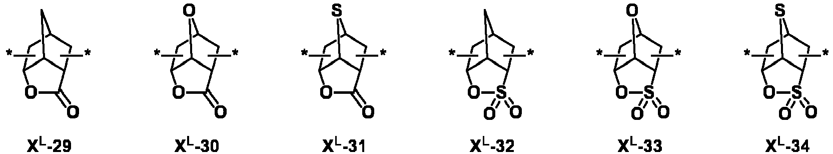

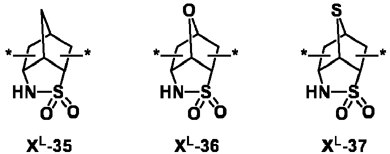

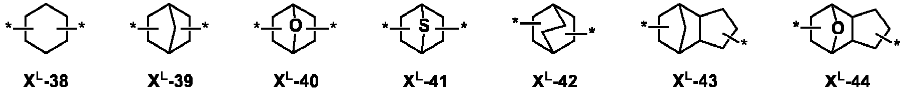

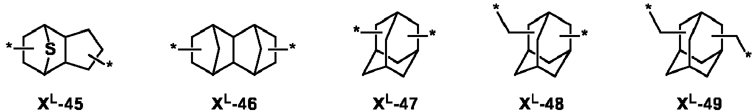

Of these, X^{L}-0 to X^{L}-22 and X^{L}-47 to X^{L}-49 are preferred, with X^{L}-0 to X^{L}-17 being more preferred.

In formula (A1), R¹ is halogen or a C₁-C₂₀ hydrocarbyl group which may contain a heteroatom.

Suitable halogen atoms include fluorine, chlorine, bromine, and iodine.

The C₁-C₂₀ hydrocarbyl group R¹ may be saturated or unsaturated and straight, branched or cyclic. Examples thereof include C₁-C₂₀ alkyl groups such as methyl, ethyl, propyl, isopropyl, n-butyl, isobutyl, sec-butyl, tert-butyl, n-pentyl, tert-pentyl, n-hexyl, n-octyl, 2-ethylhexyl, n-nonyl, and n-decyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; C₆-C₂₀ aryl groups such as phenyl, naphthyl and anthryl, and combinations thereof. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, fluorine, chlorine, bromine, iodine, cyano moiety, carbonyl moiety, ether bond, ester bond, sulfonate ester bond, carbonate bond, carbamate bond, amide bond, imide bond, lactone ring, sultone ring, thiolactone ring, lactam ring, sultam ring, carboxylic anhydride (-C(=O)-O-C(=O)-) or haloalkyl moiety.

In formula (A1), R² and R³ are each independently hydrogen, a C₁-C₁₅ saturated hydrocarbyl group or C₆-C₁₅ aryl group. The hydrocarbyl group may be substituted with a hydroxy moiety or C₁-C₆ saturated hydrocarbyloxy moiety. The aryl group may have a substituent.

The C₁-C₁₅ saturated hydrocarbyl group represented by R² and R³ may be straight, branched or cyclic. Examples thereof include C₁-C₁₅ alkyl groups such as methyl, ethyl, propyl, isopropyl, n-butyl, sec-butyl, tert-butyl, n-pentyl, tert-pentyl, n-hexyl, 2-ethylhexyl, n-octyl, n-nonyl, and n-decyl; and C₃-C₁₅ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, and adamantyl.

Examples of the C₆-C₁₅ aryl group represented by R² and R³ include phenyl, naphthyl and anthryl, with phenyl being preferred. The aryl group may have a substituent, examples of which include halogens, optionally halogenated C₁-C₆ saturated hydrocarbyl moieties, and optionally halogenated C₁-C₆ saturated hydrocarbyloxy moieties.

R² and R³ are not hydrogen at the same time. When one of R² and R³ is an optionally substituted aryl group, the other is preferably hydrogen.

R² and R³ are preferably identical, most preferably both methyl.

R² and R³ may bond together to form a ring with the carbon atom to which they are attached. Some constituent -CH₂- in the ring may be replaced by -O- or -S-. Examples of the ring include cyclopropane, cyclobutane, cyclopentane, cyclohexane, norbornane, adamantane, tricyclo[5.2.1.0^{2,6}]decane, tetracyclo[6.2.1.1^{1,6}.0^{2,7}], oxanorbornene, and thianorbornane rings.

In formula (A1), W¹ is hydrogen, a C₁-C₁₀ aliphatic hydrocarbyl group, C₂-C₁₀ aliphatic hydrocarbylcarbonyl group, or C₆-C₁₅ aryl group. The aryl group may have a substituent.

The C₁-C₁₀ aliphatic hydrocarbyl group represented by W¹ may be saturated or unsaturated and straight, branched or cyclic. Examples thereof include C₁-C₁₀ alkyl groups such as methyl, ethyl, n-propyl, isopropyl, n-butyl, isobutyl, sec-butyl, tert-butyl, n-pentyl, neopentyl, n-hexyl, n-heptyl, n-octyl, 2-ethylhexyl, n-nonyl, and n-decyl; C₃-C₁₀ cyclic saturated hydrocarbyl groups such as cyclopentyl and cyclohexyl; C₂-C₁₀ alkenyl groups such as vinyl, 1-propenyl, 2-propenyl, butenyl, and hexenyl; and C₃-C₁₀ cyclic unsaturated hydrocarbyl groups such as cyclohexenyl. Examples of the C₆-C₁₅ aryl group W¹ include phenyl, naphthyl and anthryl, with phenyl being preferred. The aryl group may have a substituent, examples of which include halogens, optionally halogenated C₁-C₆ saturated hydrocarbyl moieties, and optionally halogenated C₁-C₆ saturated hydrocarbyloxy moieties.

W¹ is preferably hydrogen or a C₁-C₁₀ alkyl group, more preferably hydrogen or a C₁-C₆ alkyl group, even more preferably hydrogen or methyl, most preferably hydrogen.

Of the monomers having formula (A1), those having the formula (A1-1) are preferred. 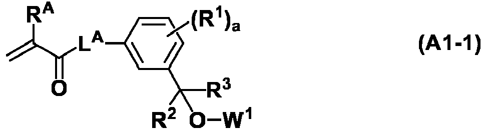 Herein "a," R^{A}, L^{A}, R¹, R², R³, and W¹ are as defined above.

Of the monomers having formula (A1-1), those having the formula (A1-2) are more preferred. 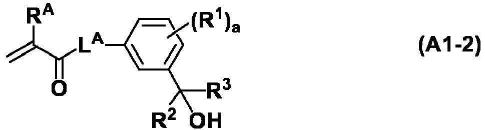 Herein "a," R^{A}, L^{A}, R¹, R², and R³ are as defined above.

Examples of the monomer having formula (A1) are shown below, but not limited thereto. Herein R^{A} is as defined above.

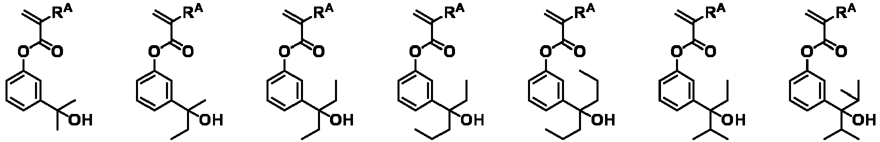

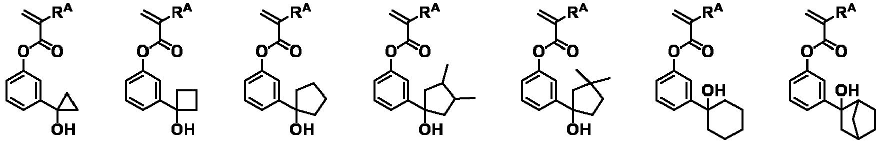

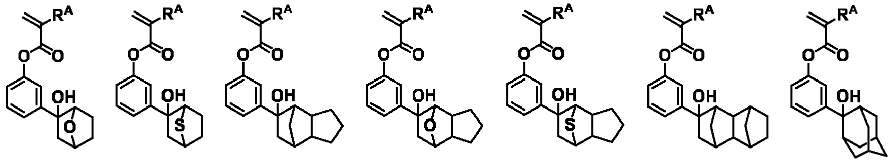

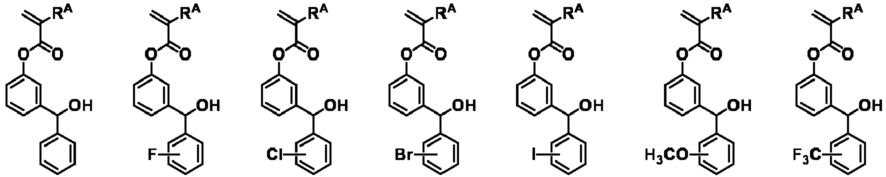

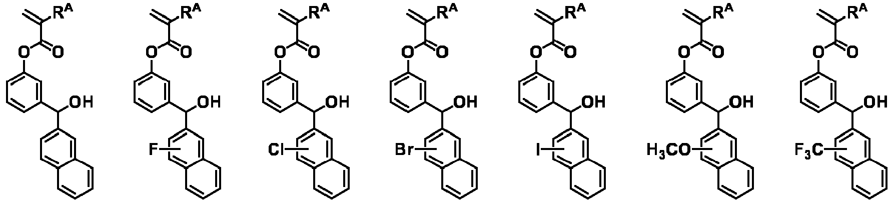

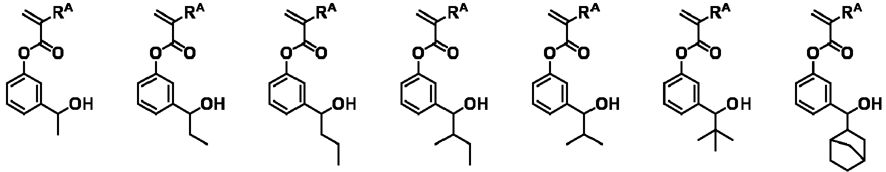

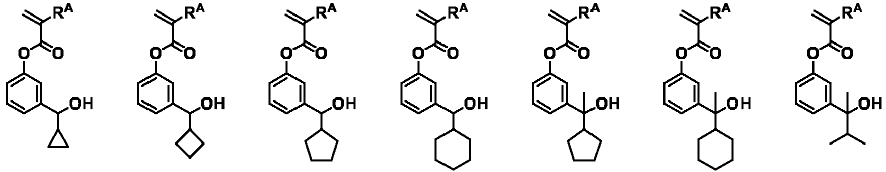

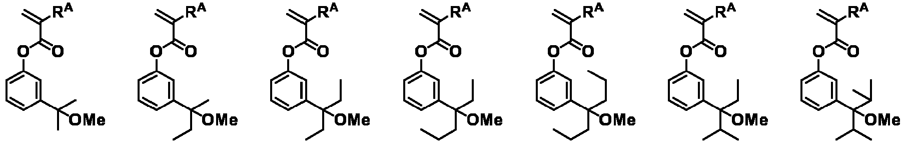

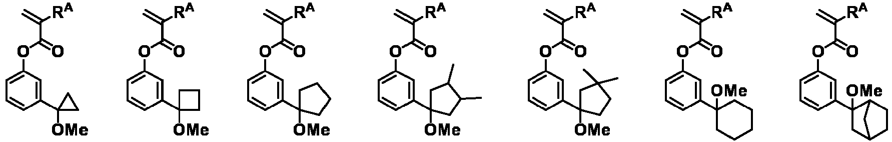

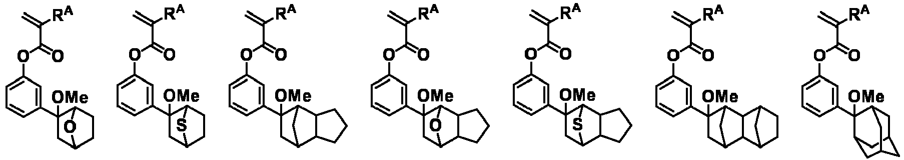

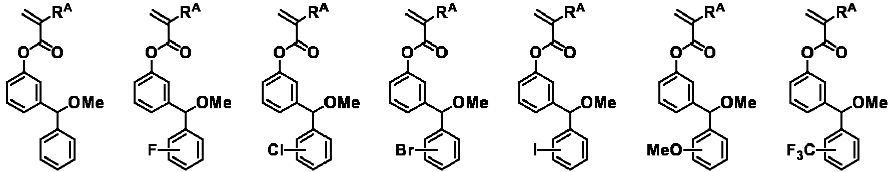

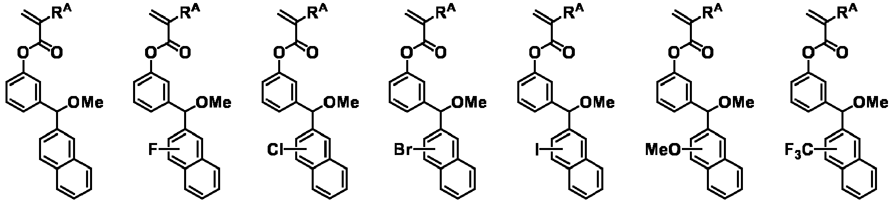

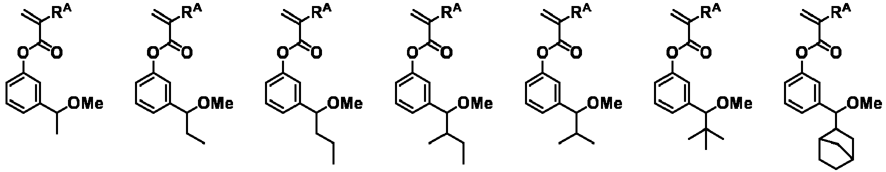

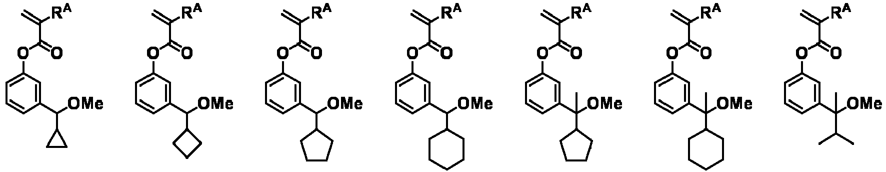

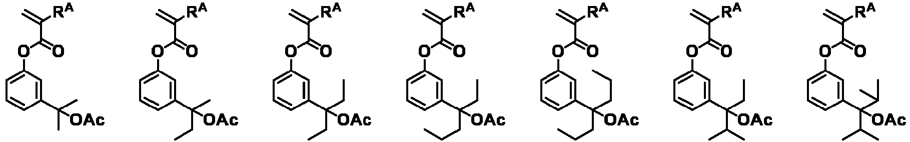

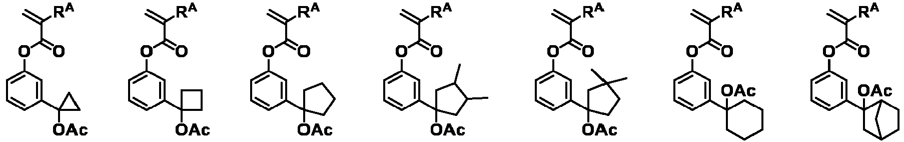

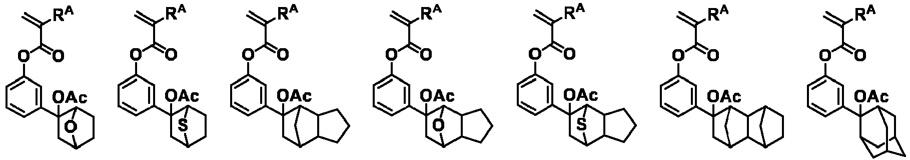

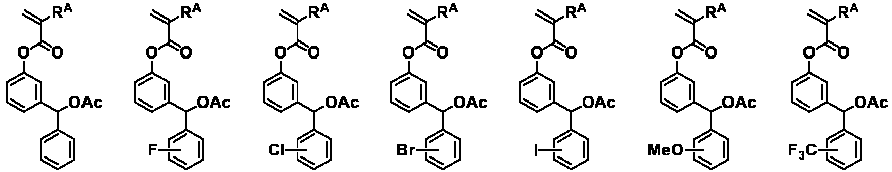

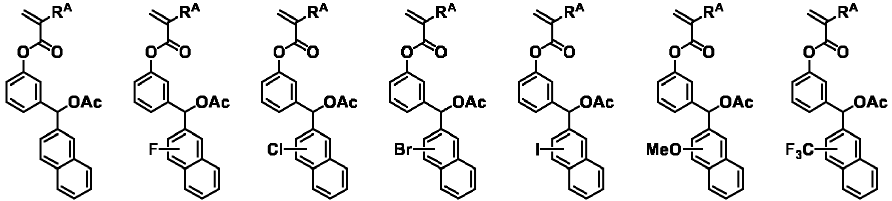

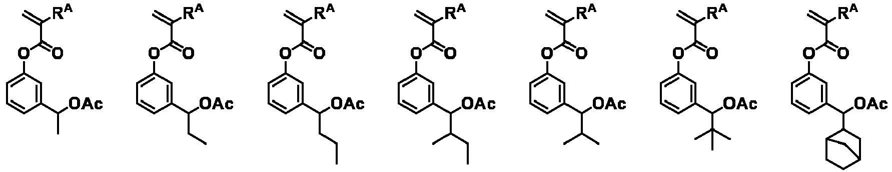

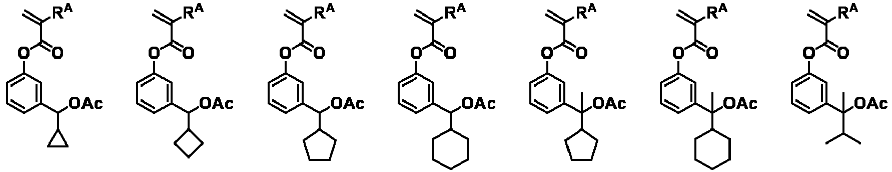

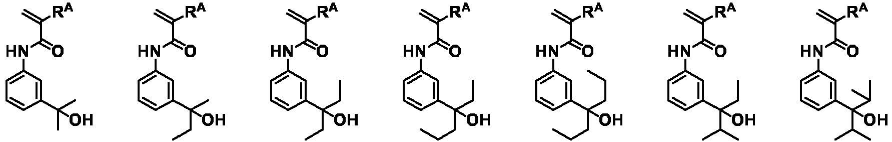

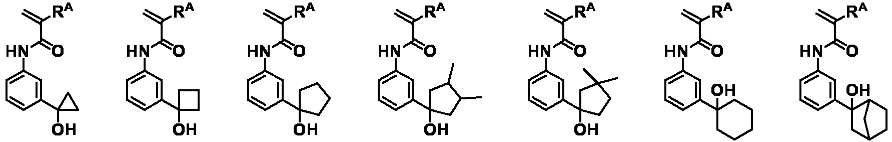

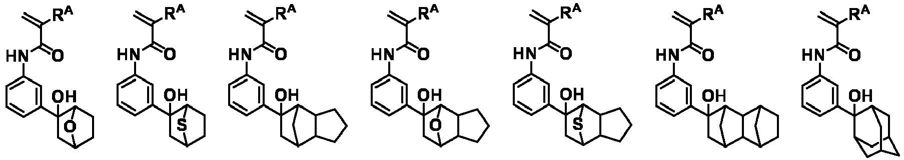

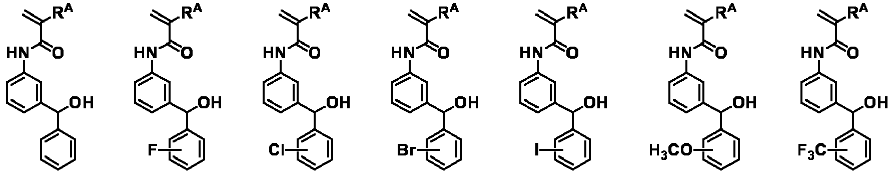

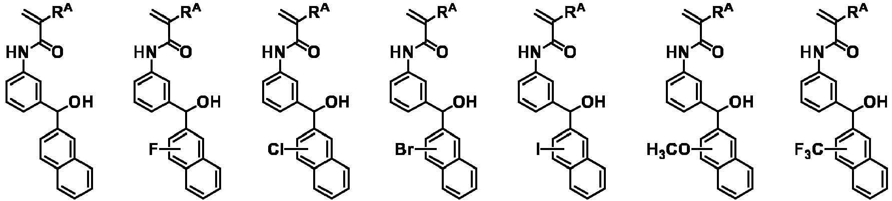

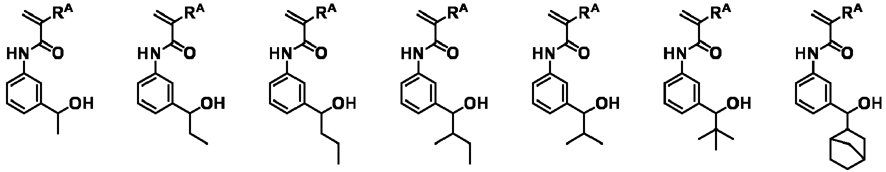

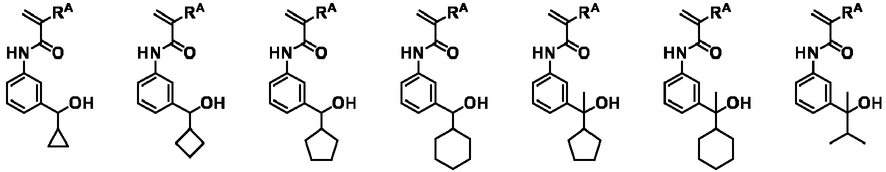

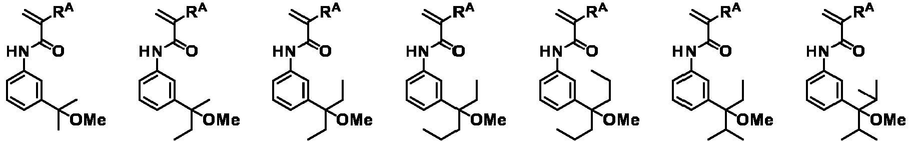

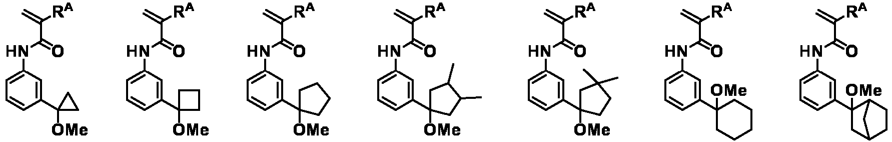

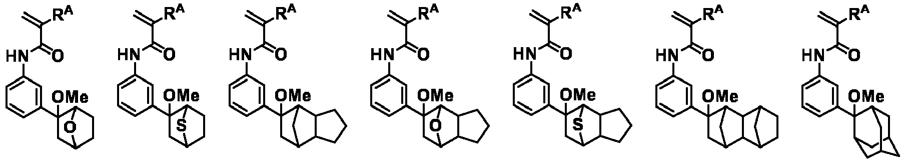

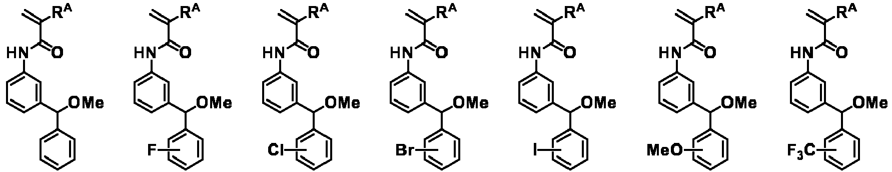

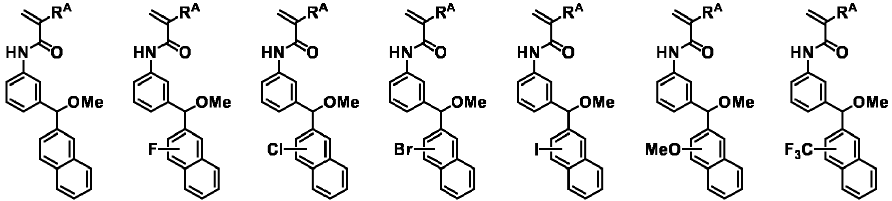

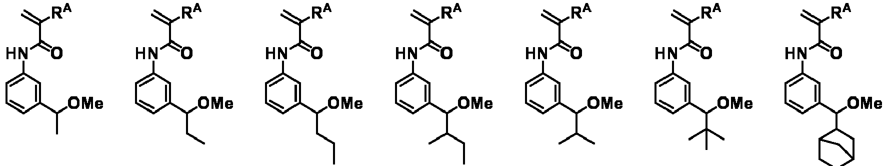

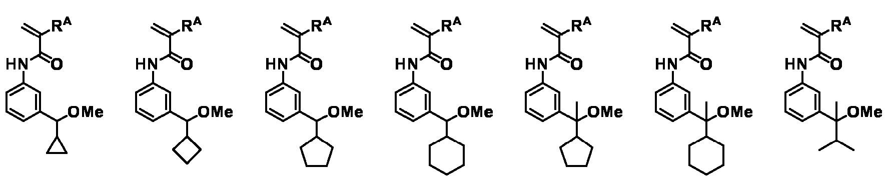

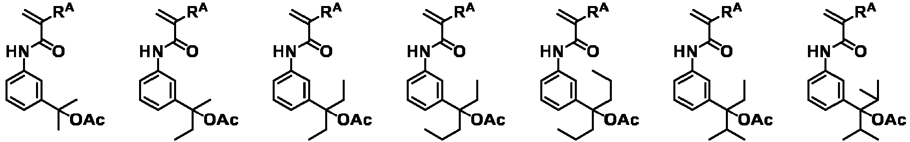

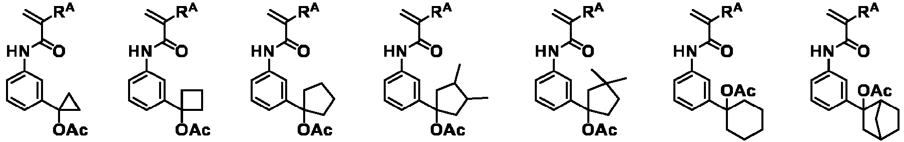

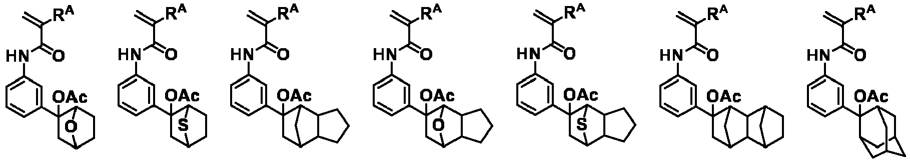

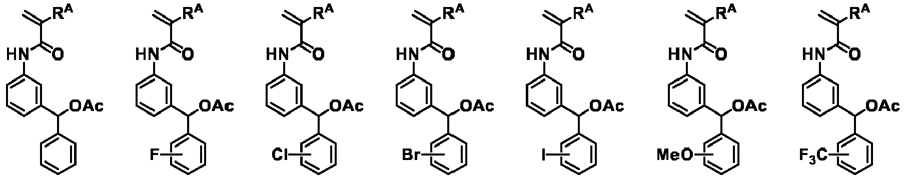

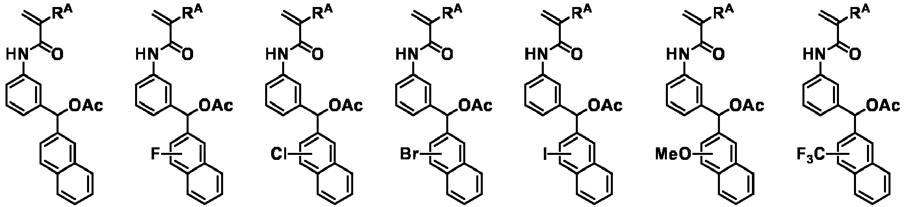

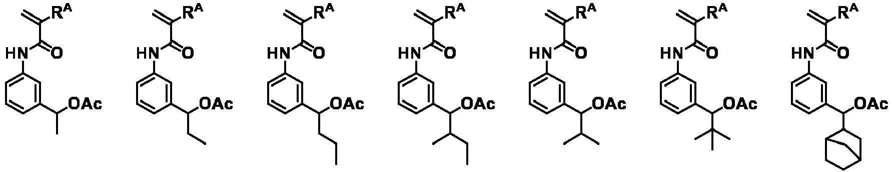

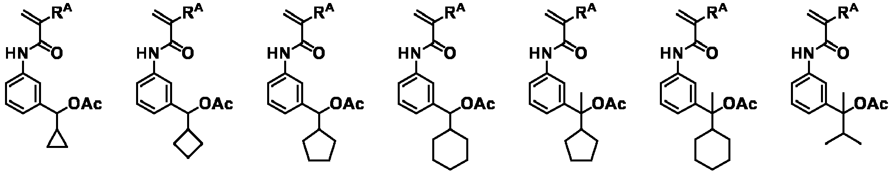

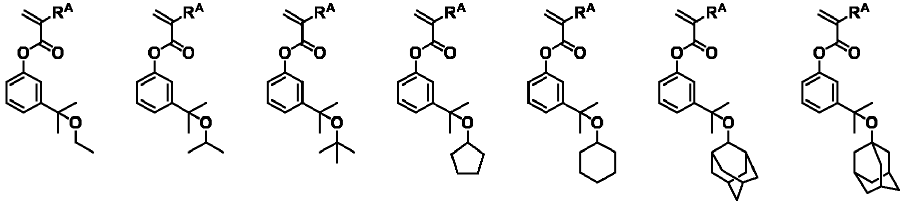

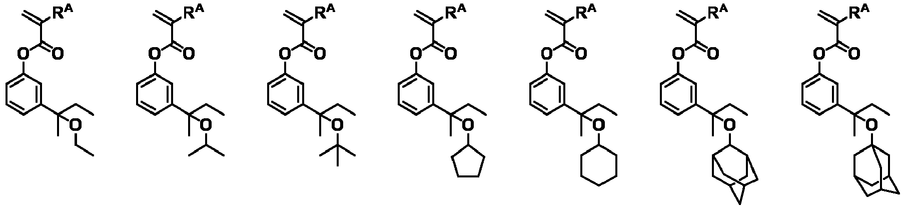

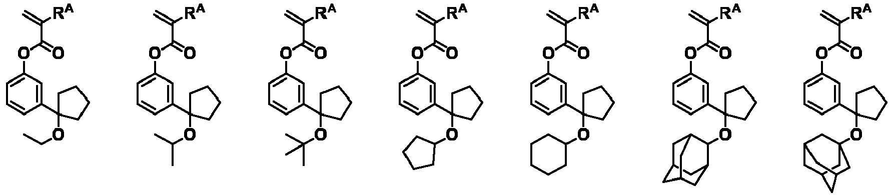

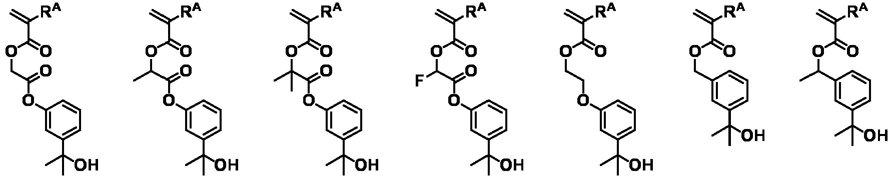

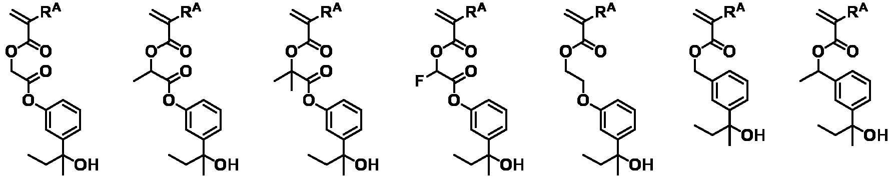

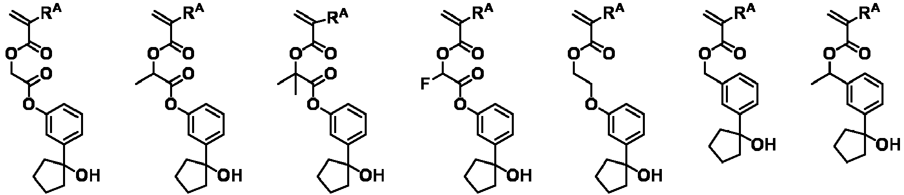

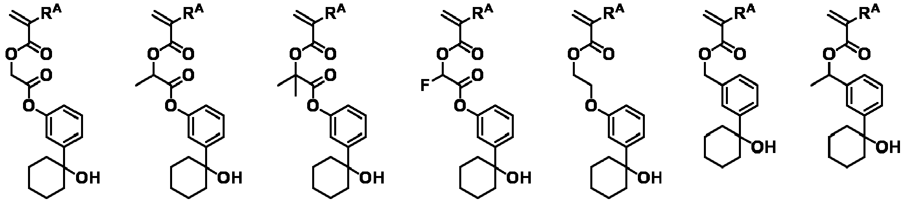

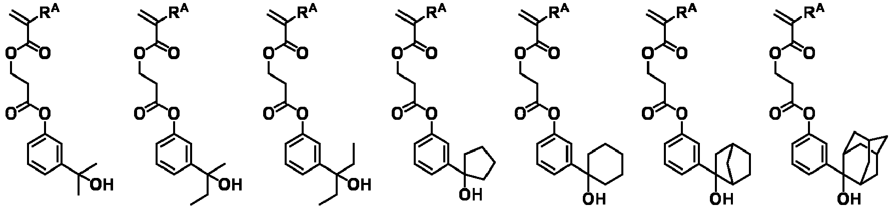

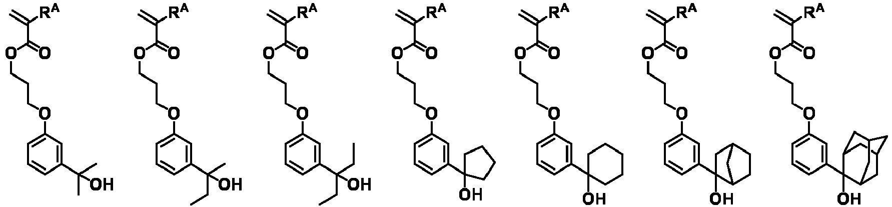

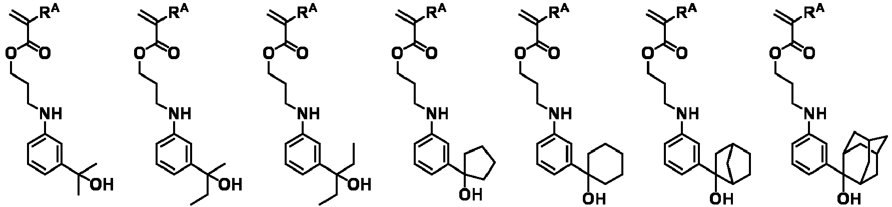

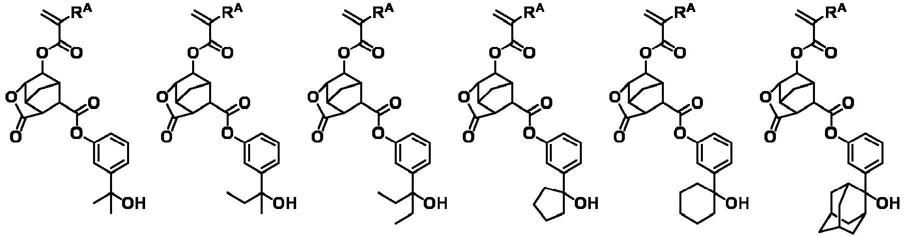

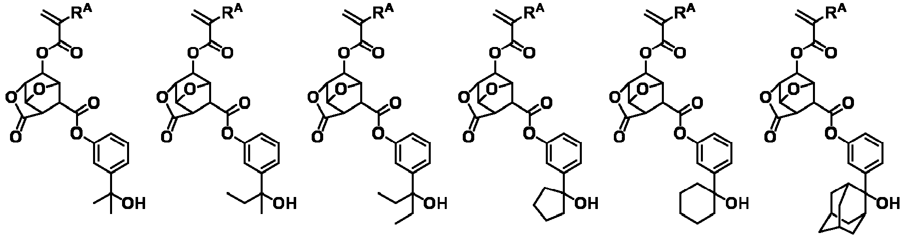

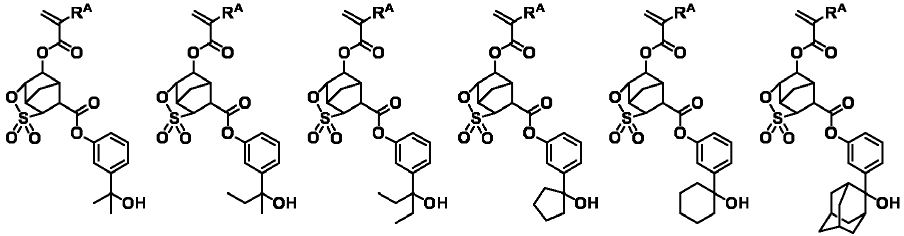

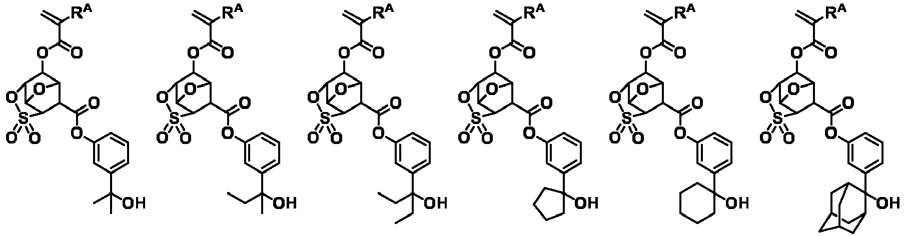

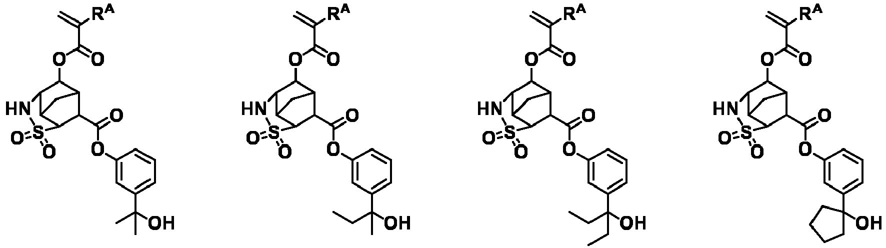

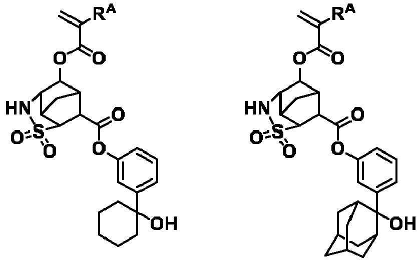

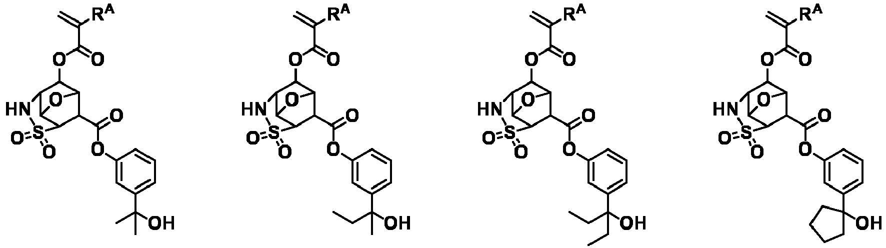

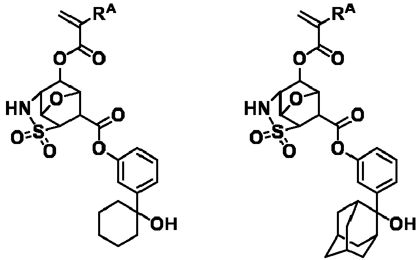

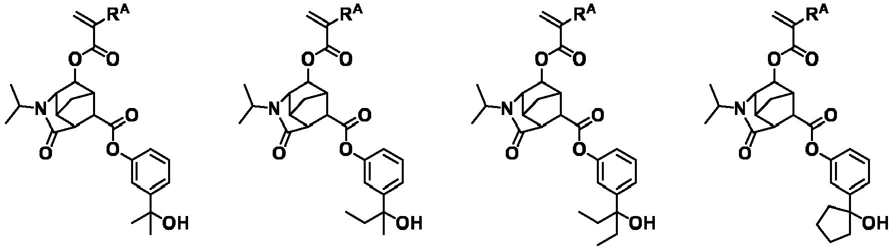

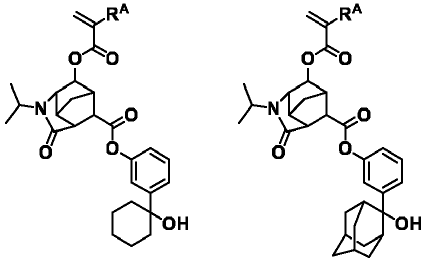

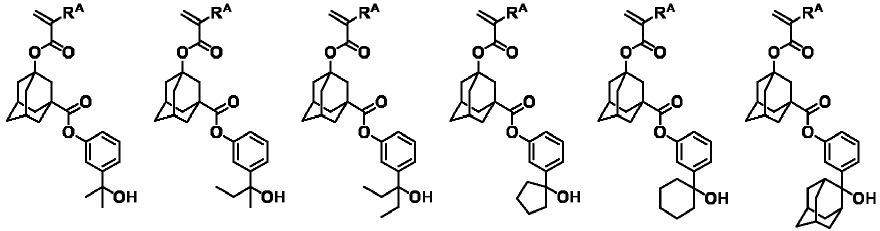

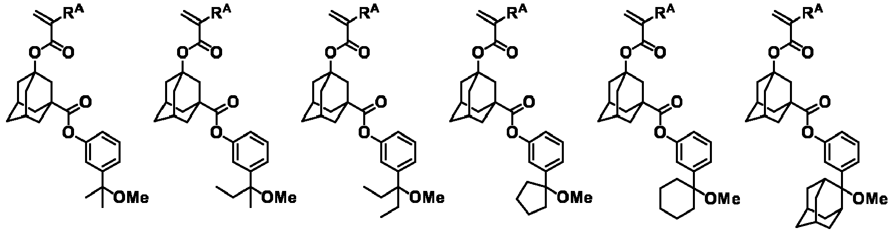

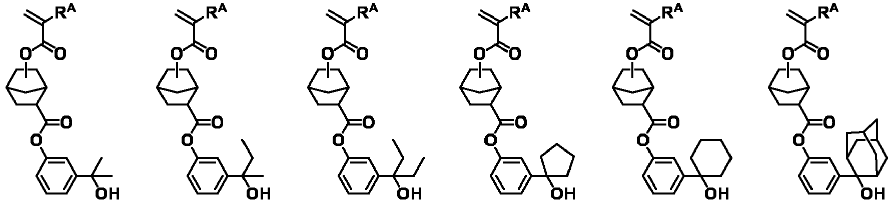

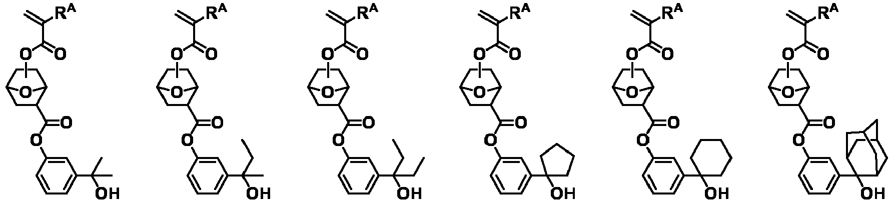

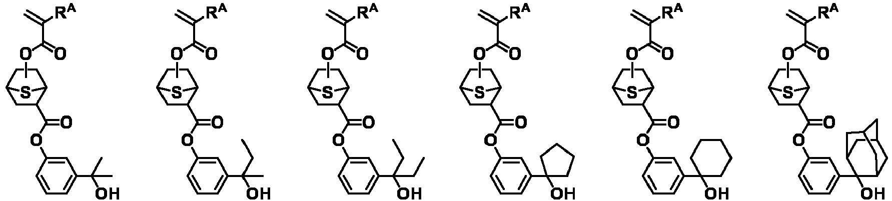

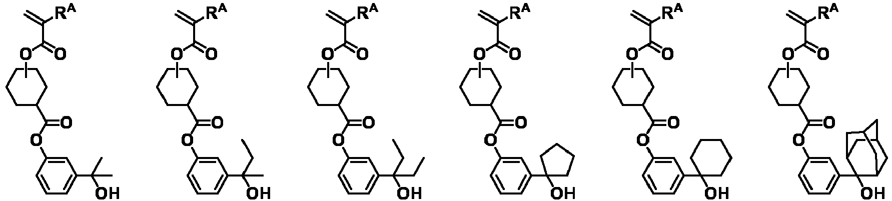

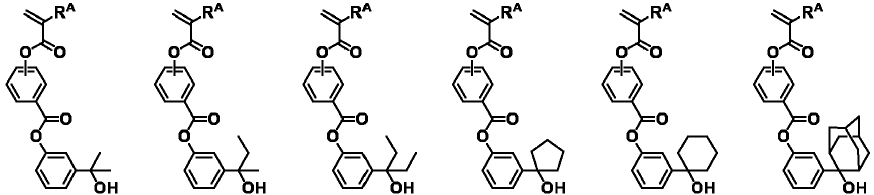

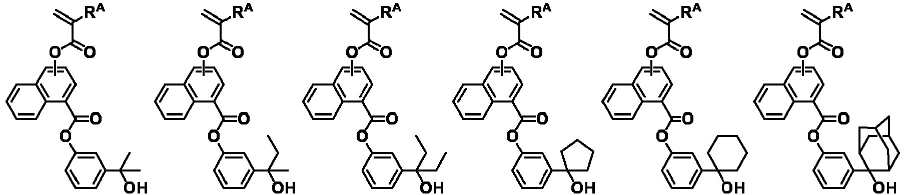

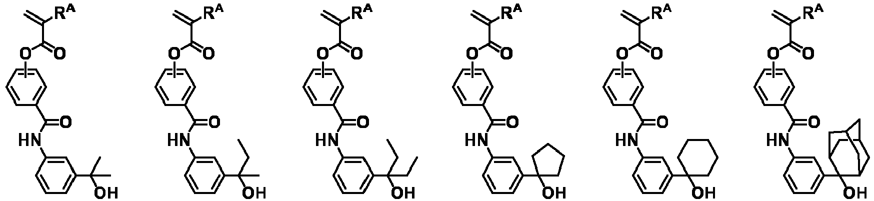

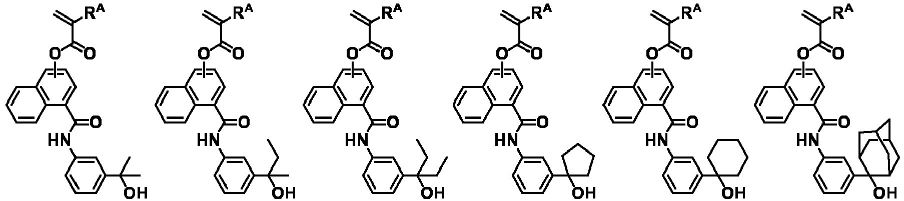

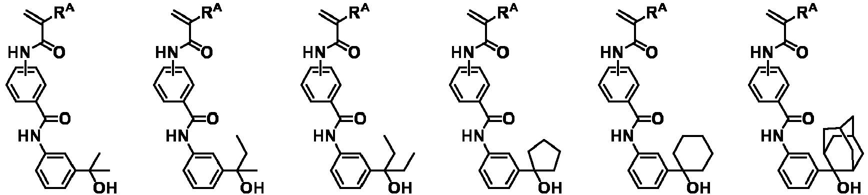

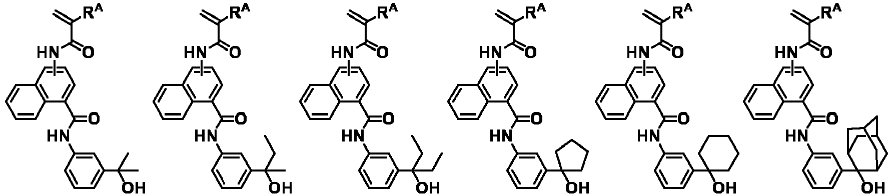

The inventive monomer can be synthesized by any well-known methods. Reference is now made to one exemplary method for synthesizing the monomer having formula (A1) wherein L^{B} is an ester bond, R² and R³ are identical, and W¹ is hydrogen, that is, monomer A1-ex as shown by the following scheme although the synthesis method is not limited thereto.

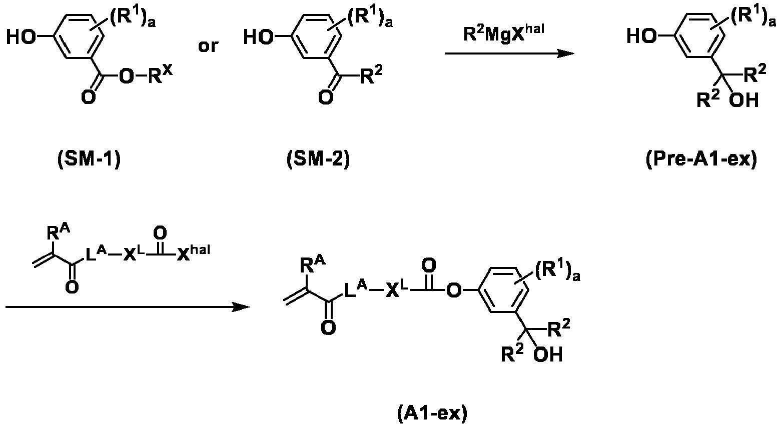

Herein "a," R^{A}, R¹, R², L^{A}, and X^{L} are as defined above. R^{X} is a C₁-C₁₀ hydrocarbyl group. X^{hal} is chlorine, bromine or iodine.

The first step is to react an ester compound SM-1 or ketone compound SM-2 with a Grignard reagent to form a diol compound Pre-A1-ex as a precursor. The ester compound SM-1 or ketone compound SM-2 may be a commercially available one or synthesized by a well-known synthesis method.

The reaction may be carried out by a well-known organic synthesis method. Specifically, the Grignard reagent is prepared by suspending metallic magnesium in an ether solvent such as tetrahydrofuran (THF) or diethyl ether and adding dropwise a dilution of a halide in the solvent to the suspension. In preparing the Grignard reagent, particularly when the halide is a chloride, the reaction can be efficiently started by adding a minor amount of 1,2-dibromoethane or iodine before the dropwise addition of the halide. A dilution of the ester compound SM-1 or ketone compound SM-2 in the solvent is added dropwise to the Grignard reagent. The reaction temperature is from room temperature to near the boiling point of the solvent. While it is desirable in view of yield to monitor the reaction by gas chromatography (GC) or silica gel thin-layer chromatography (TLC) until the reaction is complete, the reaction time is typically about 30 minutes to 2 hours. The reaction mixture is subjected to standard aqueous work-up, obtaining a precursor, diol compound Pre-A1-ex. If necessary, the monomer precursor Pre-A1-ex is purified by a standard technique such as distillation, chromatography or recrystallization.

The second step is to introduce a polymerizable group into diol compound Pre-A1-ex via an ester bond to form a monomer A1-ex.

The reaction may be carried out by a well-known organic synthesis method. Specifically, the reaction is carried out by dissolving diol compound Pre-A1-ex in a solvent (e.g., toluene, hexane, THF, or acetonitrile) in the presence of an organic base (e.g., triethylamine or pyridine) and adding dropwise an acid halide (e.g., methacrylic chloride or acrylic chloride). A promoter such as 4-dimethylaminopyridine may be added for increasing the reaction rate. The reaction temperature is from 5°C to near the boiling point of the solvent. While it is desirable in view of yield to monitor the reaction by GC or TLC until the reaction is complete, the reaction time is typically about 1 to 24 hours. The reaction mixture is subjected to standard aqueous work-up, obtaining a monomer A1-ex. If necessary, the monomer A1-ex is purified by a standard technique such as distillation, chromatography or recrystallization.

The inventive monomer is structurally characterized in that an acid dehydrating group (or acid eliminating group) is attached to the benzene ring at meta-position to the carbon atom to which a polymerizable group is attached. The acid dehydrating or eliminating group reacts with an acid generated from an acid generator to incur dehydration reaction (or elimination reaction) to create a benzyl cation. Since the neighboring hydrogen atom is released as proton, the monomer takes an olefin structure conjugated with the benzene ring and turns hydrophobic. Alternatively, hydrophobicity is also enhanced by nucleophilic attack of a phenolic hydroxy group in the copolymerized polymer chain to the created benzyl cation. If the functional group is attached to the aromatic ring at para-position to the carbon atom to which a polymerizable group is attached, the reactivity to acid is so high that the functional group in the unexposed region may also incur dehydration or elimination reaction, leading to a loss of contrast between exposed and unexposed regions. Also, if a plurality of functional groups are attached to the benzene ring, dehydration or elimination reaction does not take place to a full extent, indicating that a compound in which the olefin structure and the alcohol structure coexist can form partly in the exposed region. Therefore, when the compound in this state is developed in an alkaline developer, the hydrophilic alcohol structure is left in the exposed region, which takes up water in the developer, with the pattern profile being degraded. The inventive monomer has an acid dehydrating or eliminating group on the benzene ring at meta-position (adequate to provide a proper reactivity), which ensures that a negative pattern featuring a high dissolution contrast between exposed and unexposed regions and a satisfactory profile is formed.

### Polymer

A second embodiment of the invention is a polymer comprising repeat units derived from the monomer defined above, which are also referred to as repeat units A1.

The polymer may further comprise repeat units having the formula (A2), which are also referred to as repeat units A2. The repeat units A2 provide for etching resistance, adhesion to substrates, and solubility in alkaline developer.

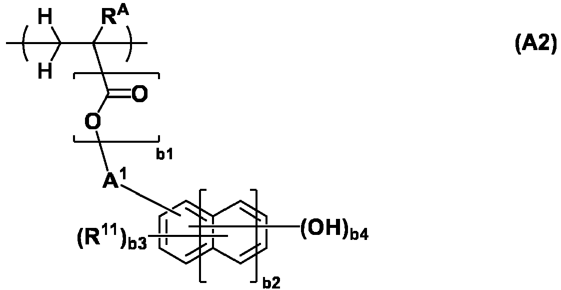

In formula (A2), b1 is 0 or 1. The subscript b2 is an integer of 0 to 2. The relevant structure represents a benzene skeleton when b2=0, a naphthalene skeleton when b2=1, or an anthracene skeleton when b2=2. The subscript b3 is an integer satisfying 0 ≤ b3 ≤ 5+2(b2)-b4. The subscript b4 is an integer of 1 to 3. In case of b2=0, preferably b3 is an integer of 0 to 3 and b4 is an integer of 1 to 3. In case of b2=1 or 2, preferably b3 is an integer of 0 to 4 and b4 is an integer of 1 to 3.

In formula (A2), R^{A} is hydrogen, fluorine, methyl or trifluoromethyl.

In formula (A2), R¹¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group. The saturated hydrocarbyl group and saturated hydrocarbyl moiety of the saturated hydrocarbyloxy group and saturated hydrocarbylcarbonyloxy group may be straight, branched or cyclic. Examples thereof include alkyl groups such as methyl, ethyl, n-propyl, isopropyl, butyl, pentyl, hexyl, and structural isomers thereof, cycloalkyl groups such as cyclopropyl, cyclobutyl, cyclopentyl and cyclohexyl, and combinations thereof. A carbon count within the upper limit ensures good solubility in alkaline developer. A plurality of R¹¹ may be identical or different when b3 is 2 or more.

In formula (A2), A¹ is a single bond or C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of b1=1 in formula (A2), the ether bond may be incorporated at any position excluding the position between the α-carbon and β-carbon relative to the ester oxygen. In case of b 1=0, the atom that bonds with the main chain becomes an ethereal oxygen, and a second ether bond may be incorporated at any position excluding the position between the α-carbon and β-carbon relative to that ethereal oxygen. Saturated hydrocarbylene groups having no more than 10 carbon atoms are desirable because of a sufficient solubility in alkaline developer.

Preferred examples of the repeat units A2 wherein b1=0 and A¹ is a single bond (meaning that the aromatic ring is directly bonded to the main chain of the polymer), that is, repeat units free of a linker: -C(=O)-O-A¹- include units derived from 3-hydroxystyrene, 4-hydroxystyrene, 5-hydroxy-2-vinylnaphthalene, and 6-hydroxy-2-vinylnaphthalene. Repeat units having the formula (A2-1) are especially preferred. 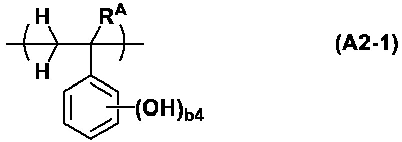 Herein R^{A} and b4 are as defined above.

Preferred examples of the repeat units A2 wherein b 1=1, that is, having a linker: -C(=O)-O-A¹- are shown below, but not limited thereto. Herein R^{A} is as defined above.

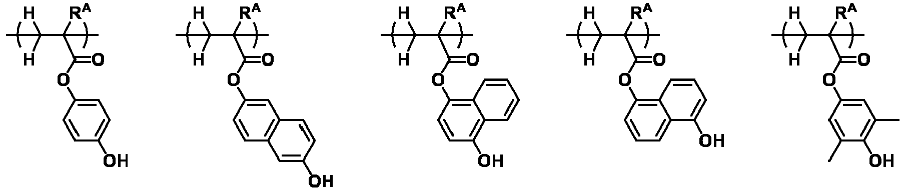

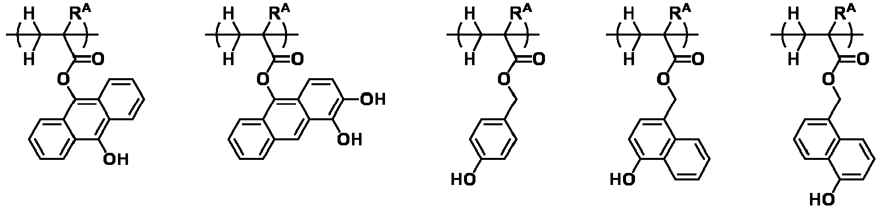

The repeat units A2 may be used alone or in admixture of two or more.

In a preferred embodiment, the polymer further comprises repeat units of at least one type selected from units having the formulae (A3), (A4) and (A5) for the purpose of enhancing etch resistance. These repeat units are simply referred to as repeat units A3, A4 and A5, respectively.

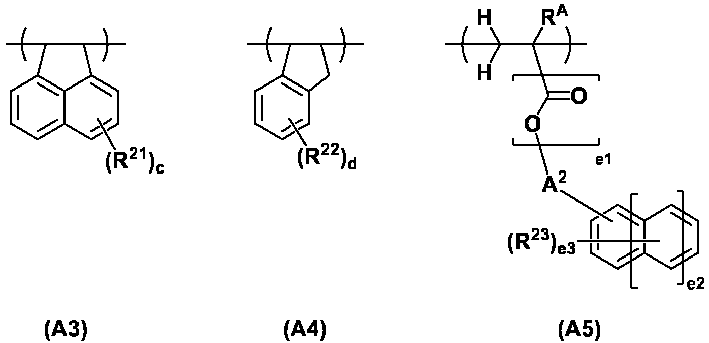

In formulae (A3) and (A4), c and d are each independently an integer of 0 to 4.

In formulae (A3) and (A4), R²¹ and R²² are each independently hydroxy, halogen, an optionally halogenated C₁-C₈ saturated hydrocarbyl group, optionally halogenated C₁-C₈ saturated hydrocarbyloxy group, or optionally halogenated C₂-C₈ saturated hydrocarbylcarbonyloxy group. The saturated hydrocarbyl group, saturated hydrocarbyloxy group and saturated hydrocarbylcarbonyloxy group may be straight, branched or cyclic. When c is 2 or more, a plurality of groups R²¹ may be identical or different. When d is 2 or more, a plurality of groups R²² may be identical or different.

In formula (A5), e1 is 0 or 1. The subscript e2 is an integer of 0 to 2, and the corresponding structure represents a benzene skeleton when e2=0, a naphthalene skeleton when e2=1, and an anthracene skeleton when e2=2. The subscript e3 is an integer of 0 to 5. In case of e2=0, preferably e3 is an integer of 0 to 3. In case of e2=1 or 2, preferably e3 is an integer of 0 to 4.

In formula (A5), R^{A} is hydrogen, fluorine, methyl or trifluoromethyl.

In formula (A5), R²³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen atom, nitro group, cyano group, C₁-C₂₀ saturated hydrocarbylsulfinyl group, or C₁-C₂₀ saturated hydrocarbylsulfonyl group. The saturated hydrocarbyl group, saturated hydrocarbyloxy group, saturated hydrocarbylcarbonyloxy group, saturated hydrocarbyloxyhydrocarbyl group, saturated hydrocarbylthiohydrocarbyl group, saturated hydrocarbylsulfinyl group and saturated hydrocarbylsulfonyl group may be straight, branched or cyclic. When e3 is 2 or more, a plurality of groups R²³ may be identical or different.

R²³ is preferably selected from halogen atoms such as chlorine, bromine and iodine; saturated hydrocarbyl groups such as methyl, ethyl, propyl, butyl, pentyl, hexyl, cyclopentyl, cyclohexyl, and structural isomers thereof; and saturated hydrocarbyloxy groups such as methoxy, ethoxy, propoxy, butoxy, pentyloxy, hexyloxy, cyclopentyloxy, cyclohexyloxy, and structural isomers of their hydrocarbon moiety. Inter alia, methoxy and ethoxy are useful.

The saturated hydrocarbylcarbonyloxy group may be readily introduced into a polymer even after polymerization, by a chemical modification method and is advantageously utilized for fine adjustment of the solubility of the polymer in alkaline developer. Examples of the saturated hydrocarbylcarbonyloxy group include methylcarbonyloxy, ethylcarbonyloxy, propylcarbonyloxy, butylcarbonyloxy, pentylcarbonyloxy, hexylcarbonyloxy, cyclopentylcarbonyloxy, cyclohexylcarbonyloxy, benzoyloxy, and structural isomers of their hydrocarbon moiety. As long as the carbon count is equal to or less than 20, an appropriate effect of controlling or adjusting (typically reducing) the solubility of the polymer in alkaline developer is obtainable, and the generation of scum or development defects may be suppressed.

Of the foregoing preferred substituent groups, such substituent groups as chlorine, bromine, iodine, methyl, ethyl and methoxy are useful because the corresponding monomers may be readily prepared.

In formula (A5), A³ is a single bond or C₁-C₁₀ saturated hydrocarbylene group in which some constituent -CH₂- may be replaced by -O-. The saturated hydrocarbylene group may be straight, branched or cyclic. Examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of e1=1 in formula (A5), the ether bond may be incorporated at any position excluding the position between the α- and β-carbons relative to the ester oxygen. In case of e1=0, the atom bonding to the backbone becomes an ether oxygen atom, and a second ether bond may be incorporated at any position excluding the position between the α- and β-carbons relative to the ether oxygen. Saturated hydrocarbylene groups having no more than 10 carbon atoms are desirable because of a sufficient solubility in alkaline developer.

Preferred examples of the repeat units A5 wherein e1 is 0 and A³ is a single bond (meaning that the aromatic ring is directly bonded to the main chain of the polymer), that is, repeat units free of the linker: -C(=O)-O-A³- include units derived from styrene, 4-chlorostyrene, 4-bromostyrene, 4-methylstyrene, 4-methoxystyrene, 4-acetoxystyrene, 2-hydroxypropylstyrene, 2-vinylnaphthalene, and 3-vinylnaphthalene.

Preferred examples of the repeat units A5 wherein e1 is 1, that is, having the linker: -C(=O)-O-A³- are shown below, but not limited thereto. R^{A} is as defined above.

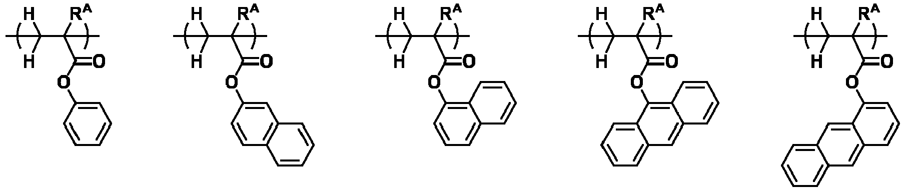

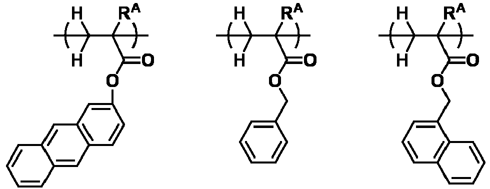

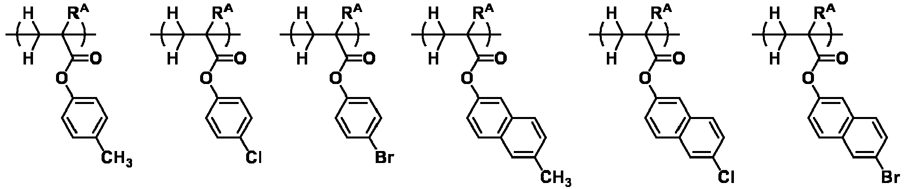

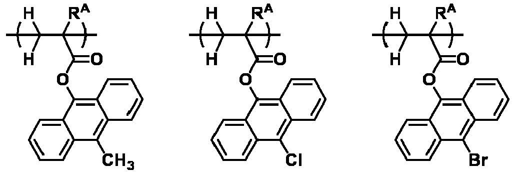

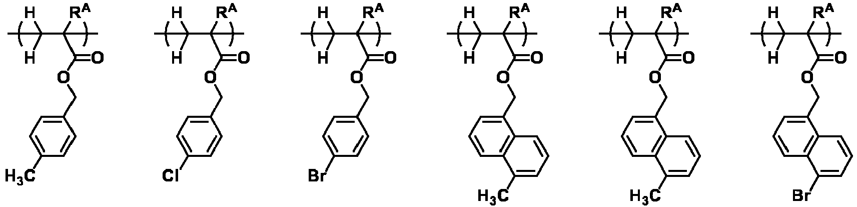

When repeat units of at least one type selected from repeat units A3 to A5 are incorporated, better performance is obtained because not only the aromatic ring possesses etch resistance, but the cyclic structure incorporated into the main chain also exerts the effect of improving etch resistance and resistance to EB irradiation during pattern inspection step.

The repeat units A3 to A5 may be used alone or in admixture of two or more.

In a preferred embodiment, the polymer further comprises repeat units of at least one type selected from repeat units having the formula (A6), repeat units having the formula (A7), repeat units having the formula (A8), repeat units having the formula (A9), repeat units having the formula (A10), repeat units having the formula (A11), repeat units having the formula (A12), and repeat units having the formula (A13), shown below. Notably these repeat units are also referred to as repeat units A6 to A13. The repeat units A6 to A13 are effective for suppressing acid diffusion and forming patterns with improved resolution and reduced LER.

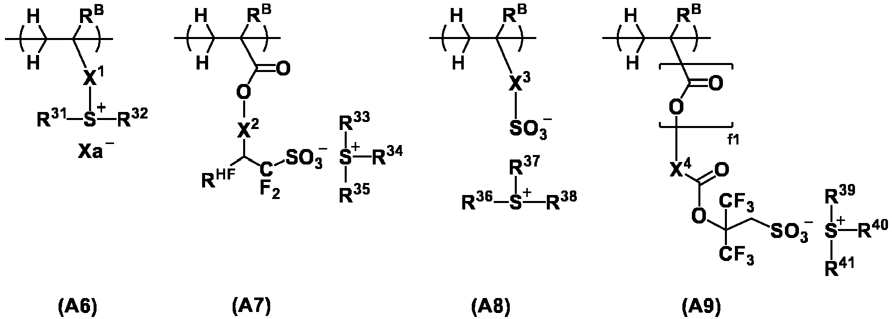

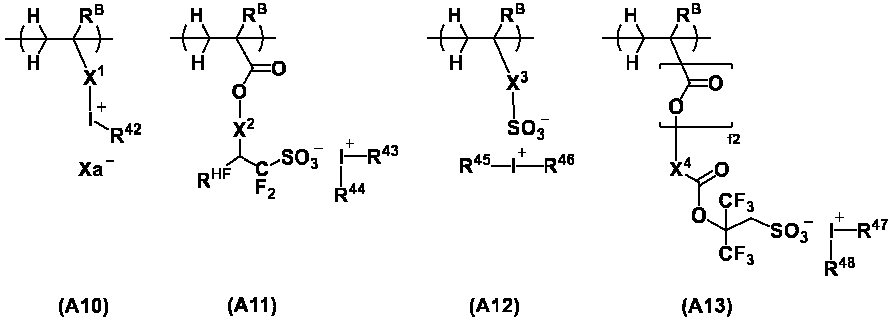

In formulae (A6) to (A13), R^{B} is each independently hydrogen or methyl. X¹ is a single bond, a C₁-C₆ aliphatic hydrocarbylene group, phenylene group, naphthylene group, or C₇-C₁₈ group obtained by combining the foregoing, -O-X¹¹-, -C(=O)-O-X¹¹-, or -C(=O)-NH-X¹¹-, wherein X¹¹ is a C₁-C₆ aliphatic hydrocarbylene group, phenylene group, naphthylene group, or C₇-C₁₈ group obtained by combining the foregoing, which may contain a carbonyl moiety, ester bond, ether bond or hydroxy moiety. X² is a single bond or -X²¹-C(=O)-O-, wherein X²¹ is a C₁-C₂₀ hydrocarbylene group which may contain a heteroatom. X³ is a single bond, methylene, ethylene, phenylene, fluorinated phenylene, trifluoromethyl-substituted phenylene group, -O-X³¹-, -C(=O)-O-X³¹-, or -C(=O)-NH-X³¹-, wherein X³¹ is a C₁-C₆ aliphatic hydrocarbylene group, phenylene group, fluorinated phenylene group, trifluoromethyl-substituted phenylene group, or C₇-C₂₀ group obtained by combining the foregoing, which may contain a carbonyl moiety, ester bond, ether bond or hydroxy moiety. X⁴ is each independently a single bond or a C₁-C₃₀ hydrocarbylene group which may contain a heteroatom. The subscripts f1 and f2 are each independently 0 or 1. When X⁴ is a single bond, f1 and f2 are 0.

In formulae (A6) and (A10), Xa⁻ is a non-nucleophilic counter ion, examples of which include those described in JP-A 2007-145797 and JP-A 2010-113209.

In formulae (A7) and (A11) wherein X² is -X²¹-C(=O)-O-, X²¹ is a C₁-C₂₀ hydrocarbylene group which may contain a heteroatom, examples of which are shown below, but not limited thereto.

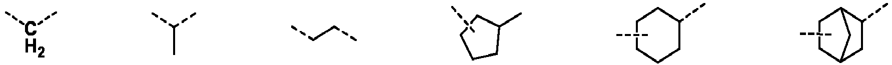

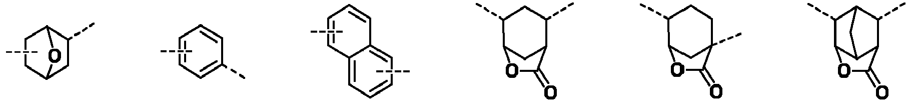

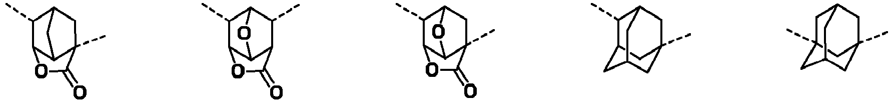

In formulae (A7) and (A11), R^{HF} is hydrogen or trifluoromethyl. Examples of the repeat units A7 and A11 wherein R^{HF} is hydrogen include those described in JP-A 2010-116550. Examples of the repeat units A7 and A11 wherein R^{HF} is trifluoromethyl include those described in JP-A 2010-077404. Examples of the repeat units A8 and A12 include those described in JP-A 2007-145797 and JP-A 2010-113209.

Preferred examples of the anion in the monomers from which repeat units A9 and A13 are derived are shown below, but not limited thereto. 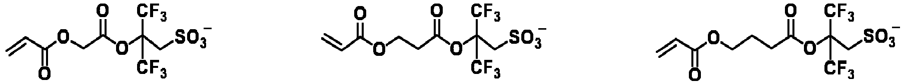 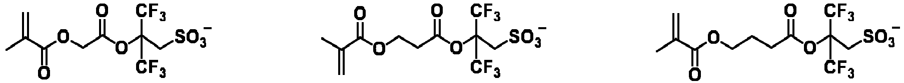 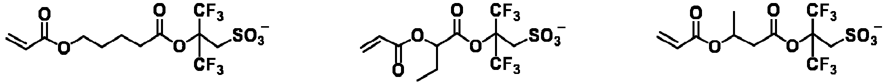 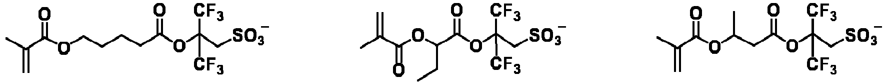 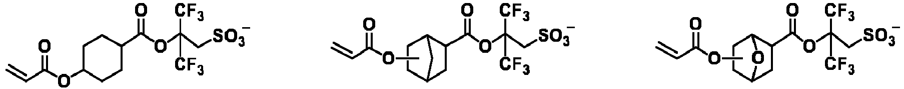 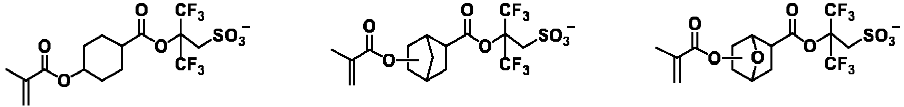 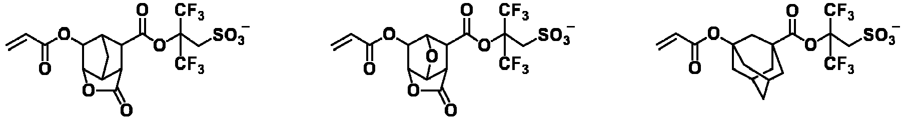 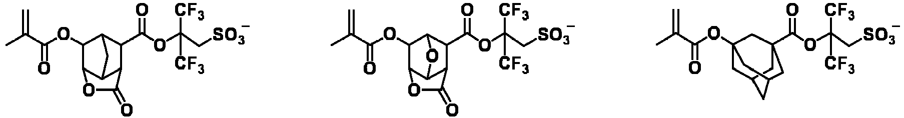 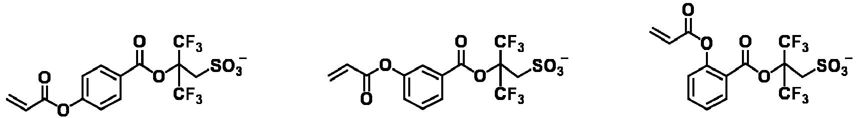 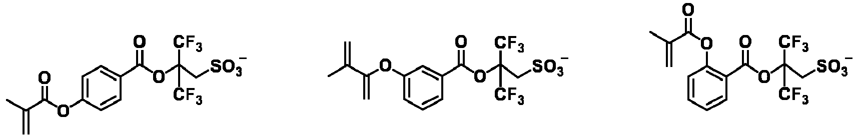 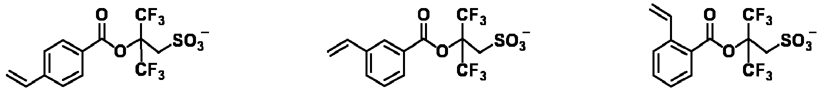 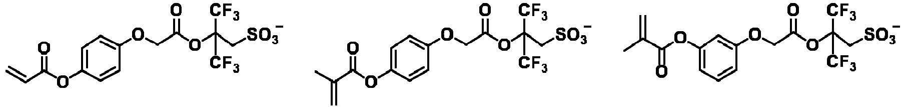 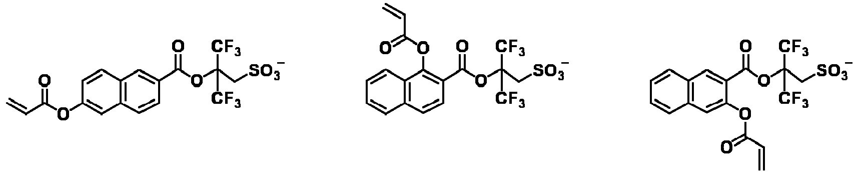 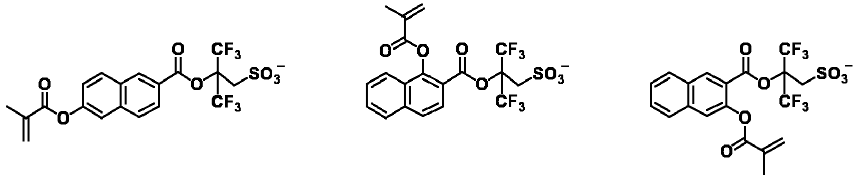 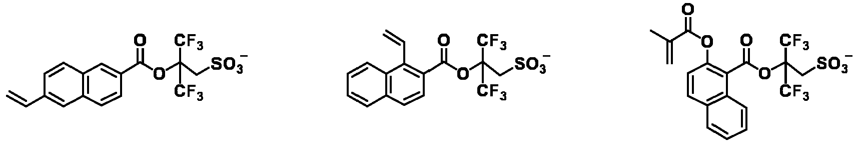 In formulae (A6) to (A13), R³¹ to R⁴⁸ are each independently halogen or a C₁-C₂₀ hydrocarbyl group which may contain a heteroatom. Suitable halogen atoms include fluorine, chlorine, bromine and iodine. The hydrocarbyl group may be saturated or unsaturated and straight, branched or cyclic. Examples of the hydrocarbyl group are as exemplified above for the hydrocarbyl group represented by R¹ in formula (A1). In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, fluorine, chlorine, bromine, iodine, cyano moiety, nitro moiety, carbonyl moiety, ether bond, ester bond, sulfonate ester bond, carbonate bond, lactone ring, sultone ring, carboxylic anhydride (-C(=O)-O-C(=O)-) or haloalkyl moiety.

A pair of R³¹ and R³² may bond together to form a ring with the sulfur atom to which they are attached. Also, R³³ and R³⁴, R³⁶ and R³⁷, or R³⁹ and R⁴⁰ may bond together to form a ring with the sulfur atom to which they are attached. Examples of the ring are shown below.

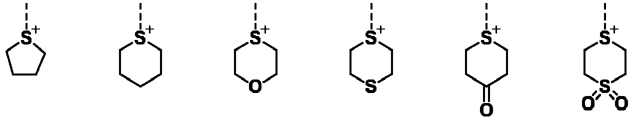

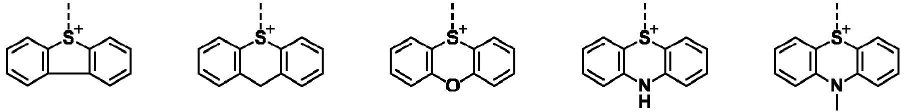

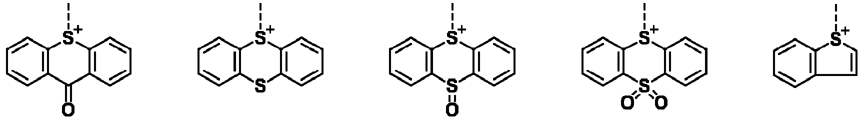

Examples of the sulfonium cation in repeat units A7 to A9 are shown below, but not limited thereto.

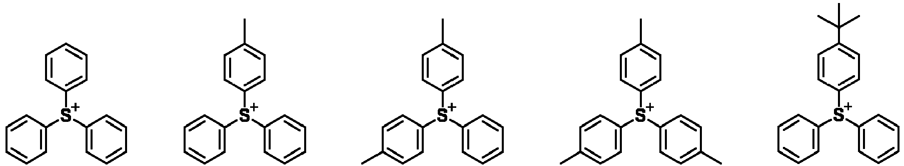

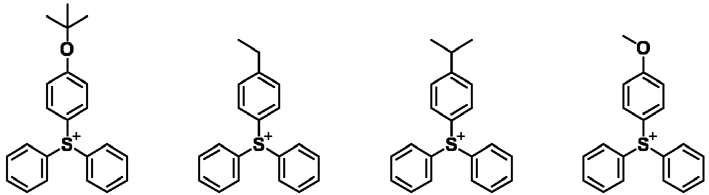

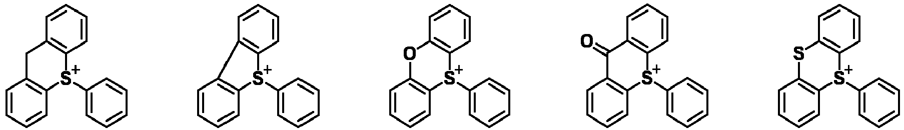

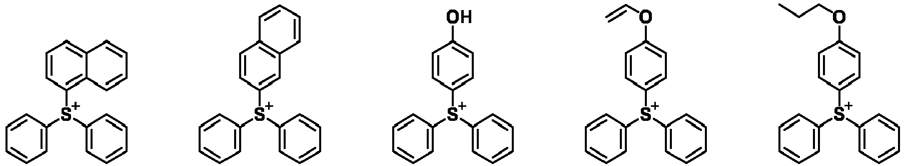

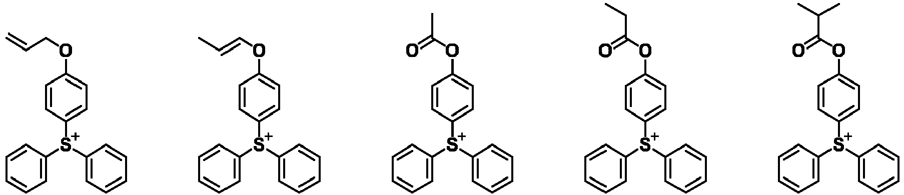

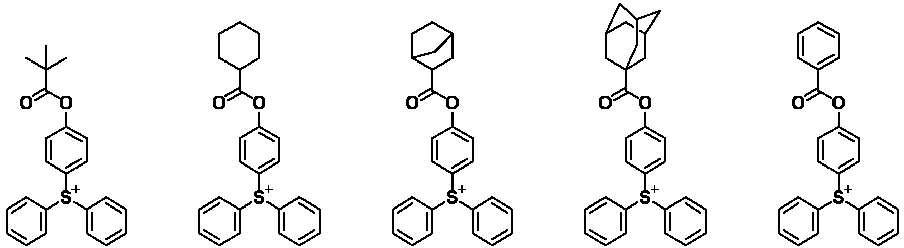

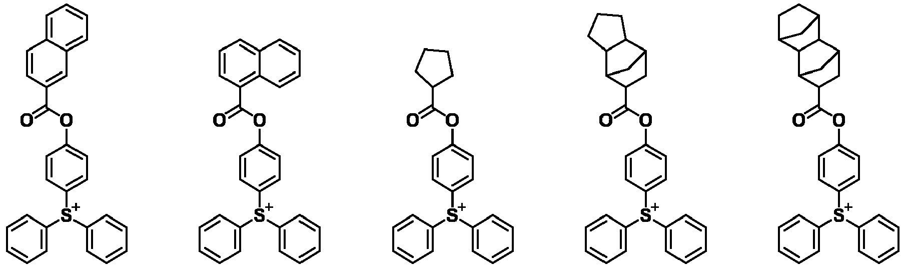

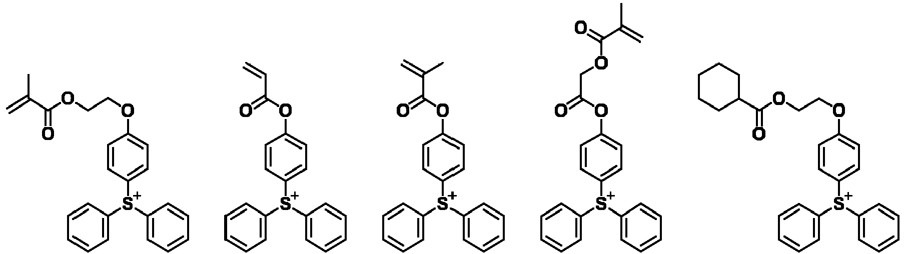

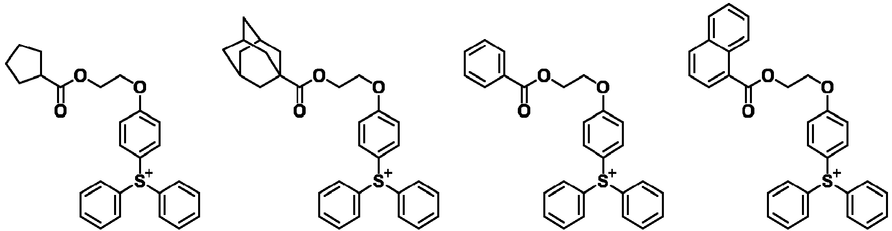

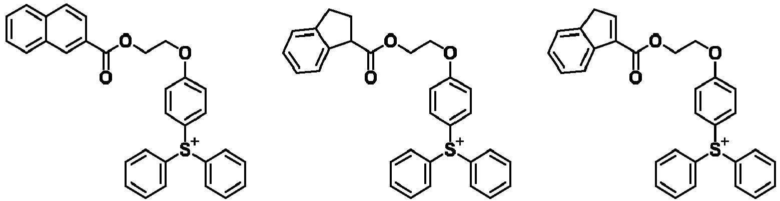

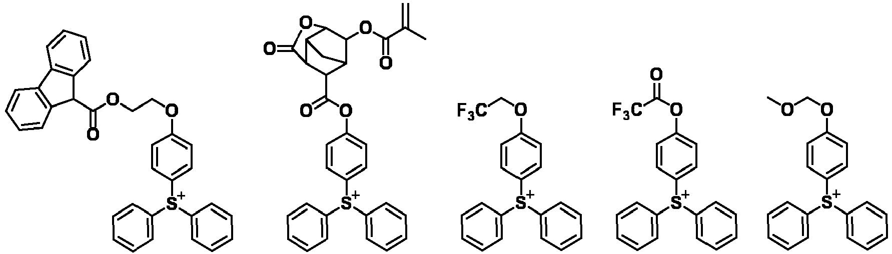

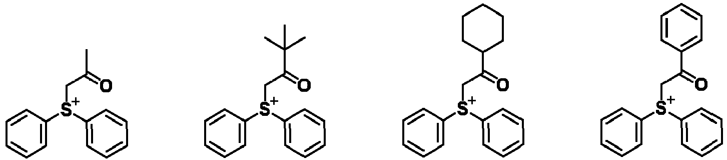

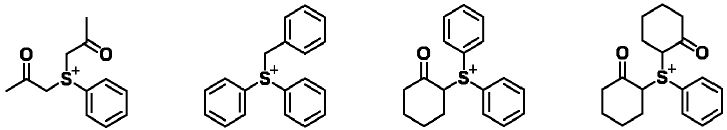

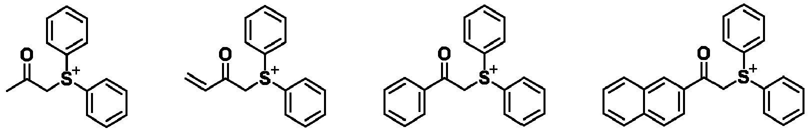

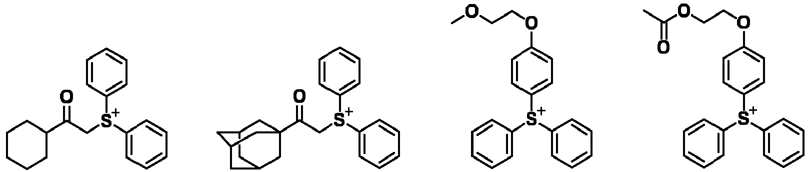

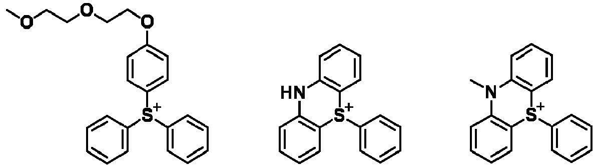

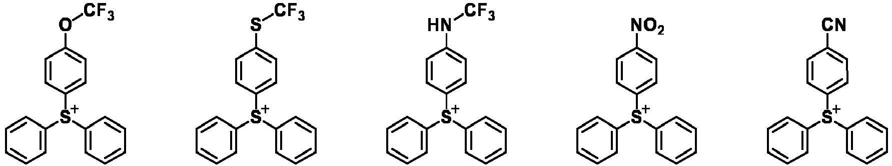

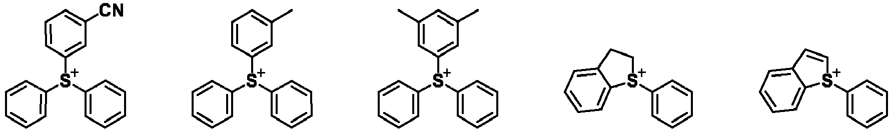

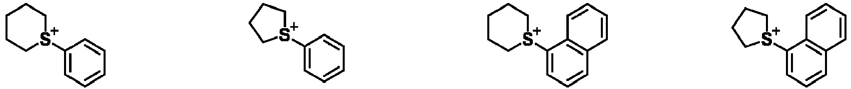

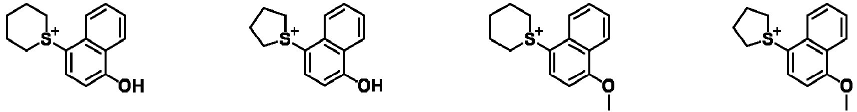

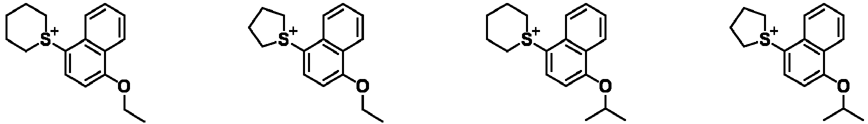

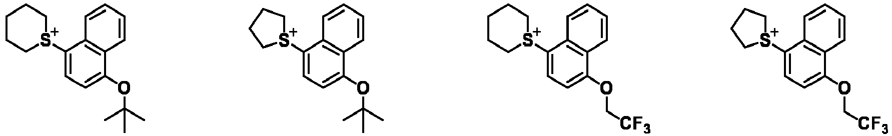

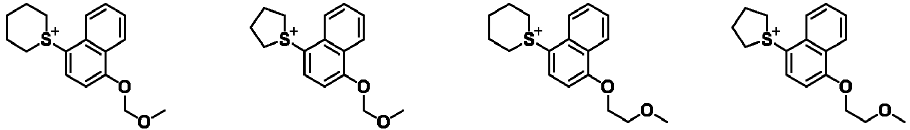

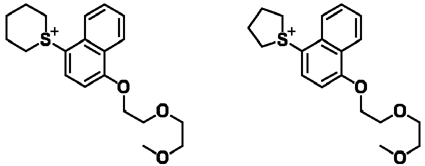

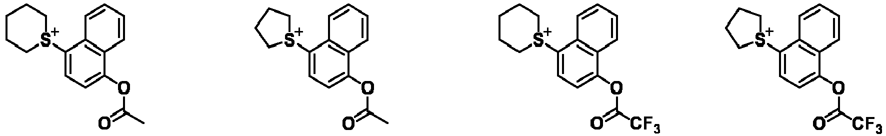

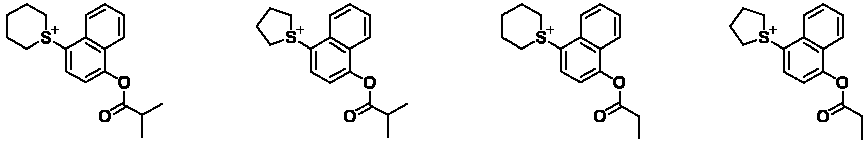

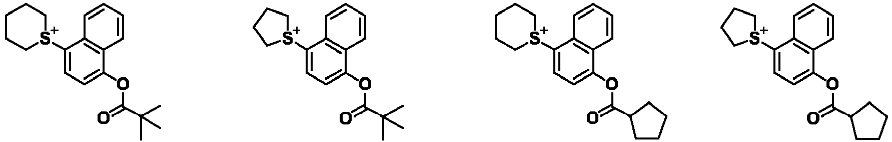

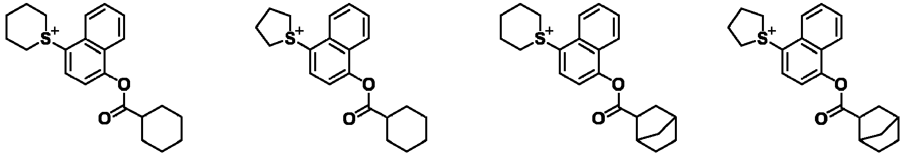

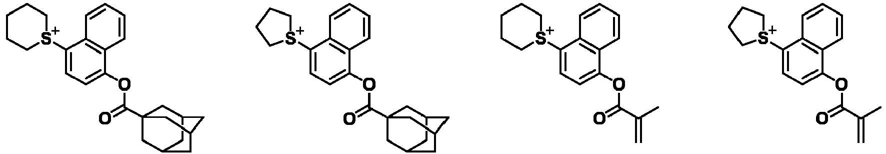

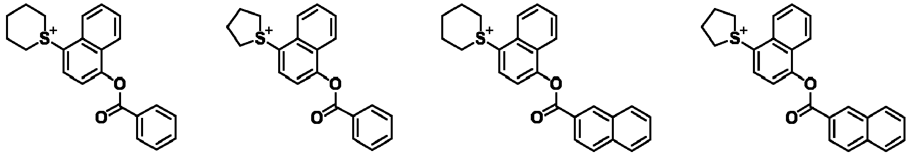

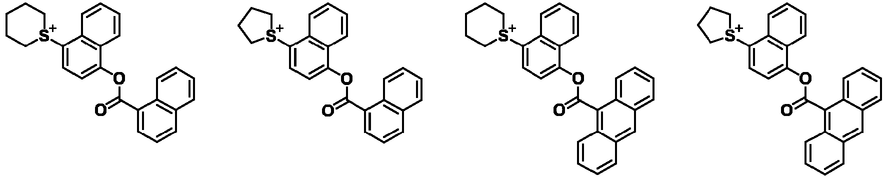

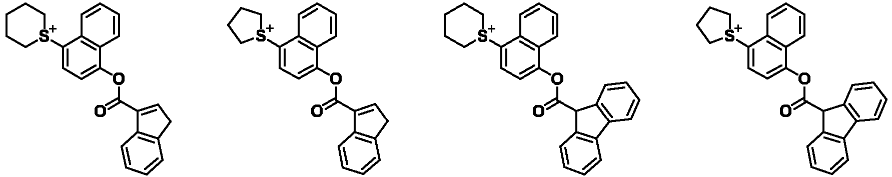

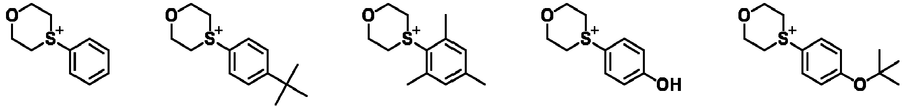

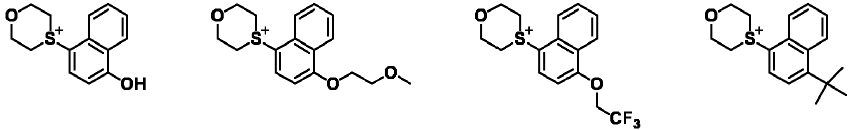

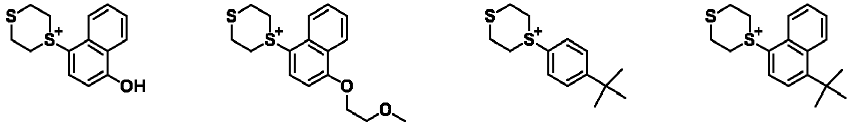

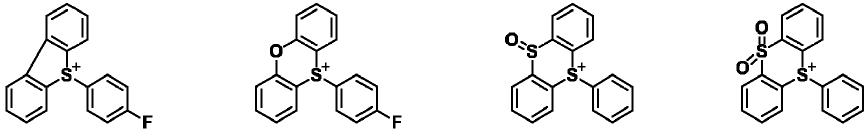

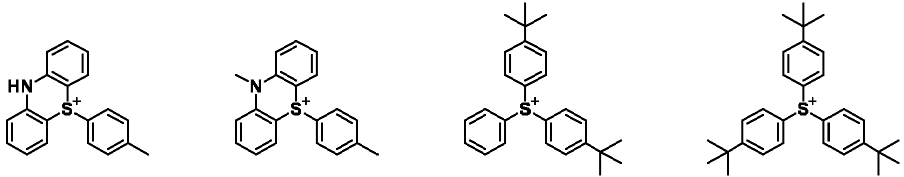

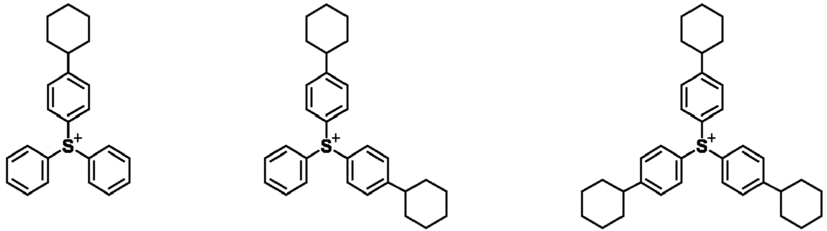

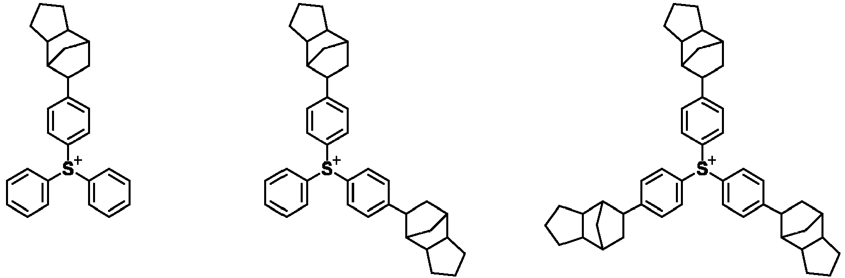

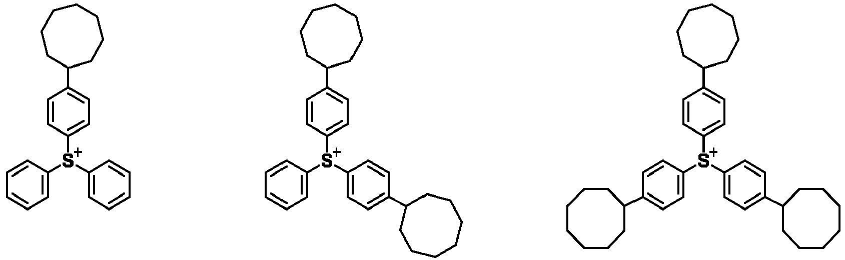

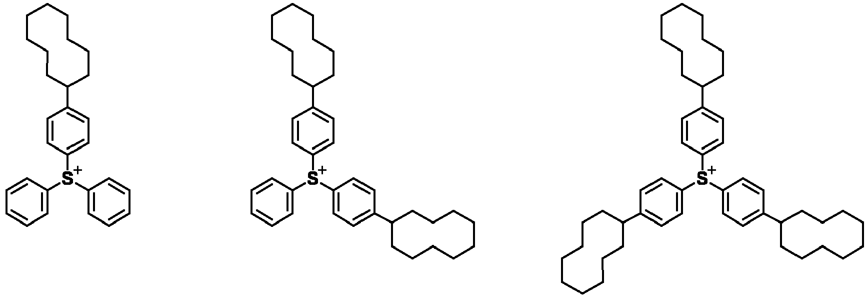

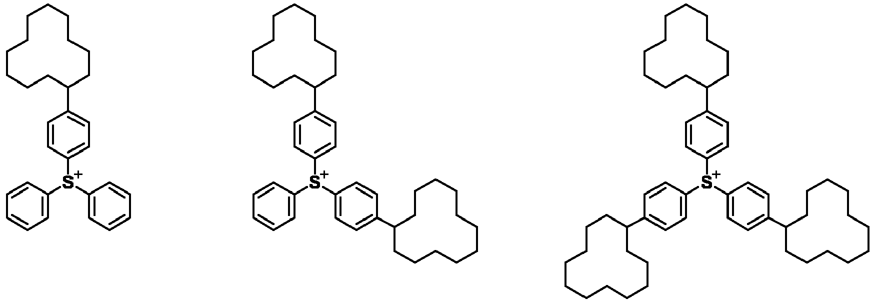

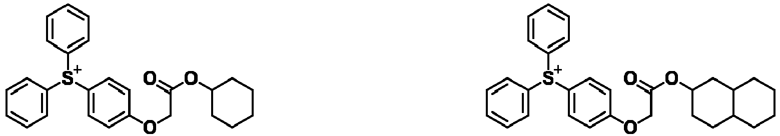

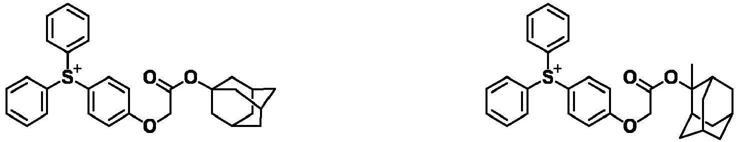

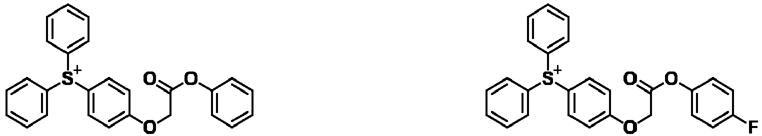

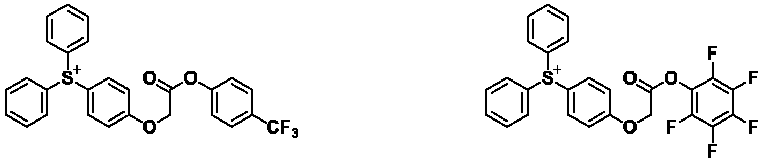

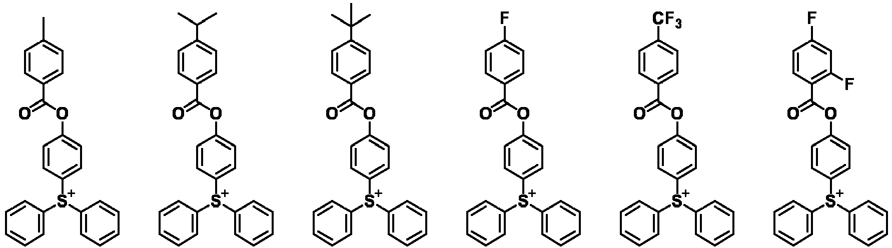

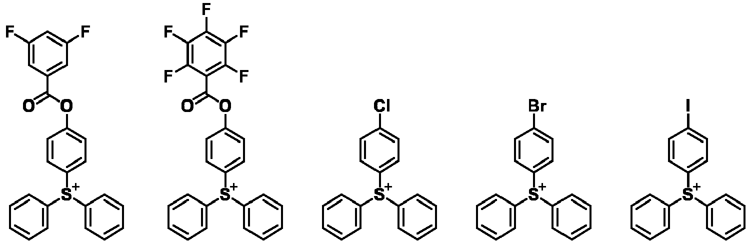

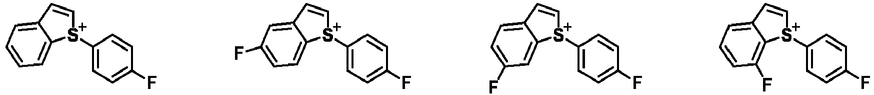

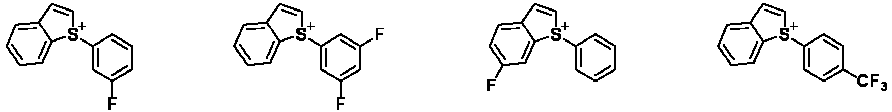

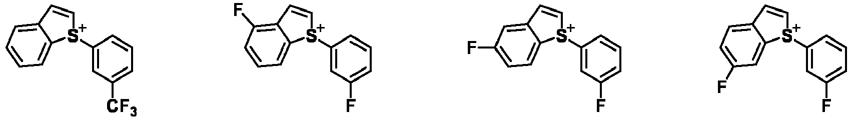

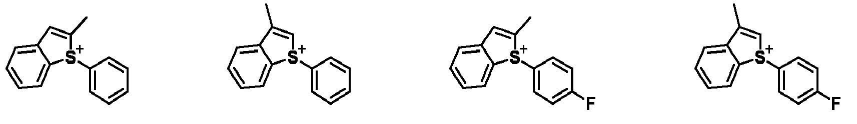

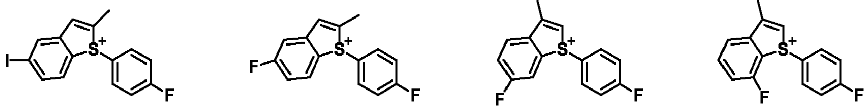

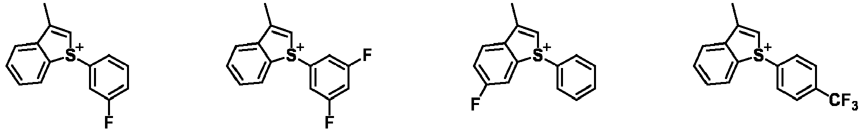

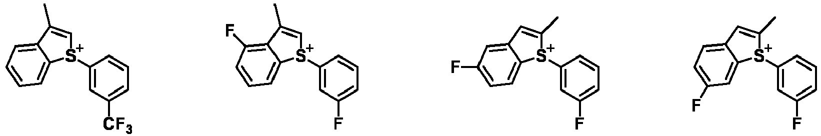

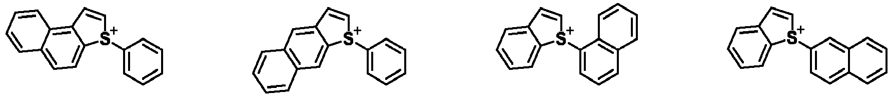

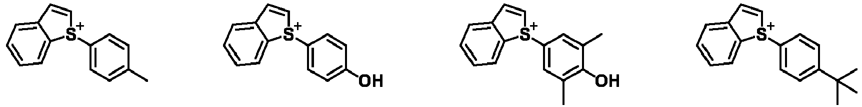

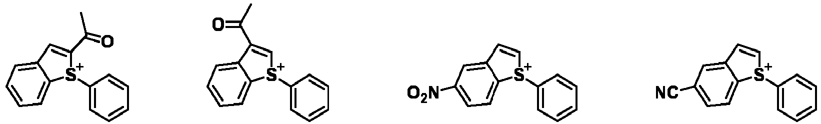

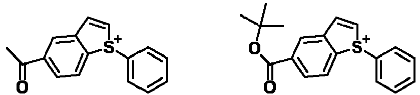

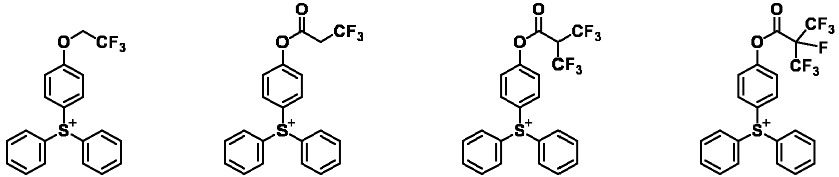

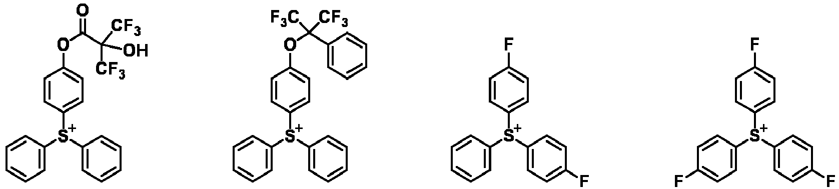

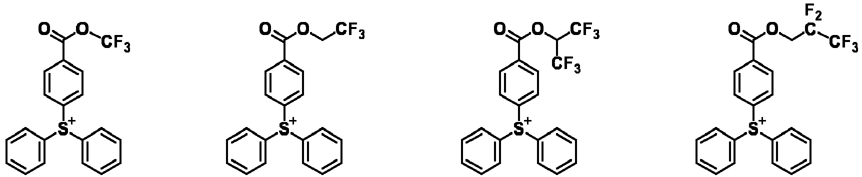

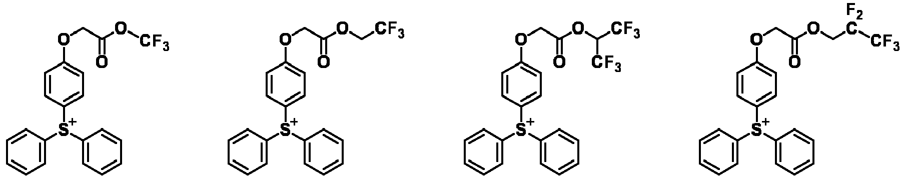

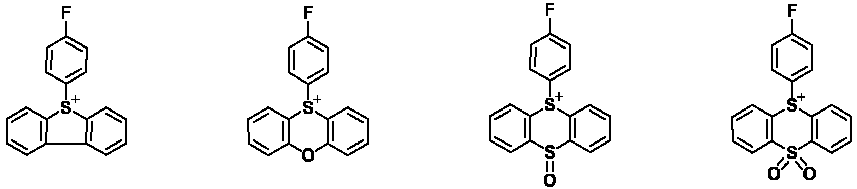

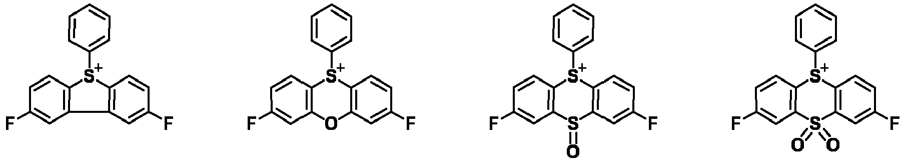

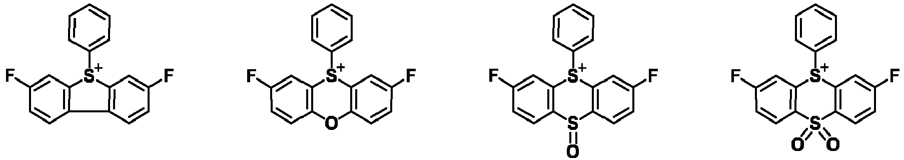

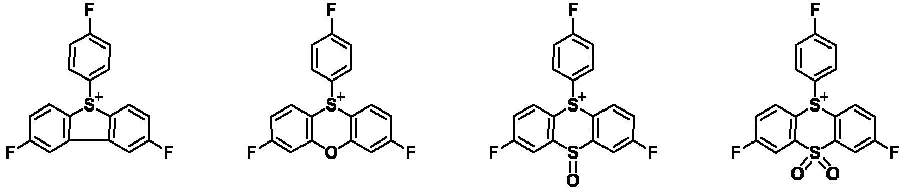

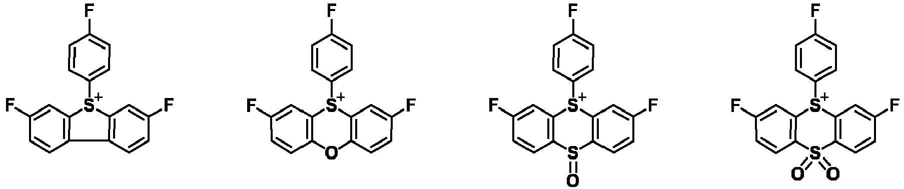

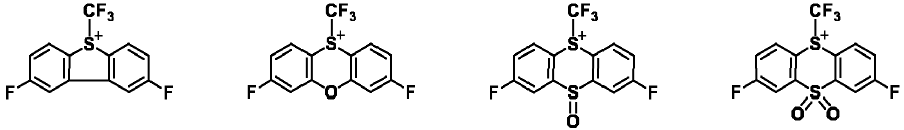

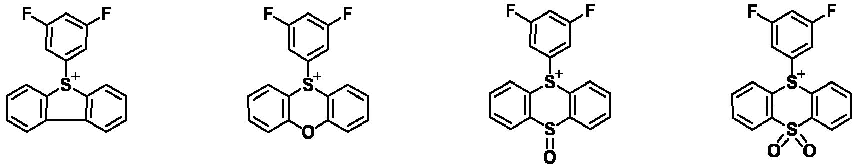

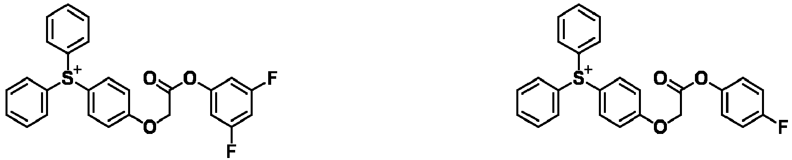

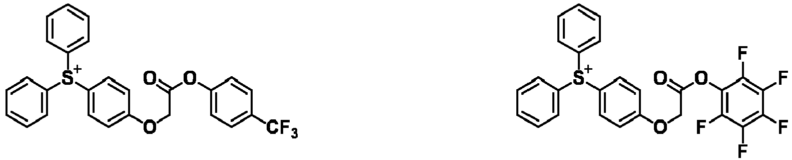

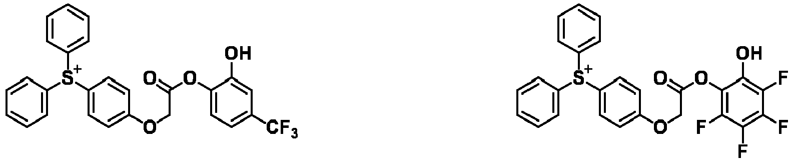

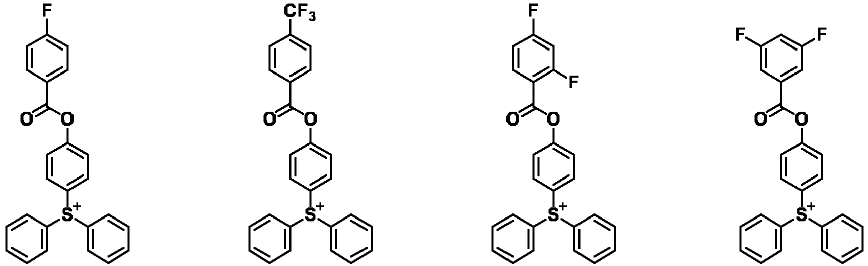

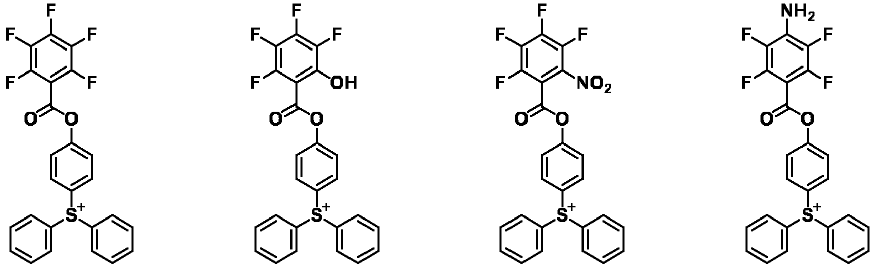

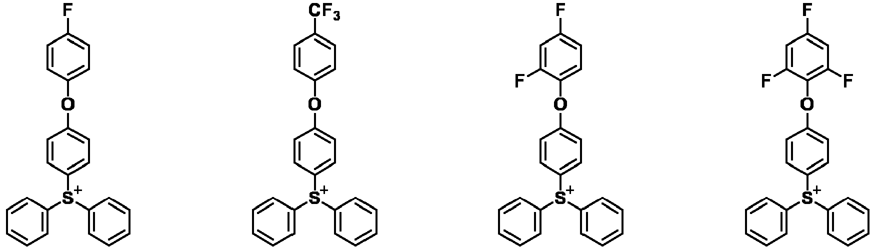

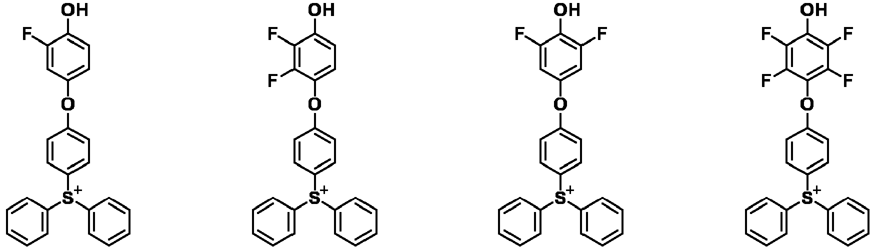

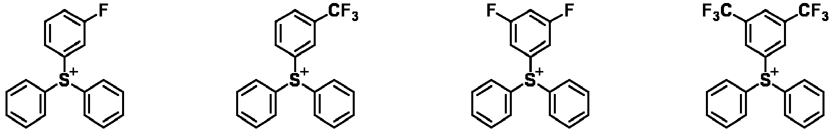

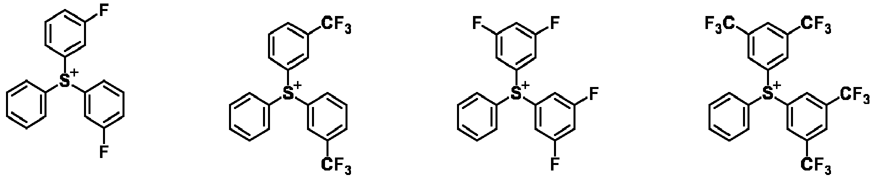

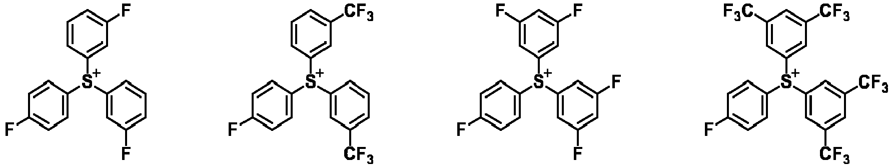

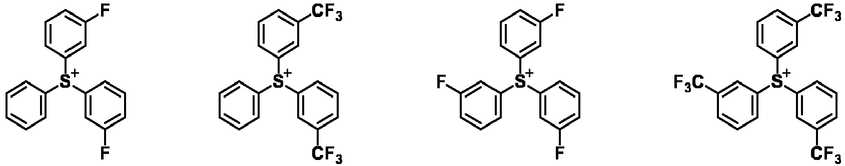

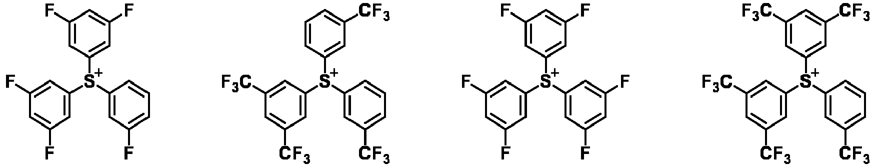

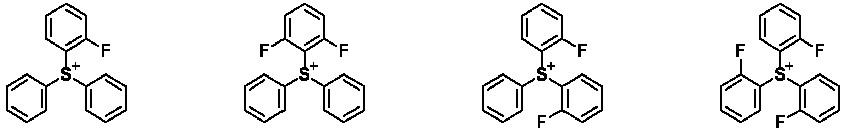

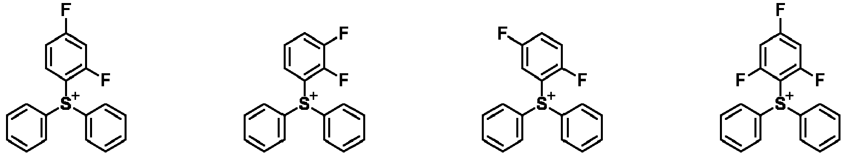

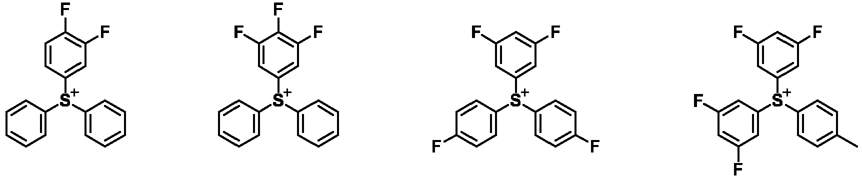

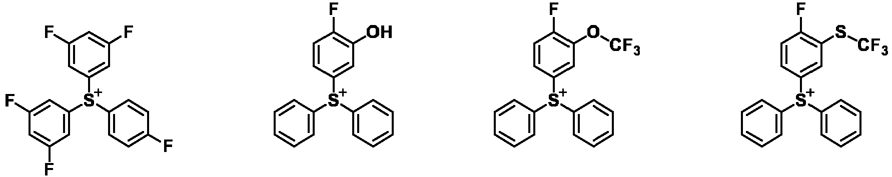

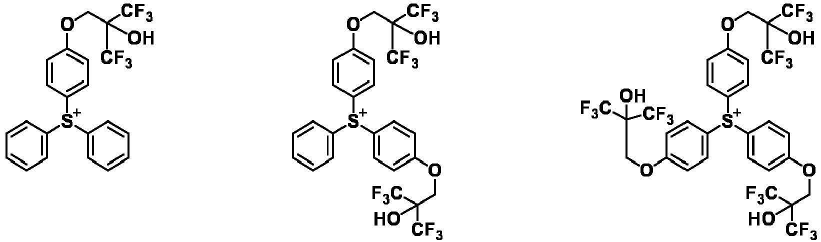

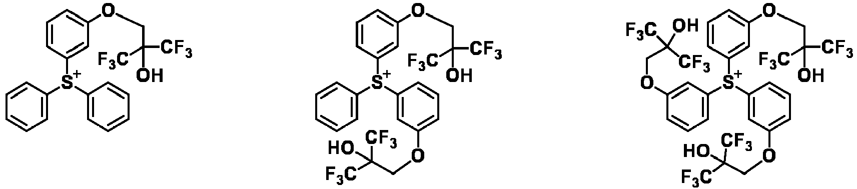

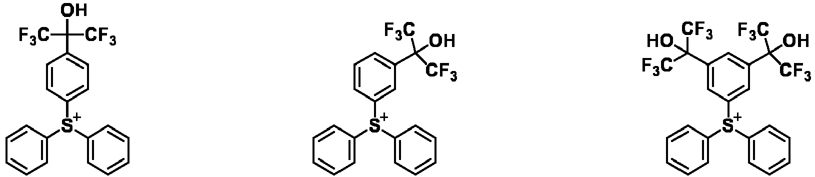

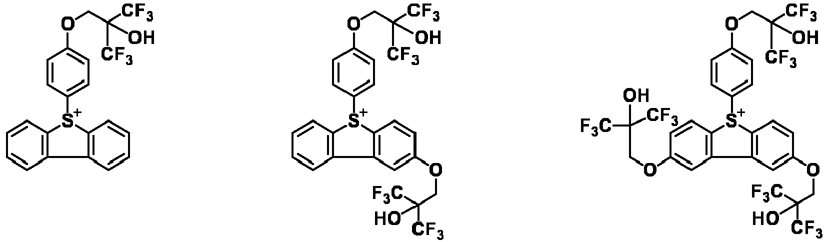

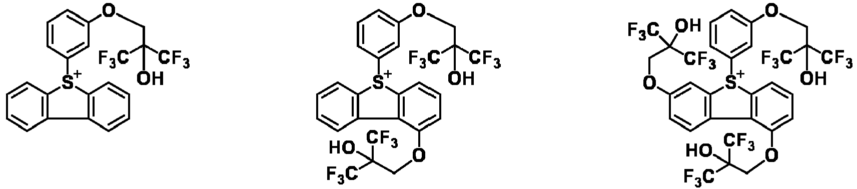

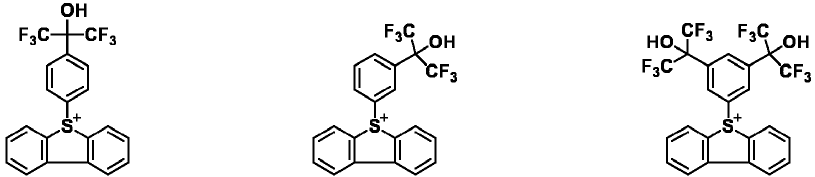

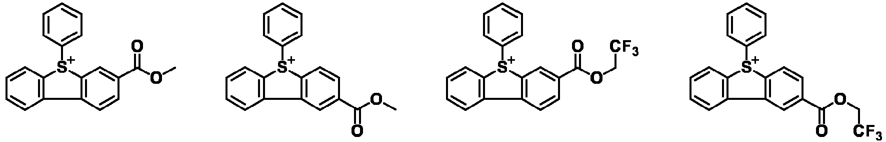

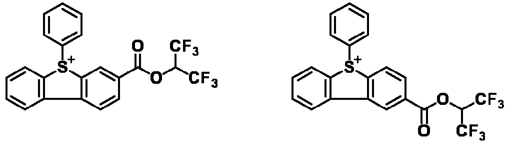

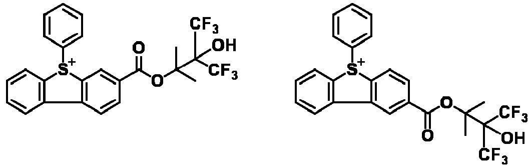

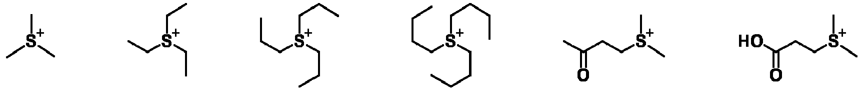

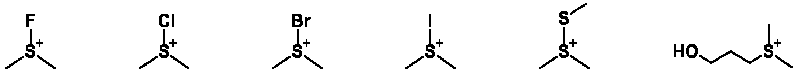

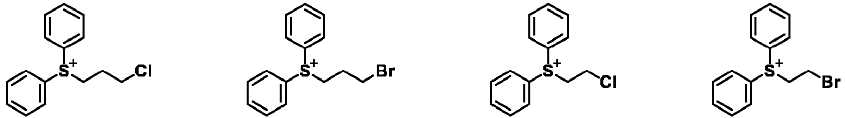

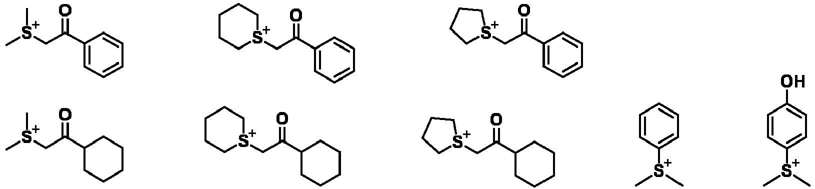

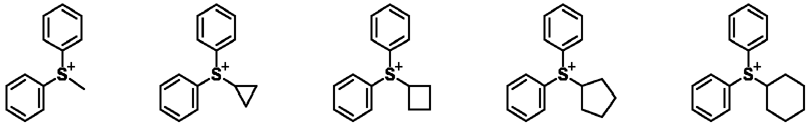

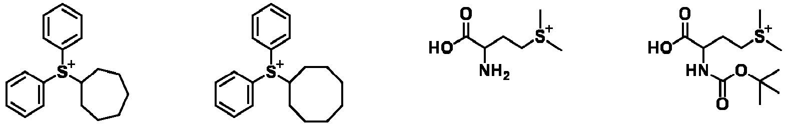

Examples of the iodonium cation in repeat units A11 to A13 are shown below, but not limited thereto.

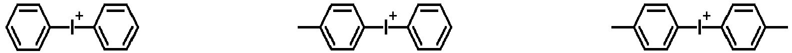

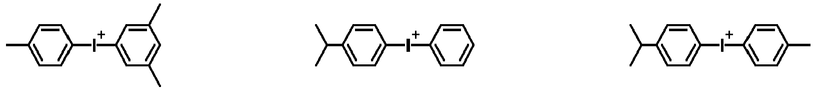

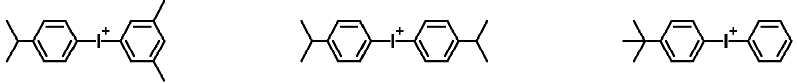

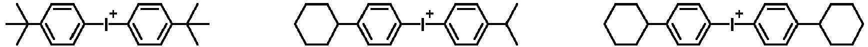

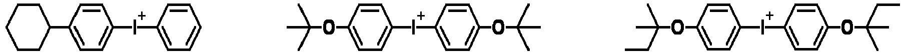

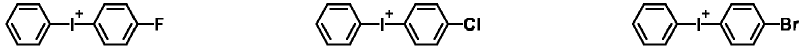

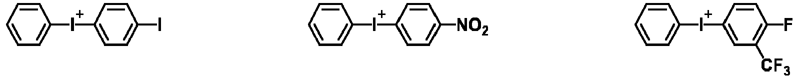

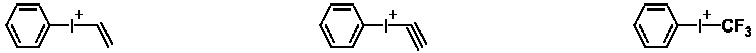

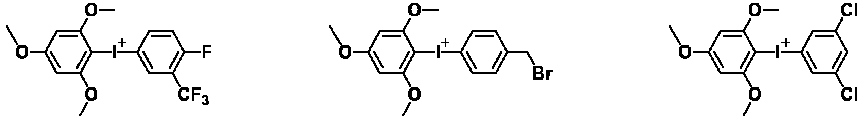

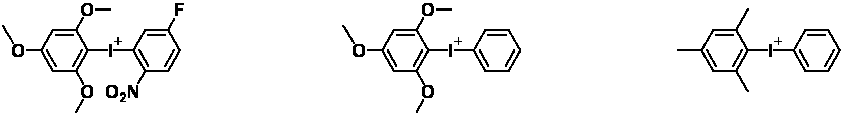

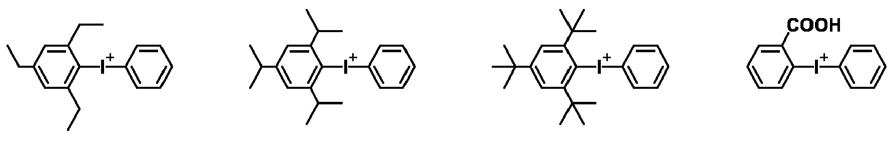

The repeat units A6 to A13 are capable of generating an acid upon receipt of high-energy radiation. The binding of these units into a polymer enables appropriate control of acid diffusion and formation of a pattern with reduced LER. Since the acid-generating unit is bound to a polymer, the phenomenon that acid volatilizes from the exposed region and re-deposits on the unexposed region during bake in vacuum is suppressed. This is effective for reducing LER and for suppressing unwanted negative-turning reaction in the unexposed region for thereby reducing pattern defects.

Each of repeat units A6 to A13 may be of one type or a combination of plural types.

For fine adjustment of properties of a resist film, the polymer may further comprise (meth)acrylate or other repeat units having an adhesive group such as lactone structure or hydroxy group other than phenolic hydroxy.

Examples of the (meth)acrylate unit having an adhesive group include repeat units having the following formulae (A14) to (A16), which are also referred to as repeat units A14 to A16. While these units do not exhibit acidity, they may be used as auxiliary units for providing adhesion to substrates or adjusting solubility.

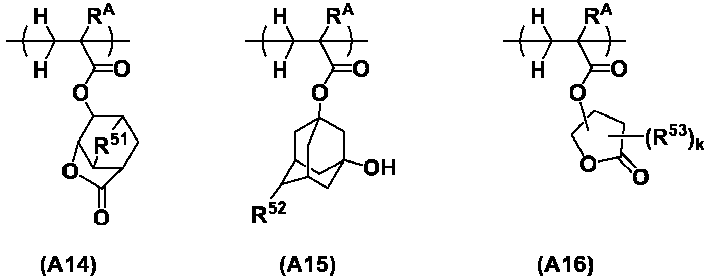

In formulae (A14) to (A16), R^{A} is each independently hydrogen, fluorine, methyl or trifluoromethyl. R⁵¹ is -O- or methylene. R⁵² is hydrogen or hydroxy. R⁵³ is a C₁-C₄ saturated hydrocarbyl group, and k is an integer of 0 to 3. Each of repeat units A14 to A16 may be of one type or a combination of plural types.

The content of repeat units A1 is preferably 5 to 70 mol%, more preferably 10 to 60 mol% based on the overall repeat units of the polymer for achieving the effect of accelerating negative-turning reaction. The content of repeat units A2 is preferably 30 to 95 mol%, more preferably 50 to 85 mol% of the overall repeat units of polymer for providing a high contrast between a negative-turning region which is exposed to high-energy radiation and a non-negative-turning region which is not exposed to high-energy radiation, for achieving a high resolution. The content of repeat units A3 to A5 is preferably 0 to 30 mol%, more preferably 3 to 20 mol% based on the overall repeat units of the polymer for achieving the effect of enhancing etch resistance. The polymer may contain 0 to 30 mol%, preferably 0 to 20 mol% of other repeat units.

In the embodiment wherein the polymer does not contain repeat units A6 to A13, the content of repeat units A2 is preferably 25 to 95 mol%, more preferably 40 to 85 mol%, the content of repeat units A3 to A5 is preferably 0 to 30 mol%, more preferably 3 to 20 mol%, and the content of repeat units A5 is preferably 5 to 70 mol%, more preferably 10 to 60 mol%, based on the overall repeat units of the polymer. Other repeat units may be incorporated in a content of 0 to 30 mol%, preferably 0 to 20 mol%.

In the other embodiment wherein the polymer contains repeat units A6 to A13, the content of repeat units A2 is preferably 25 to 94.5 mol%, more preferably 36 to 85 mol%, the content of repeat units A3 to A5 is preferably 0 to 30 mol%, more preferably 3 to 20 mol%, the content of repeat units A5 is preferably 5 to 70 mol%, more preferably 10 to 60 mol%, the total content of repeat units A1 to A5 is preferably 60 to 99.5 mol%, and the content of repeat units A6 to A13 is preferably 0.5 to 20 mol%, more preferably 1 to 10 mol%, based on the overall repeat units of the polymer. Other repeat units may be incorporated in a content of 0 to 30 mol%, preferably 0 to 20 mol%.

The polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to WO 2006/121096, JP-A 2008-102383, JP-A 2008-304590, and JP-A 2004-115630, for example.

### Chemically amplified negative resist composition

A further embodiment of the invention is a chemically amplified negative resist composition comprising (A) a base polymer containing the polymer defined above.

The repeat units A1 to A5 preferably account for at least 60 mol%, more preferably at least 70 mol%, even more preferably at least 80 mol% based on the overall repeat units of the polymer. This ensures that the chemically amplified negative resist composition has satisfactory properties.

The polymer should preferably have a Mw of 1,000 to 50,000, and more preferably 2,000 to 20,000. A Mw of at least 1,000 eliminates the risk that pattern features are rounded at their top to invite degradations of resolution and LER. A Mw of up to 50,000 eliminates the risk that LER increases when a pattern with a line width of up to 100 nm is formed. As used herein, Mw is measured by GPC versus polystyrene standards using tetrahydrofuran (THF) or dimethylformamide (DMF).

The polymer preferably has a narrow molecular weight distribution or dispersity (Mw/Mn) of 1.0 to 2.0, more preferably 1.0 to 1.8. A polymer with such a narrow dispersity eliminates the risk that foreign particles are left on the pattern after development and the pattern profile is aggravated.

The base polymer may contain two or more polymers which are different in compositional ratio, Mw and/or Mw/Mn. A mixture of a polymer free of repeat units A6 to A13 and a polymer containing repeat units A6 to A13 is acceptable. In the chemically amplified negative resist composition, the amount of the polymer free of repeat units A6 to A13 is preferably 2 to 5,000 parts by weight, more preferably 10 to 1,000 parts by weight per 100 parts by weight of the polymer containing repeat units A6 to A13.

It is now considered that the chemically amplified negative resist composition is used in the fabrication of masks. The lithography of advanced generation implies a coating thickness of up to 150 nm, preferably up to 100 nm. The base polymer on which the chemically amplified negative resist composition is based should preferably have a dissolution rate in alkaline developer (typically 2.38 wt% aqueous solution of tetramethylammonium hydroxide (TMAH)) of up to 80 nm/sec, more preferably up to 50 nm/sec for forming small-size patterns because a strong development process is often employed in order to minimize defects resulting from resist residues. In an example where LSI chips are manufactured from a wafer using the chemically amplified negative resist composition in combination with the EUV lithography, the coating thickness is often up to 100 nm as viewed from the need to form a pattern of narrow lines with a width of up to 50 nm.

Since a thin film has a risk that the pattern can be degraded by development, the polymer should preferably have a dissolution rate of up to 80 nm/sec, more preferably up to 50 nm/sec.

### (B) Acid generator

The negative resist composition may comprise an acid generator as component (B). The acid generator used herein may be any compound (PAG) capable of generating an acid in response to actinic ray or radiation. The PAG used herein is not particularly limited as long as it can generate an acid upon exposure to high-energy radiation. Suitable PAGs include sulfonium salts, iodonium salts, sulfonyldiazomethane, N-sulfonyloxyimide, and oxime-O-sulfonate acid generators.

Suitable PAGs include nonafluorobutane sulfonate, partially fluorinated sulfonates described in JP-A 2012-189977, paragraphs [0247]-[0251], partially fluorinated sulfonates described in JP-A 2013-101271, paragraphs [0261]-[0265], and those described in JP-A 2008-111103, paragraphs [0122]-[0142] and JP-A 2010-215608, paragraphs [0080]-[0081]. Among others, arylsulfonate and alkanesulfonate type PAGs are preferred because they generate acids having an appropriate strength to promote the reaction of crosslinker (C) (to be described later) with base polymer (A).

In order that the effect of improving LER is exerted by combining the acid generator with quencher (E) (to be described later), the acid generator preferably generates an acid having a pKa value of -3.0 or larger, more preferably in the range of -3.0 to 2.0, even more preferably in the range of -2.0 to 1.5.

A salt compound having an anion of the structure shown below is preferred as the acid generator. Examples of the pairing cation include the above-illustrated examples of the sulfonium cation in repeat units A7 to A9 and the above-illustrated examples of the iodonium cation in repeat units A11 to A13.

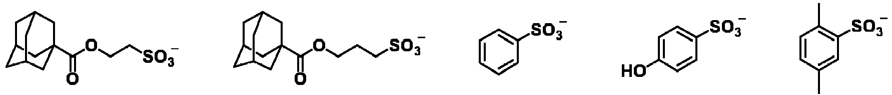

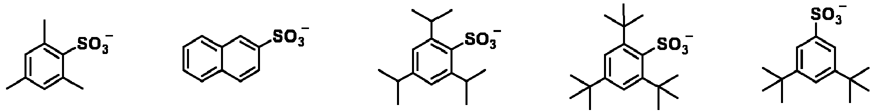

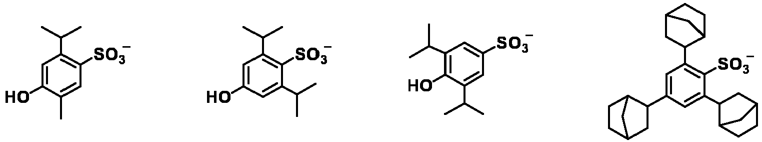

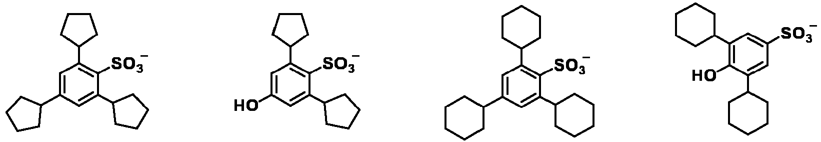

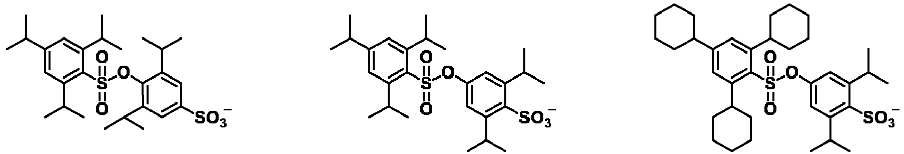

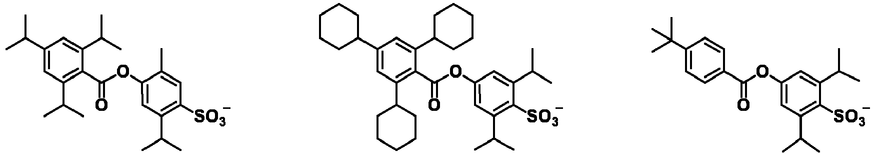

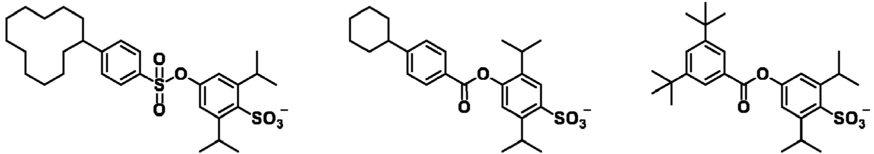

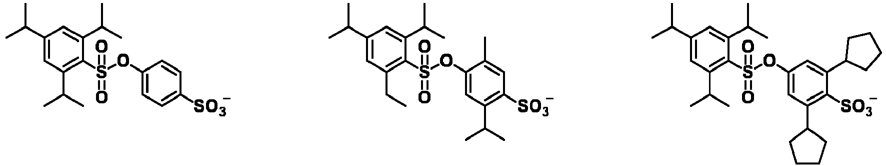

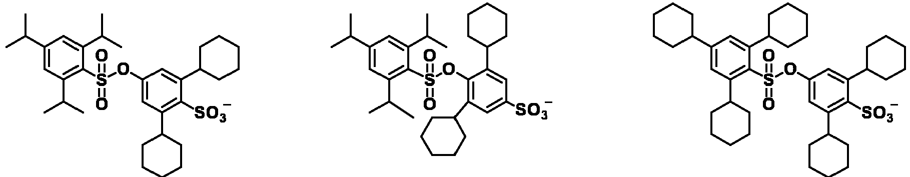

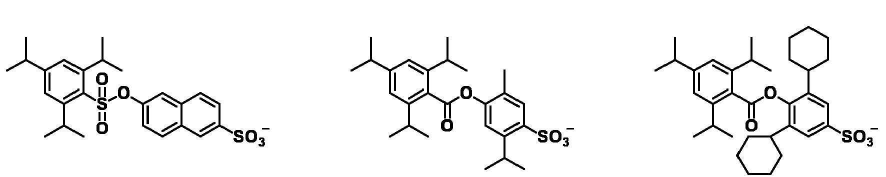

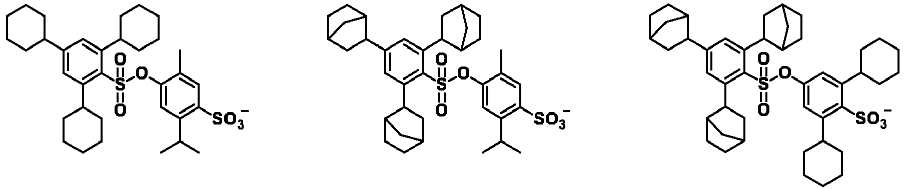

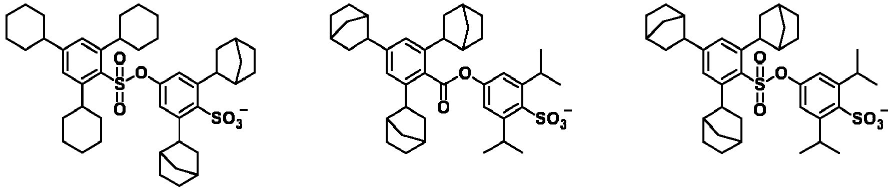

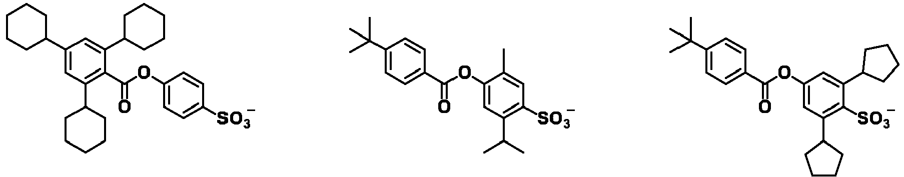

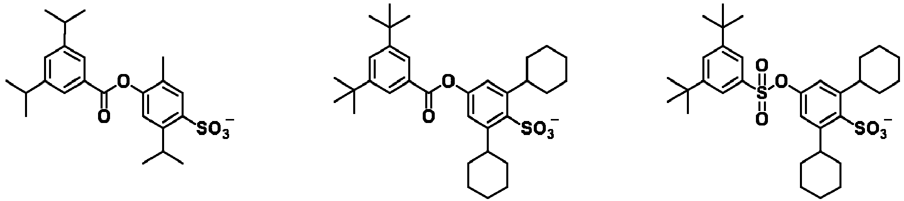

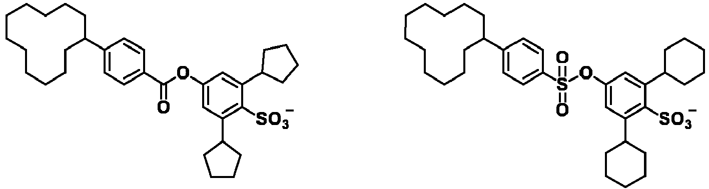

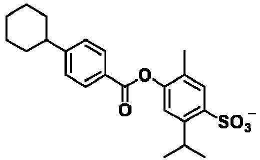

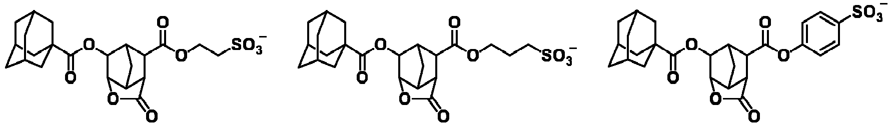

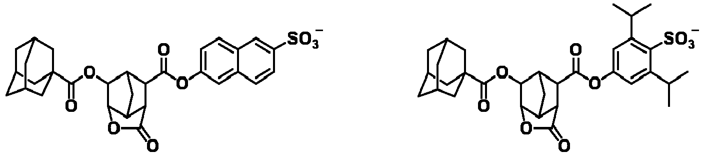

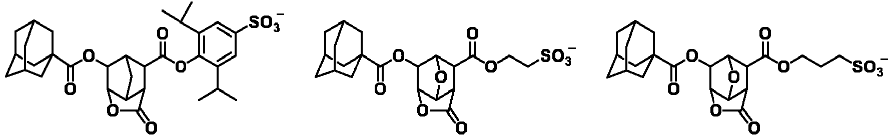

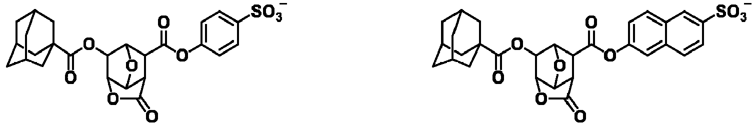

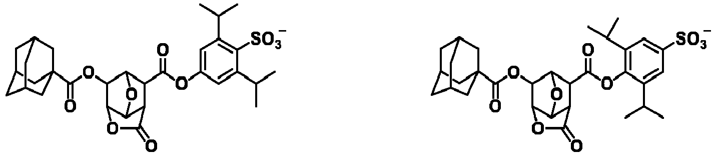

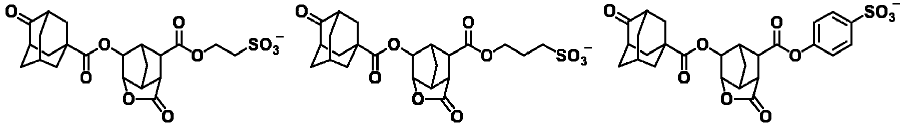

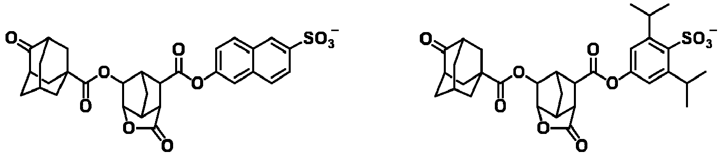

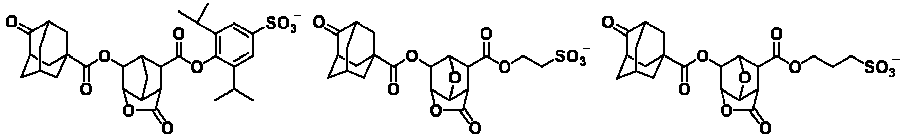

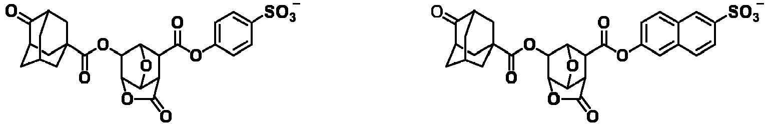

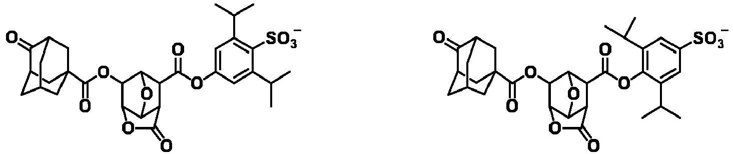

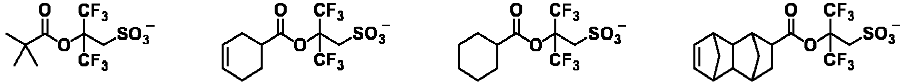

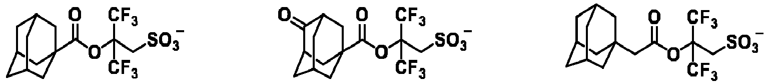

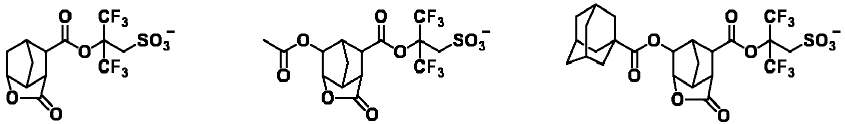

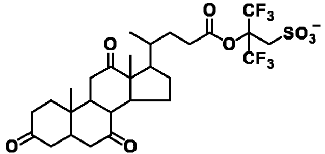

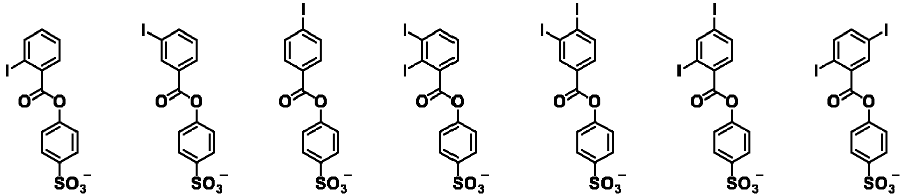

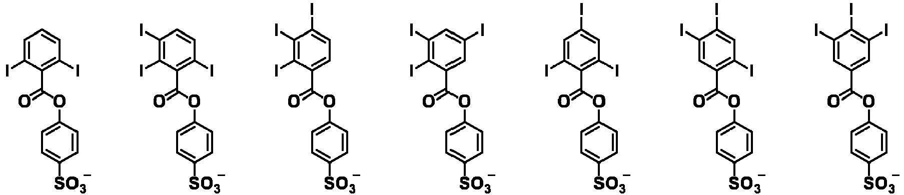

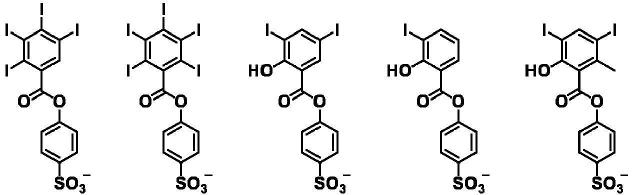

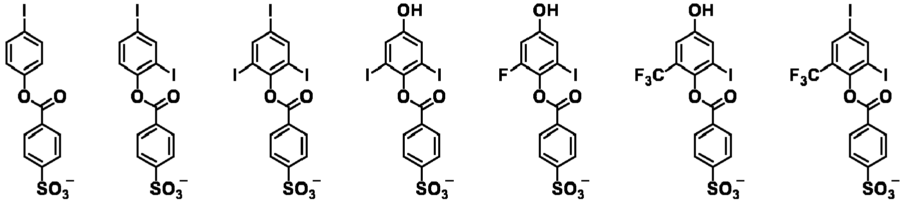

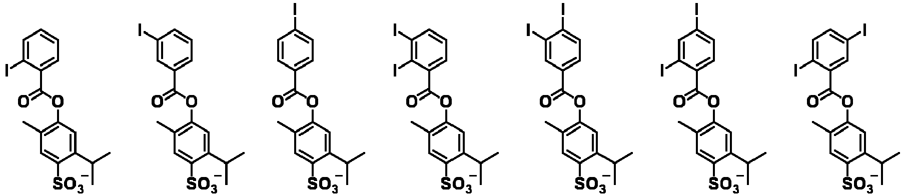

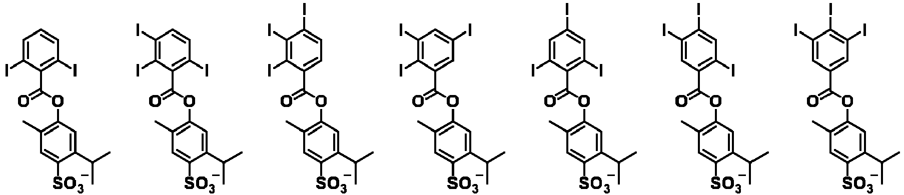

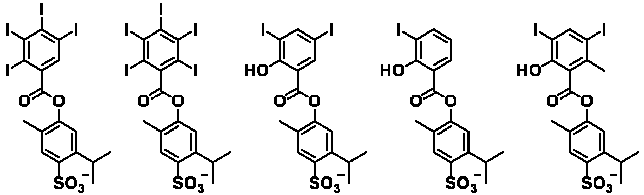

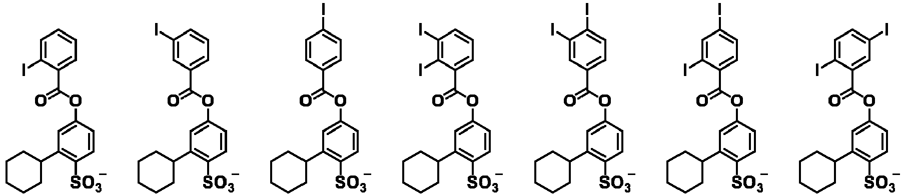

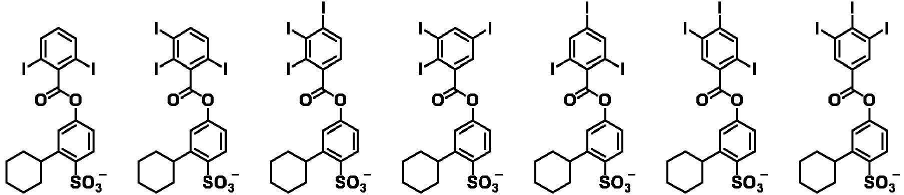

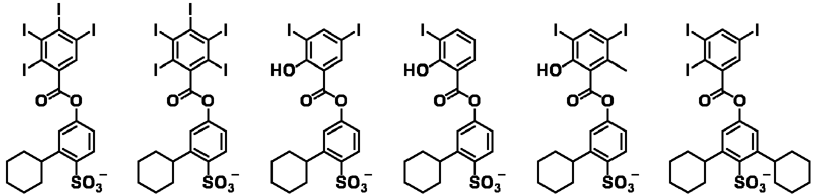

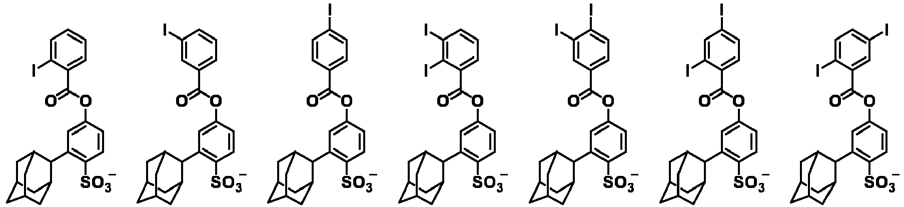

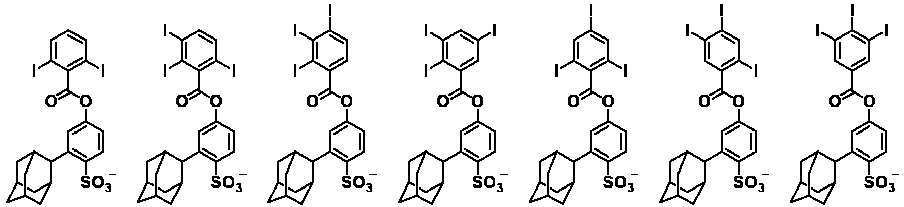

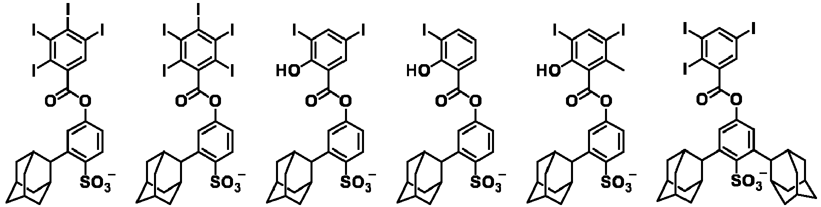

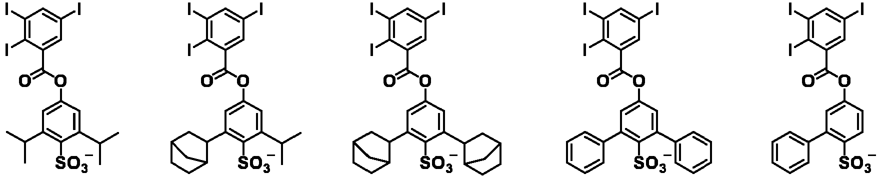

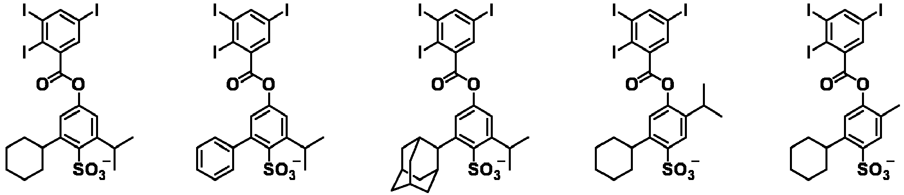

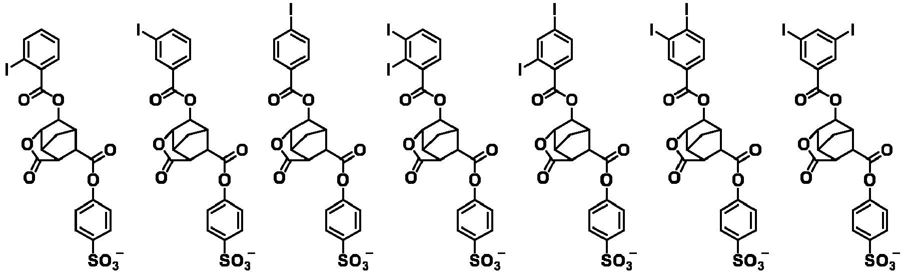

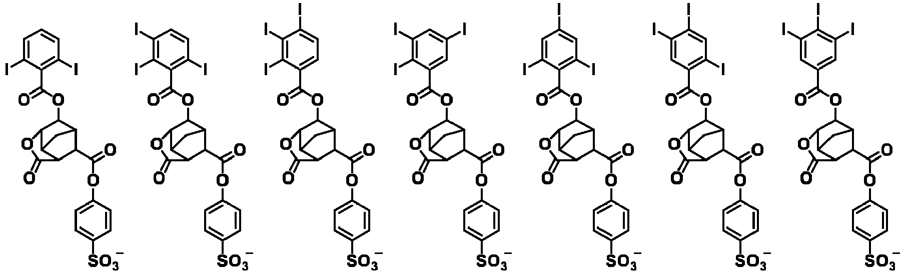

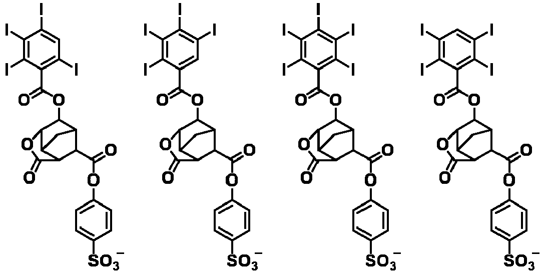

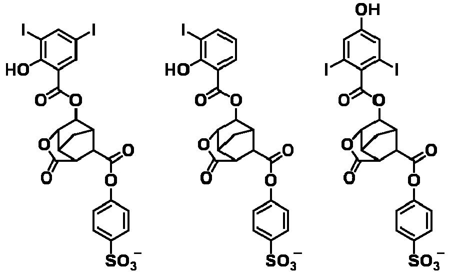

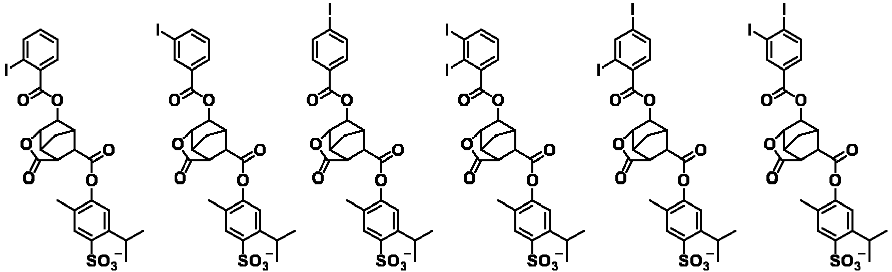

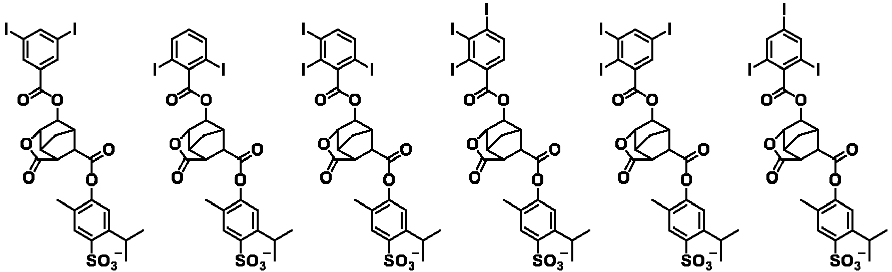

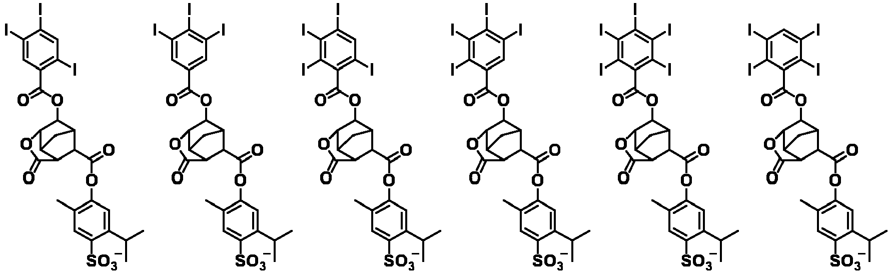

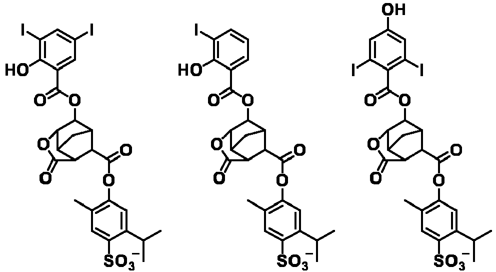

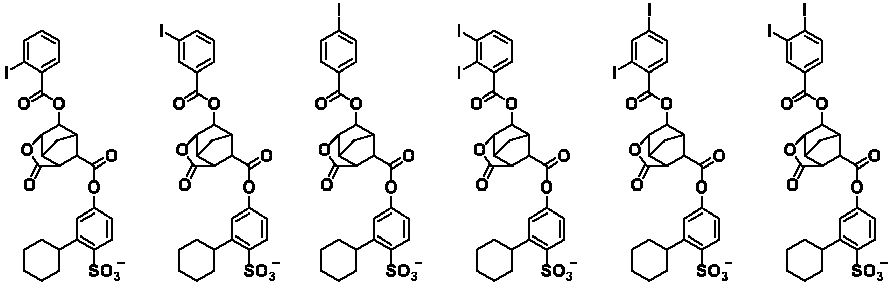

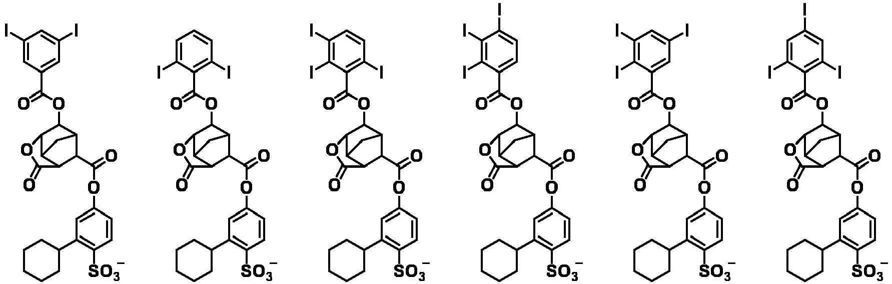

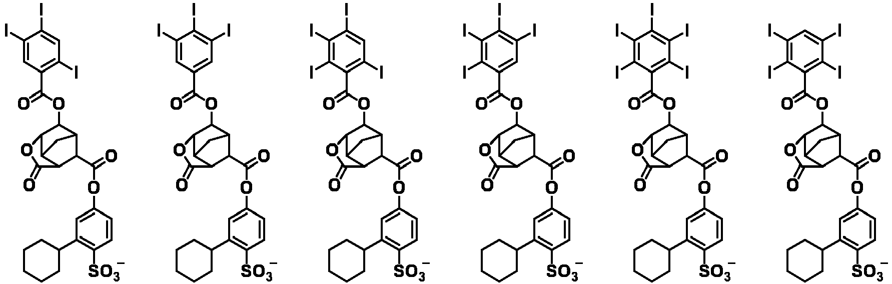

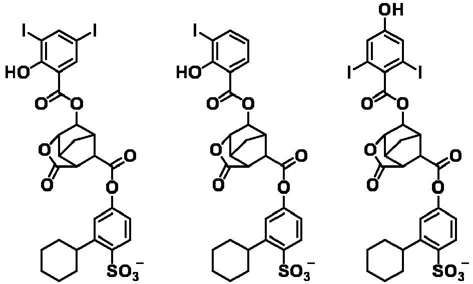

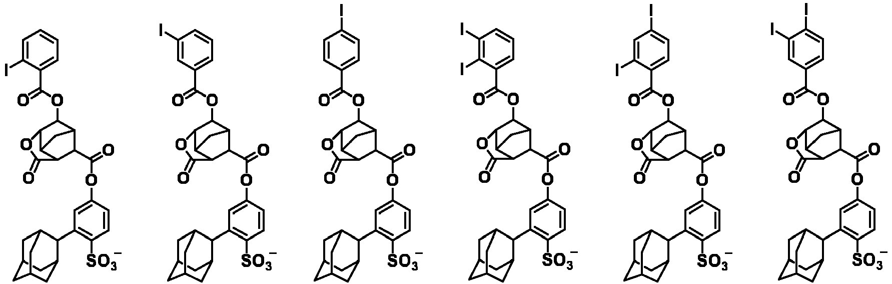

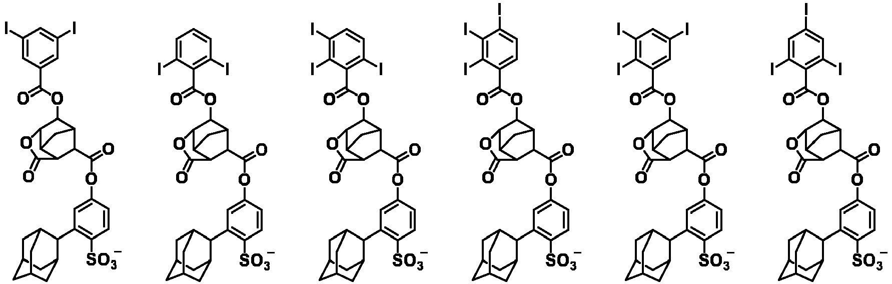

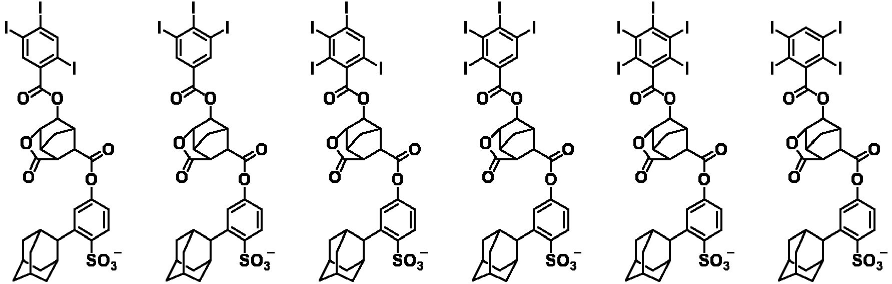

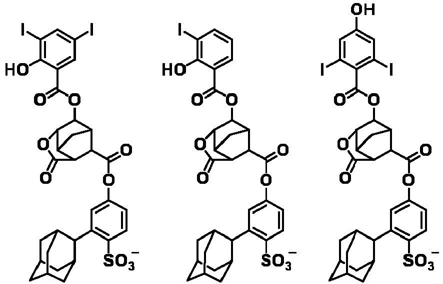

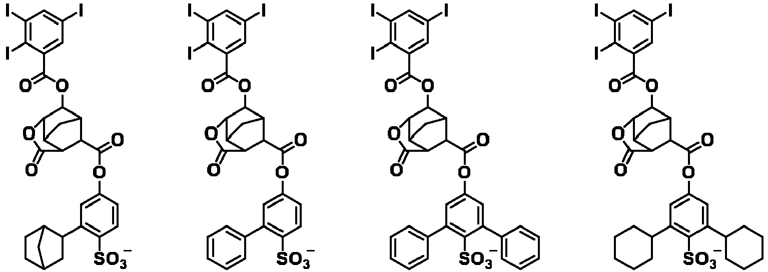

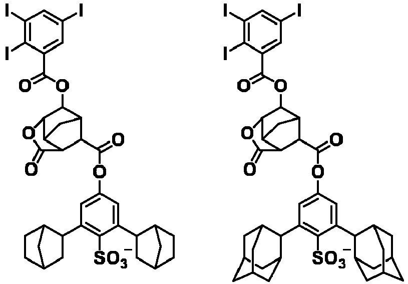

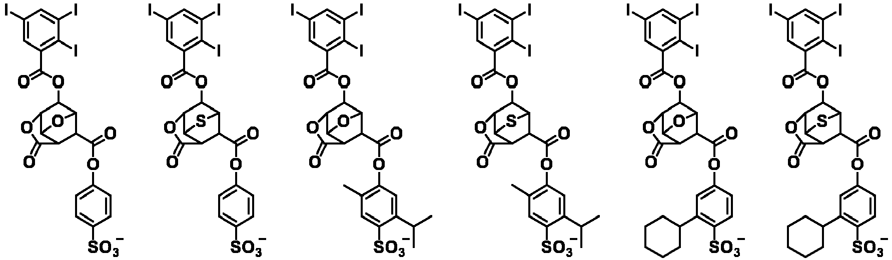

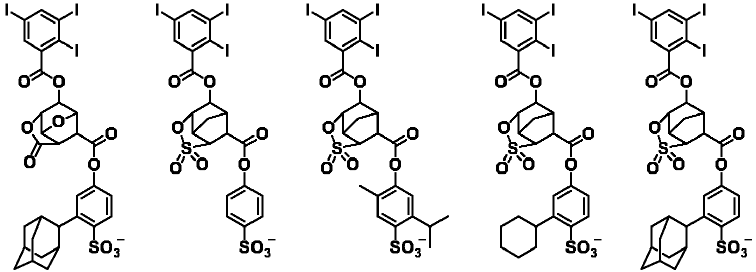

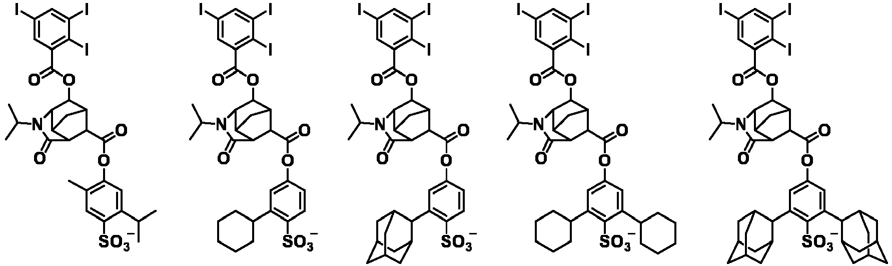

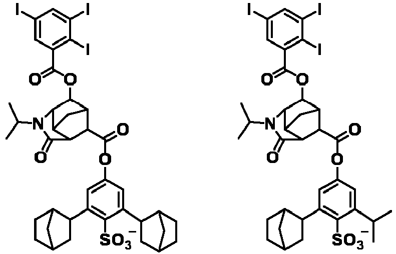

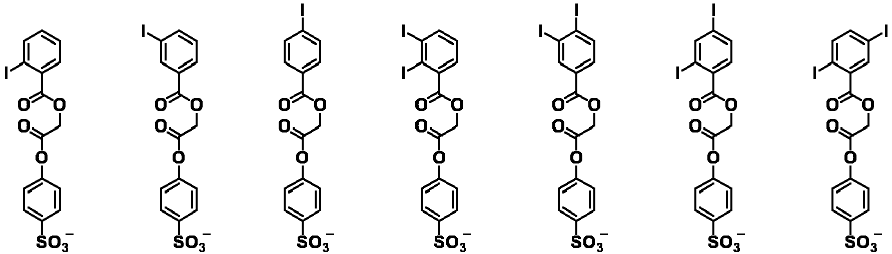

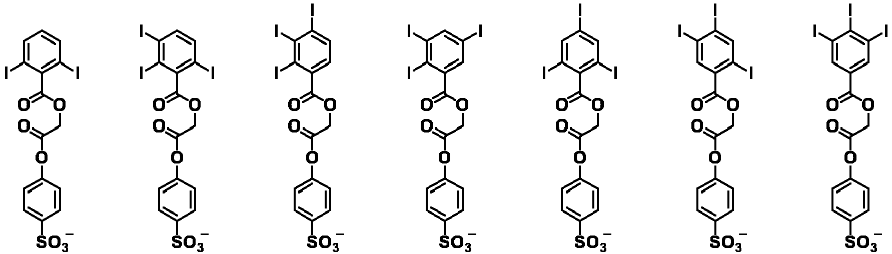

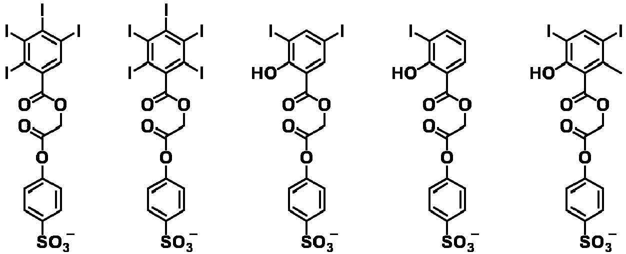

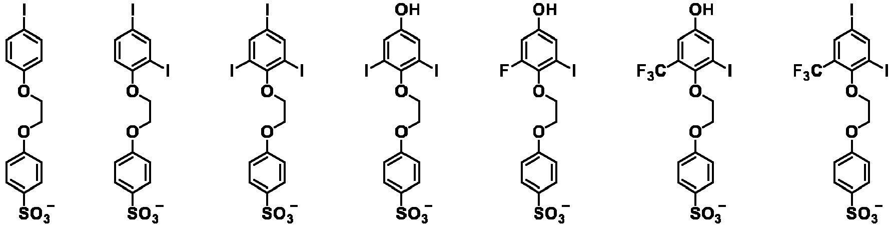

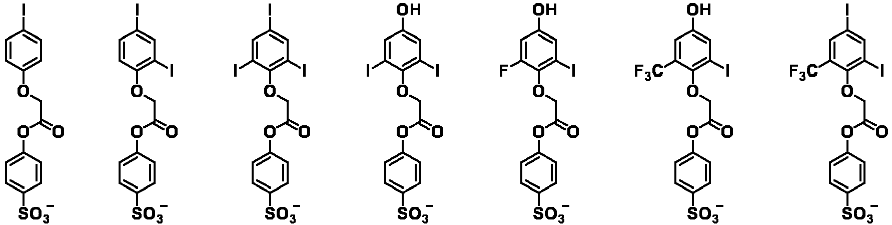

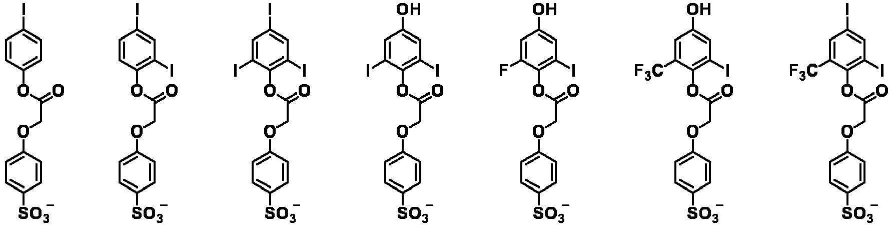

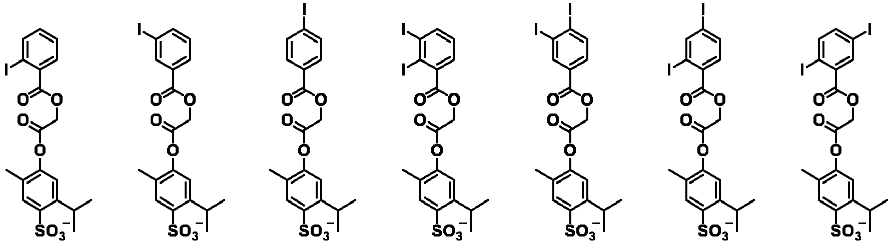

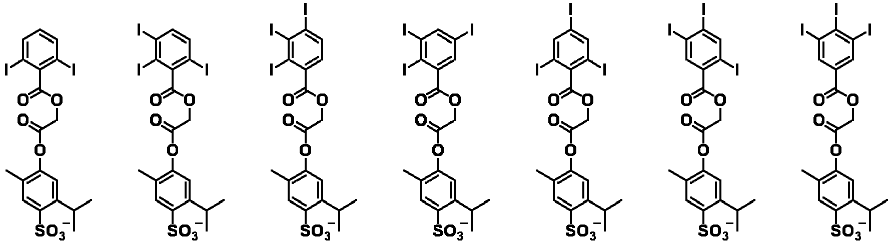

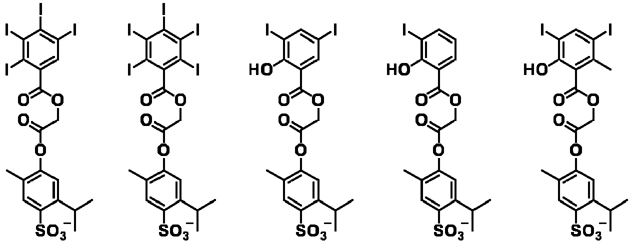

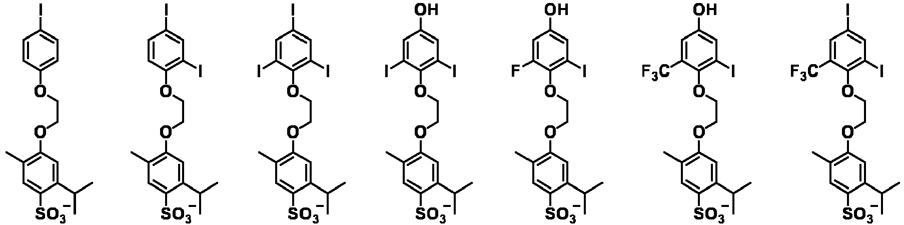

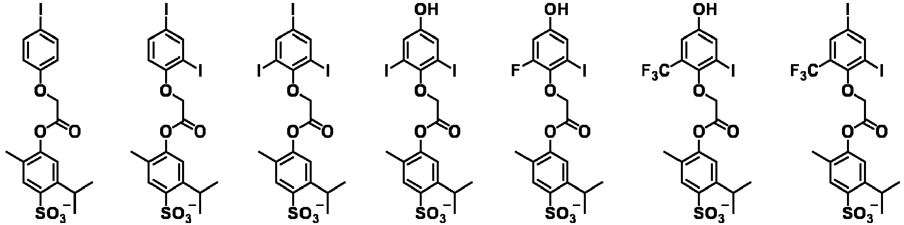

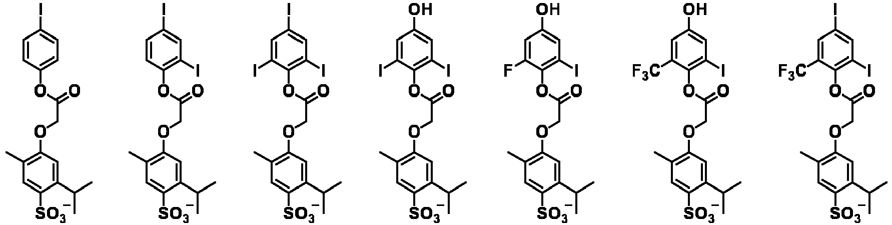

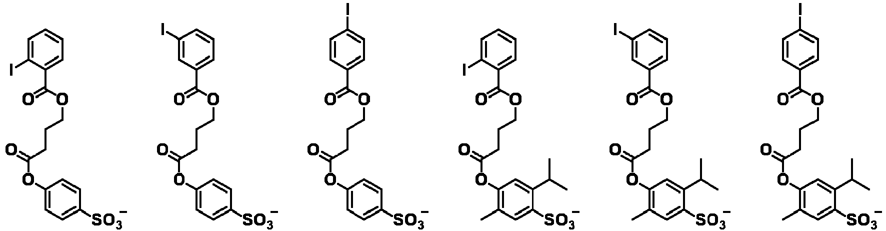

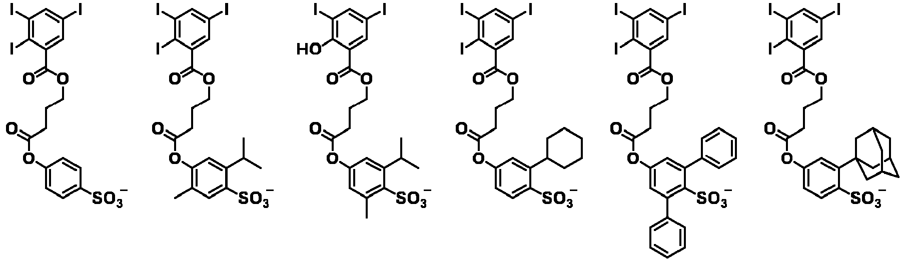

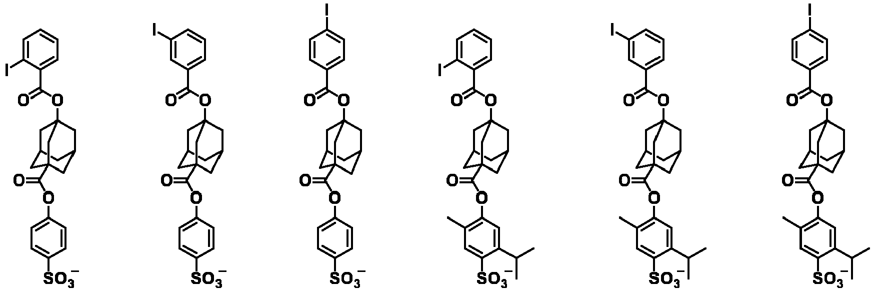

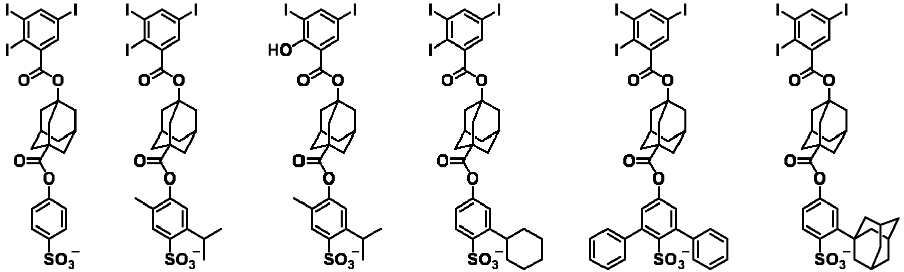

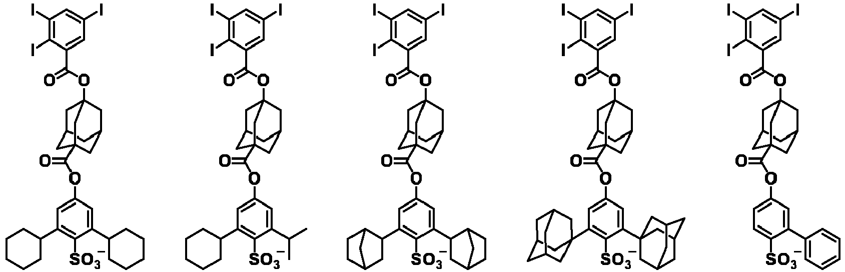

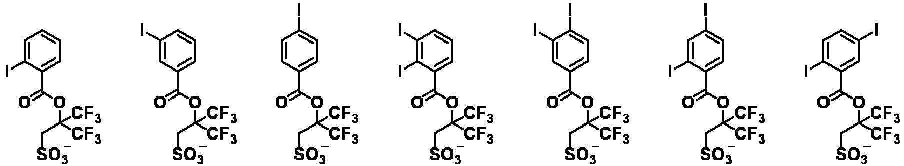

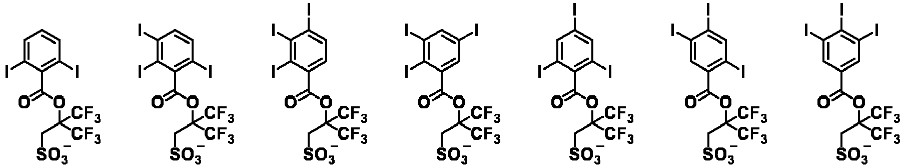

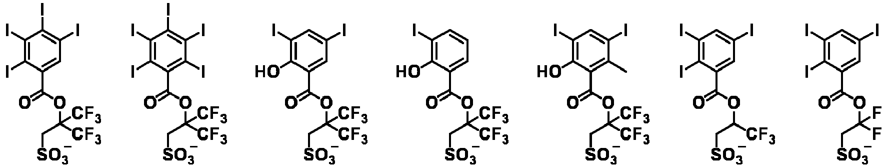

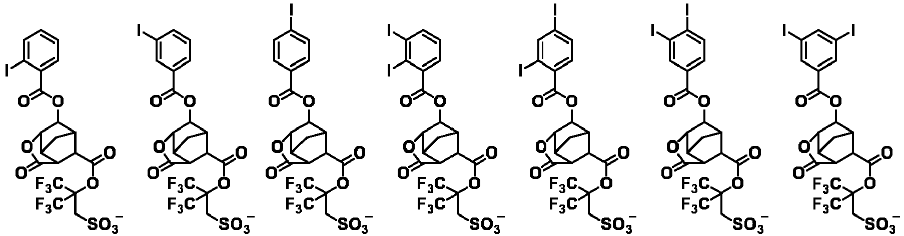

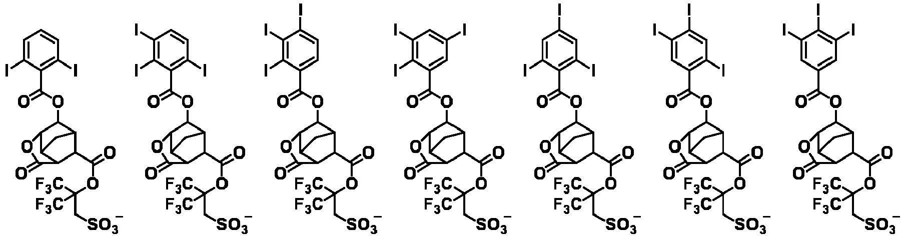

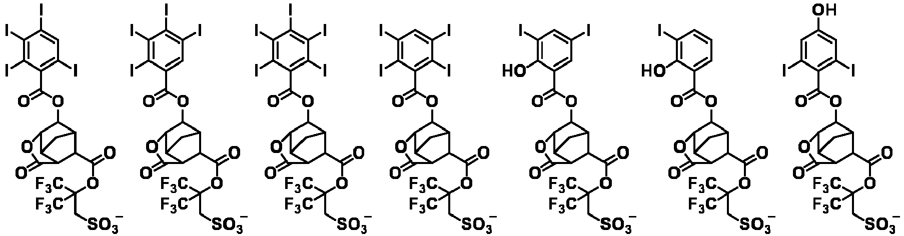

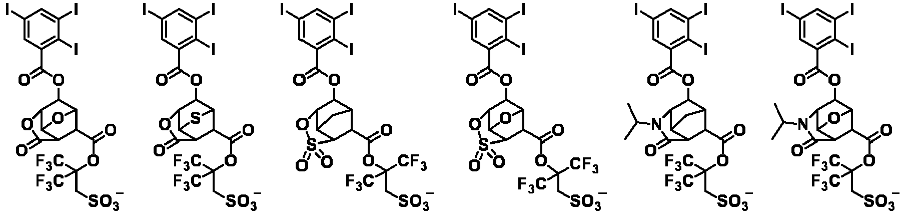

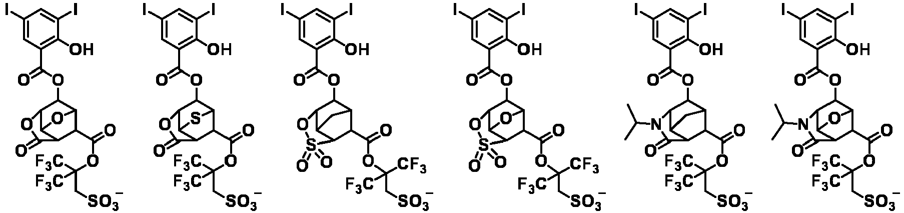

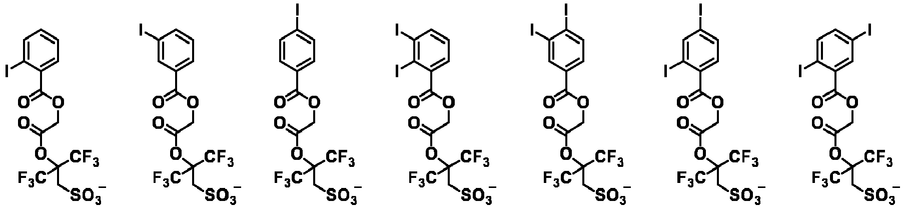

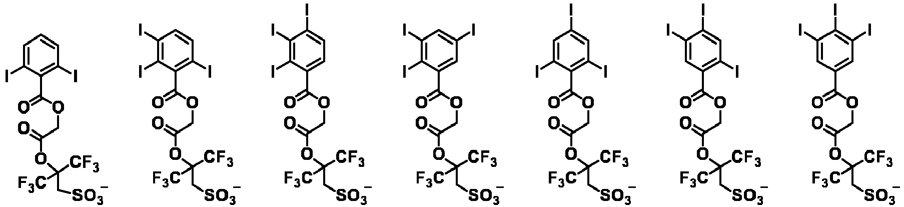

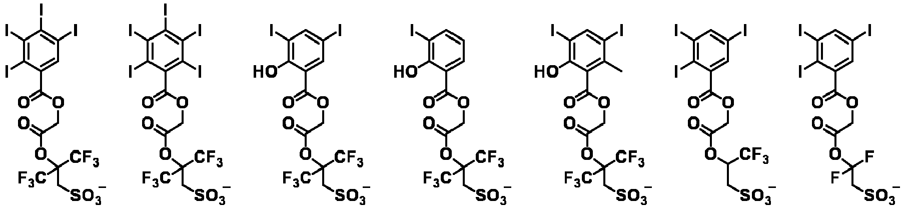

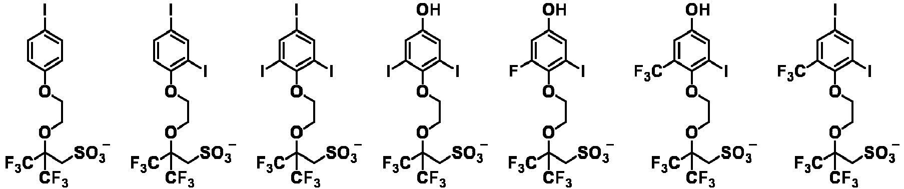

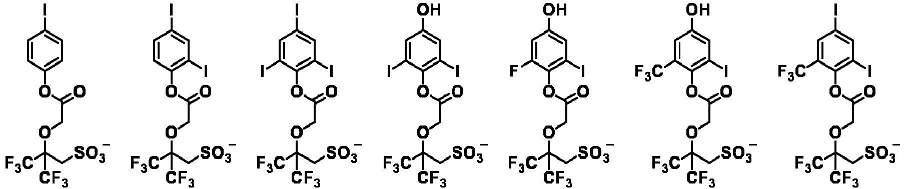

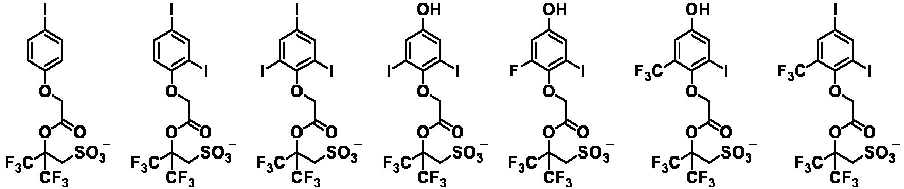

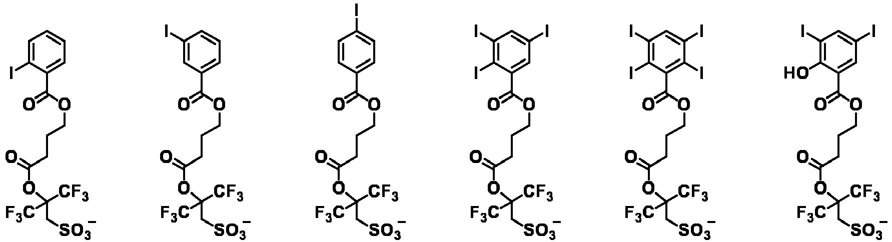

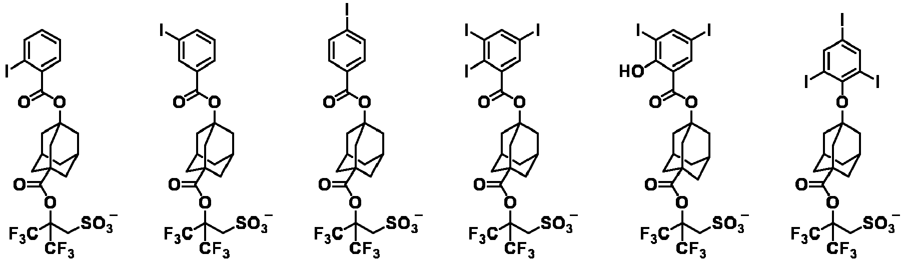

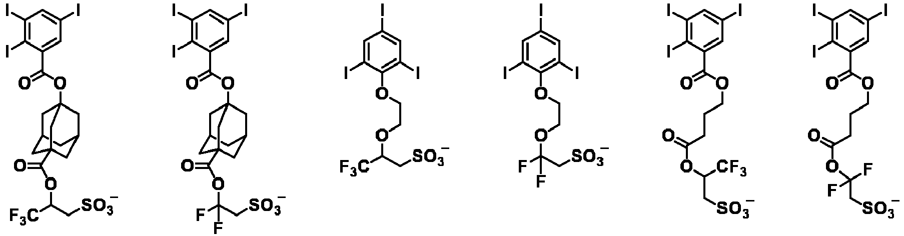

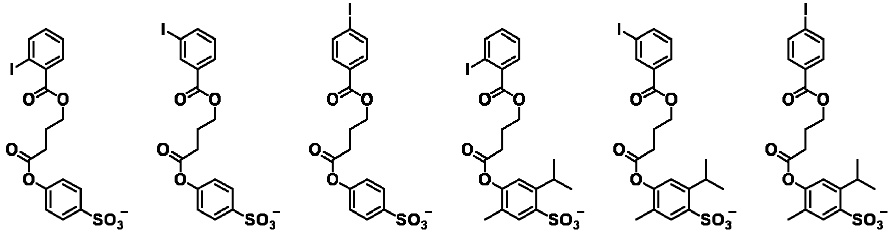

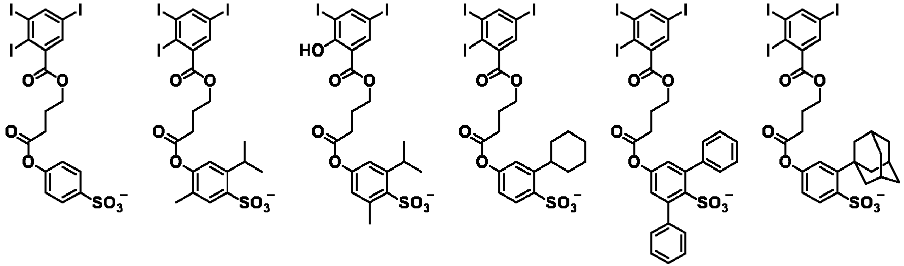

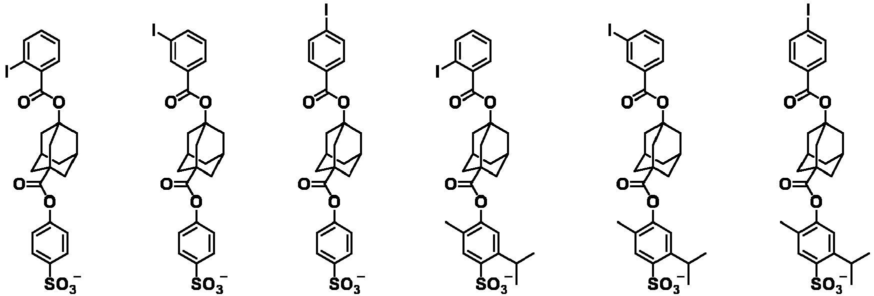

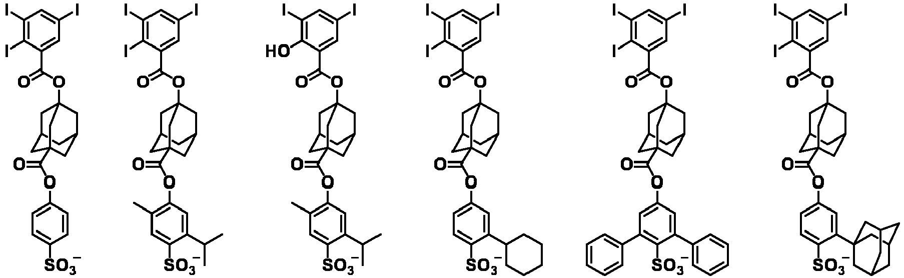

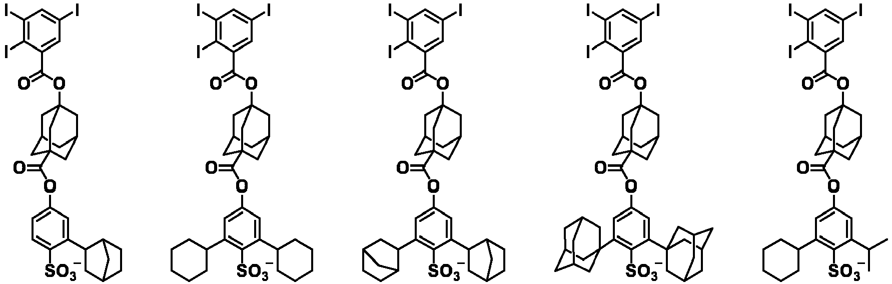

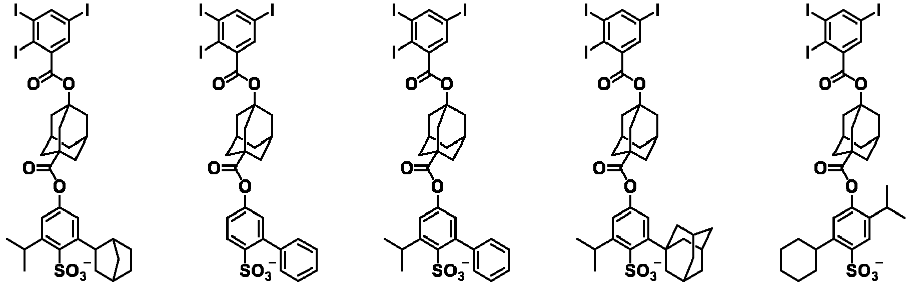

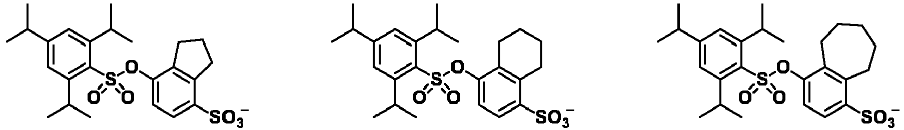

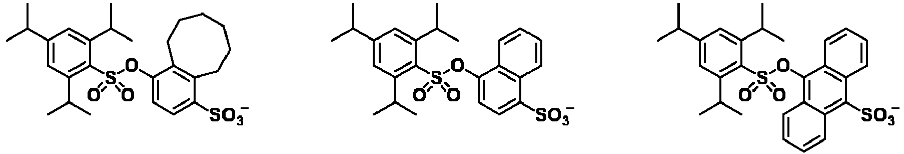

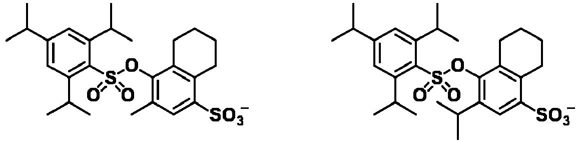

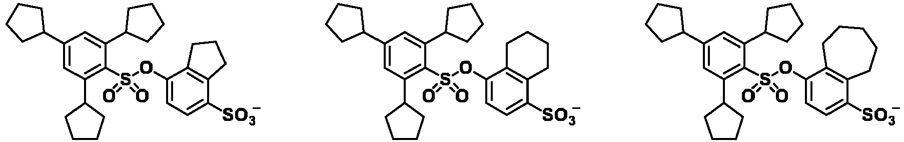

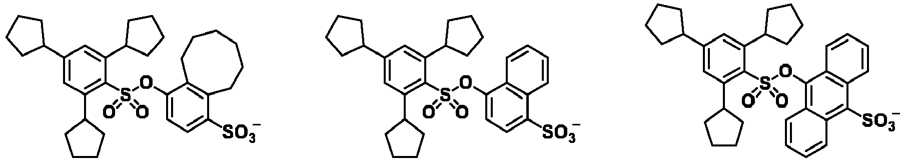

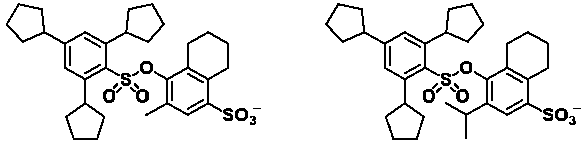

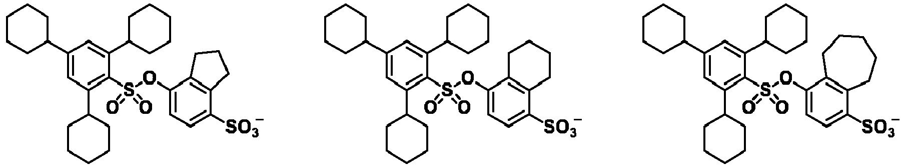

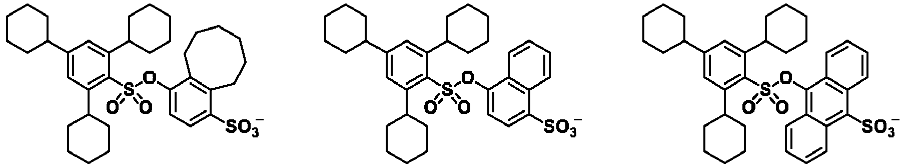

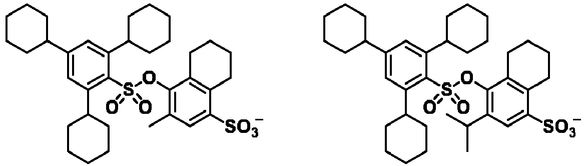

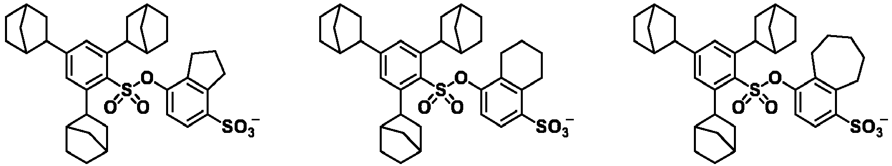

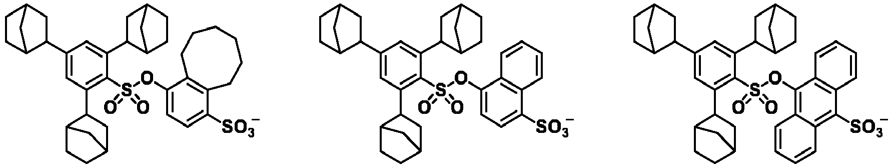

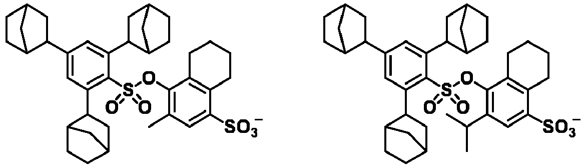

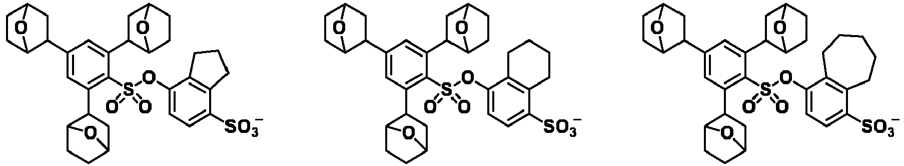

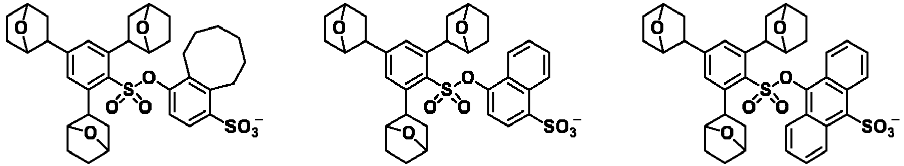

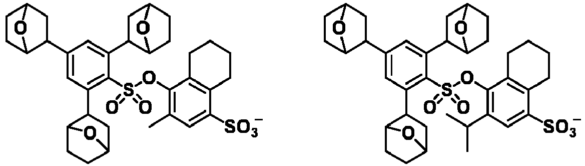

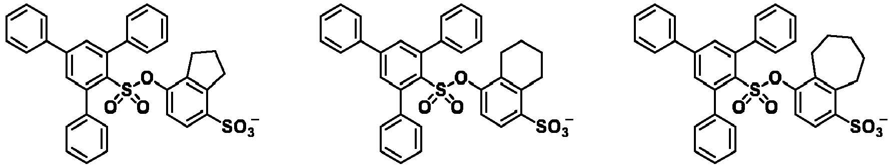

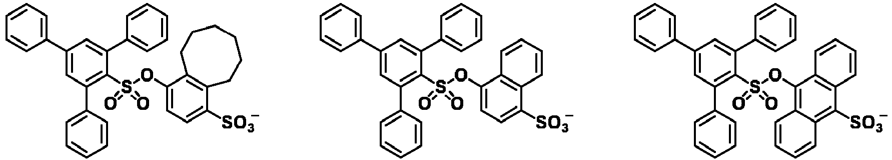

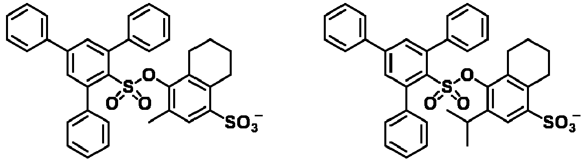

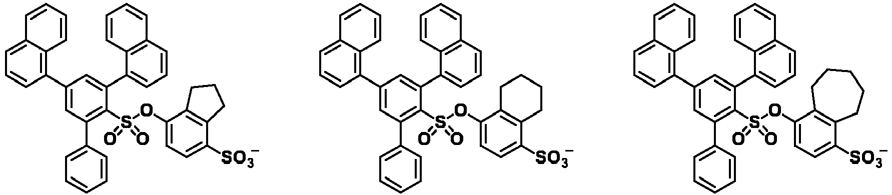

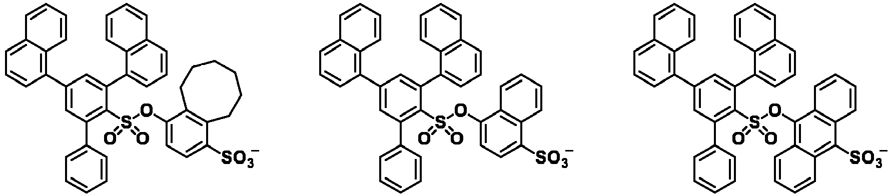

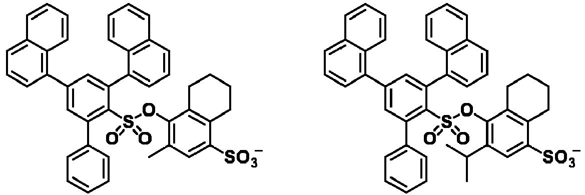

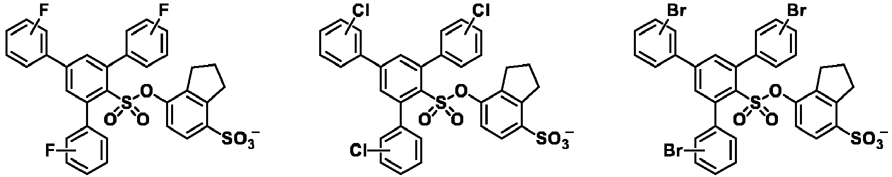

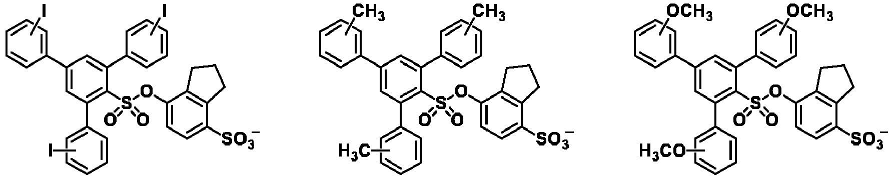

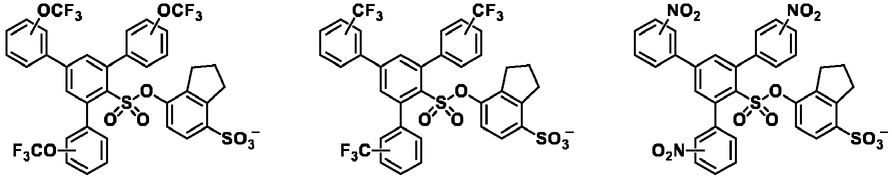

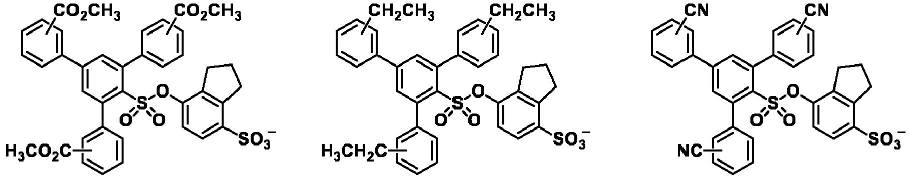

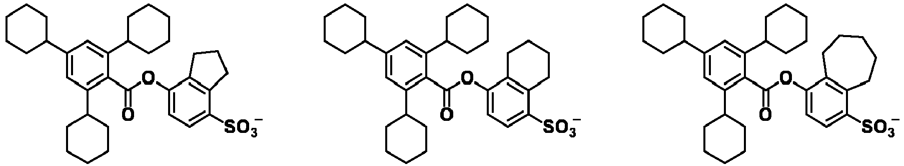

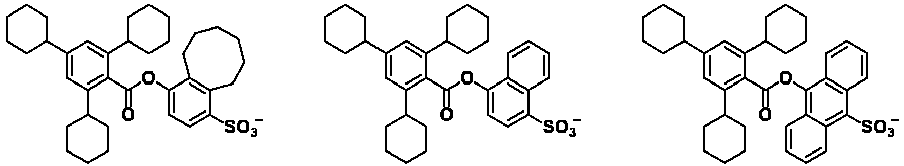

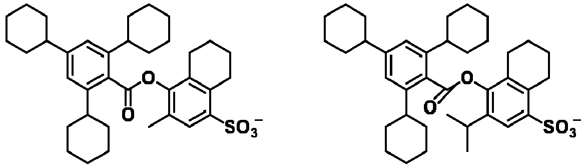

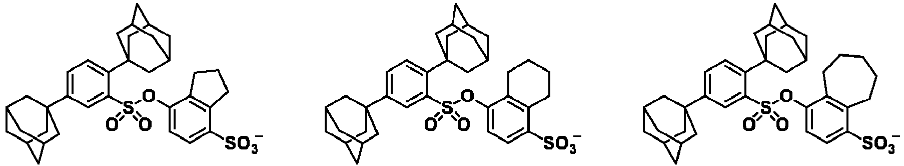

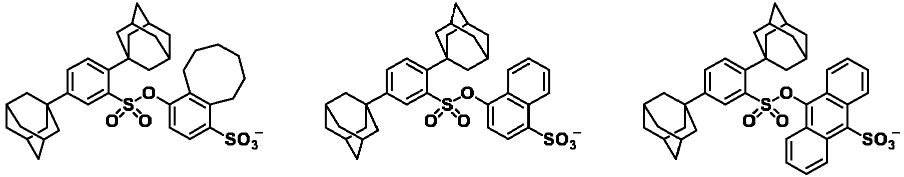

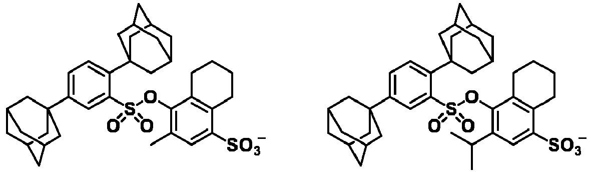

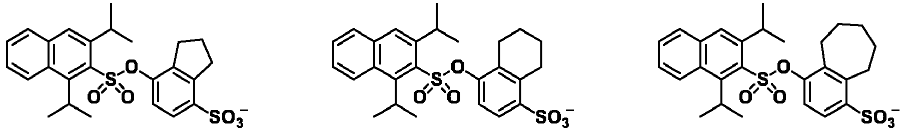

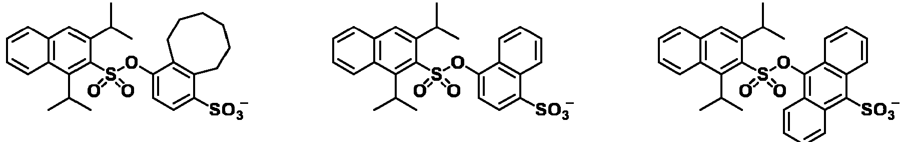

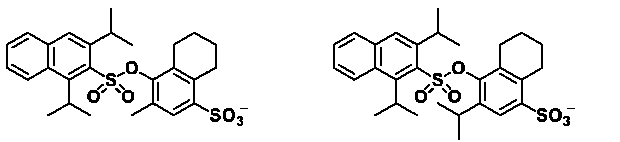

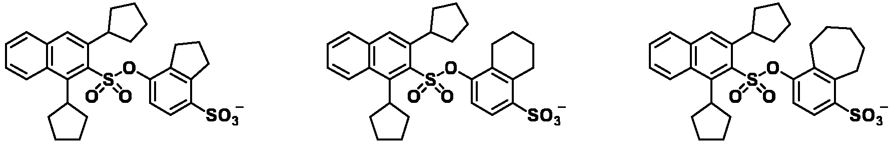

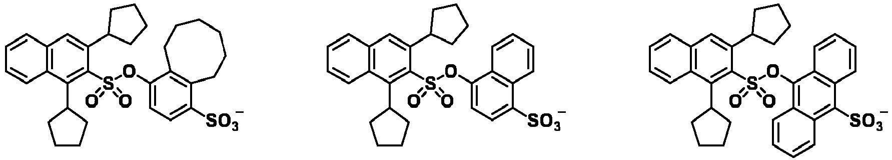

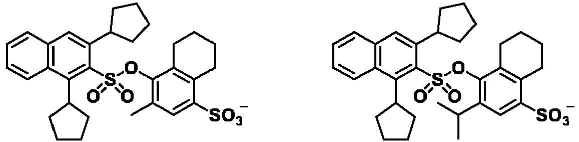

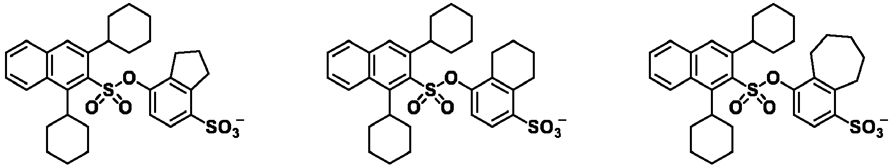

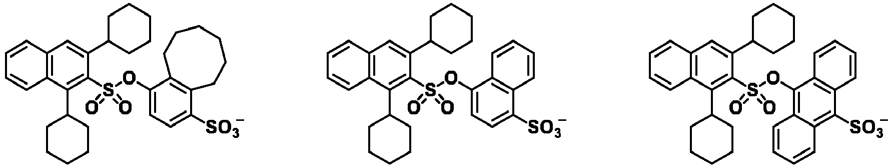

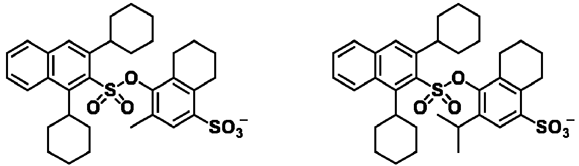

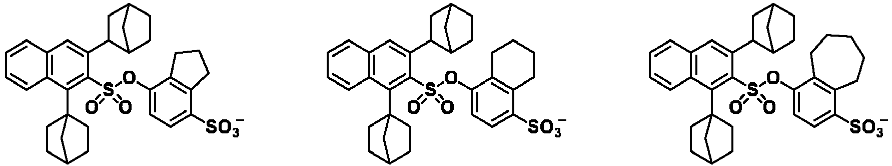

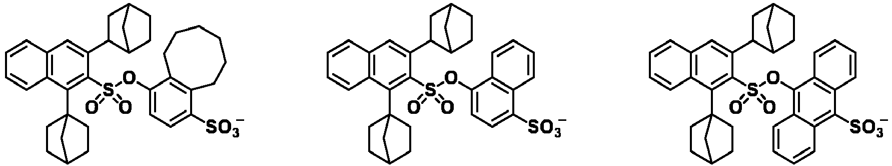

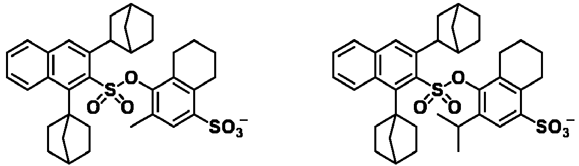

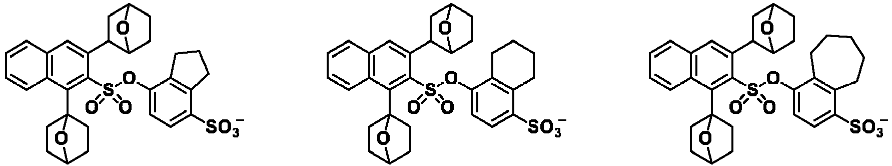

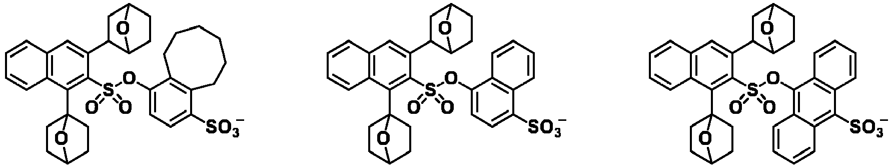

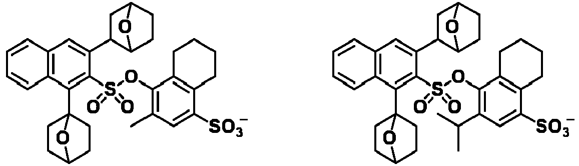

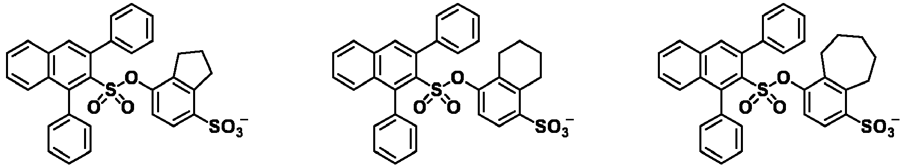

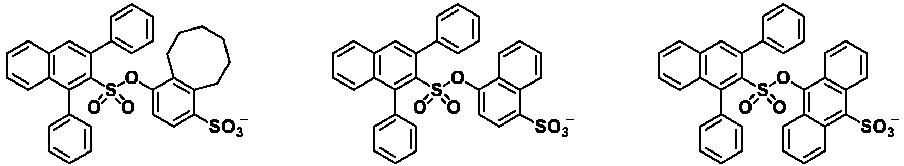

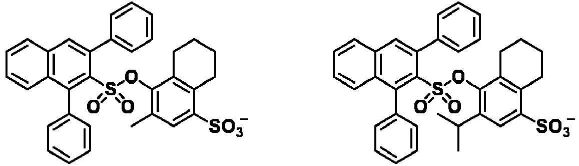

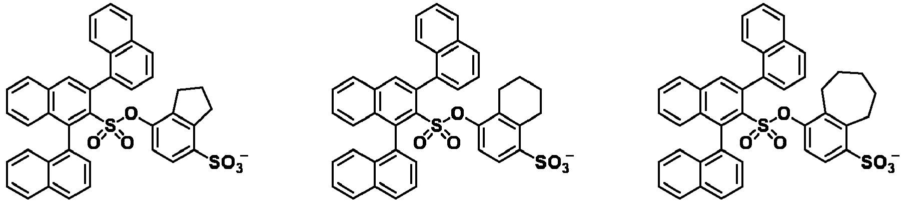

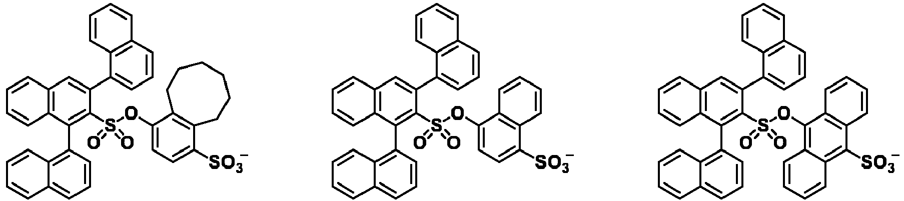

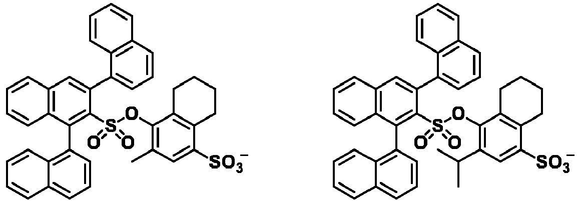

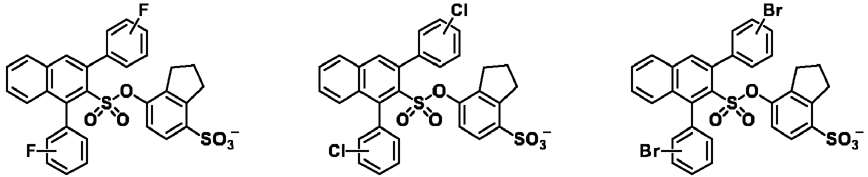

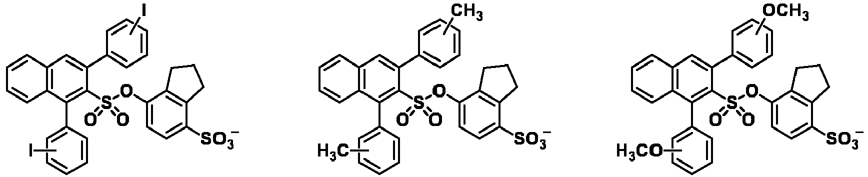

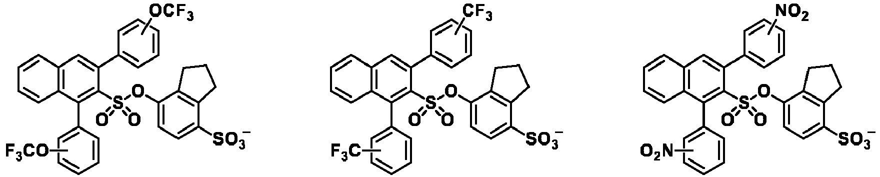

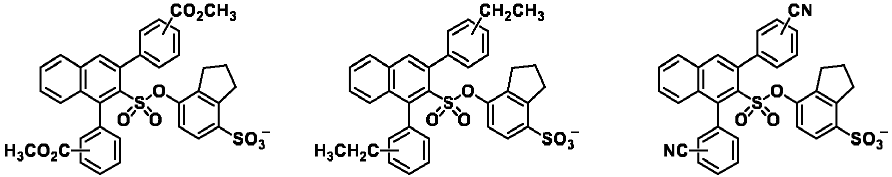

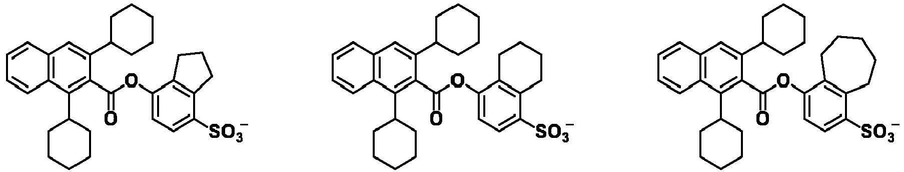

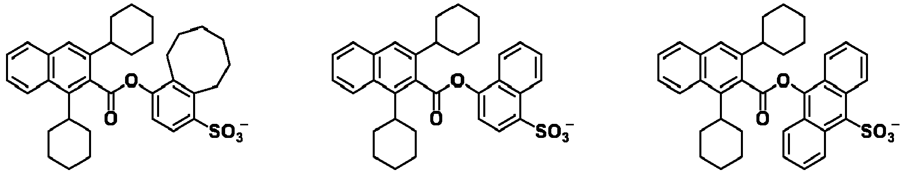

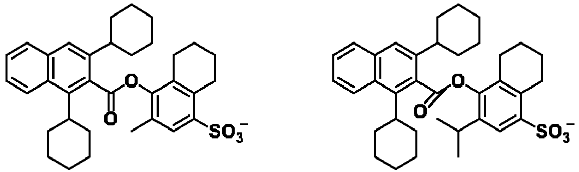

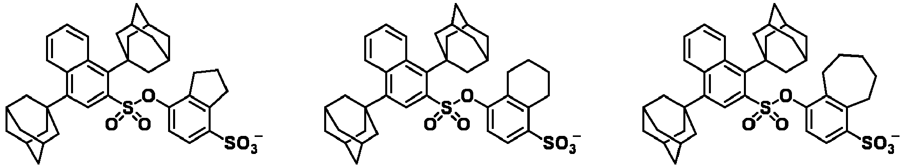

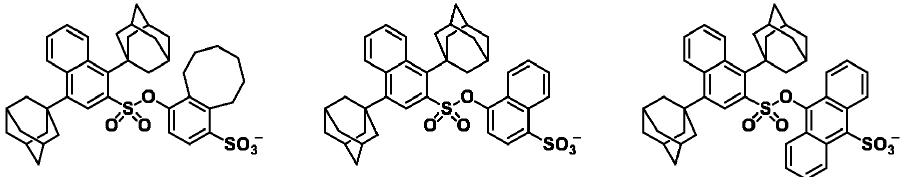

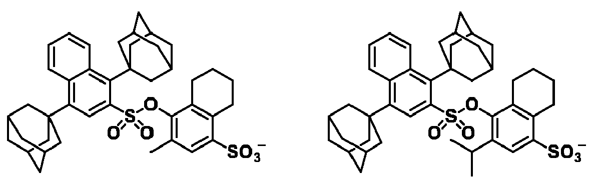

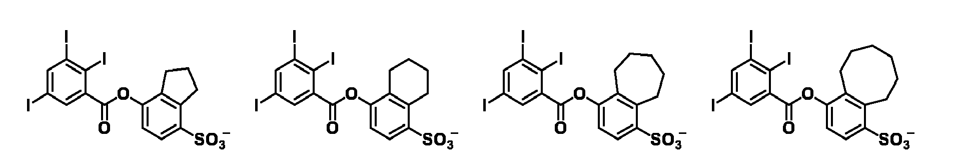

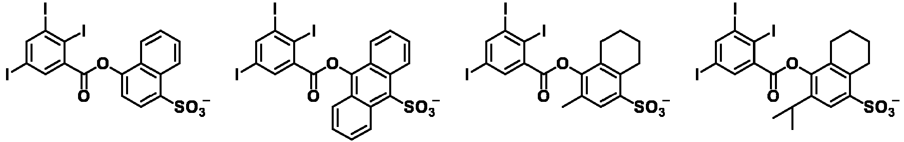

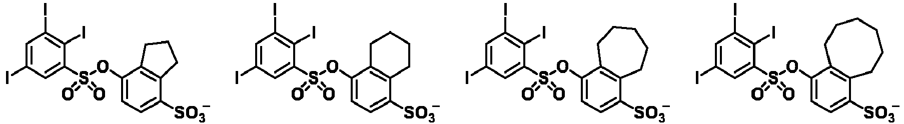

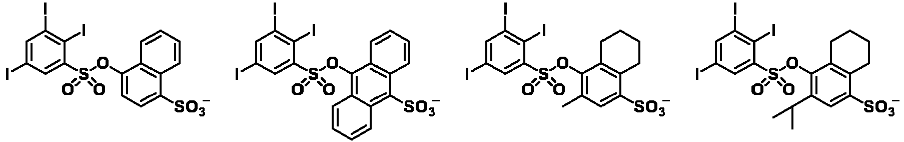

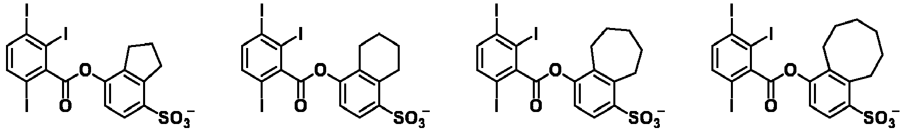

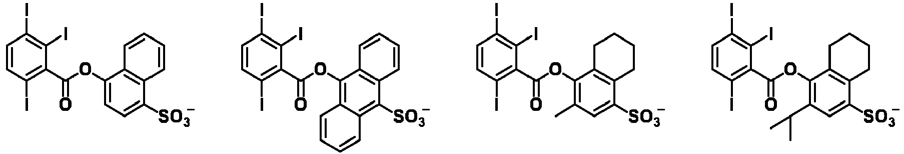

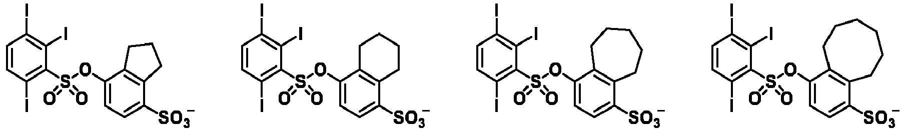

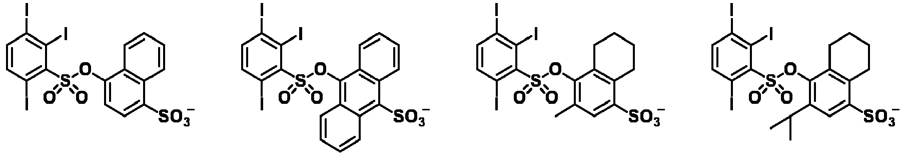

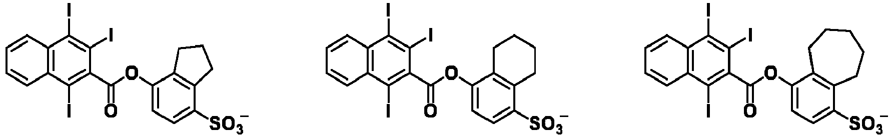

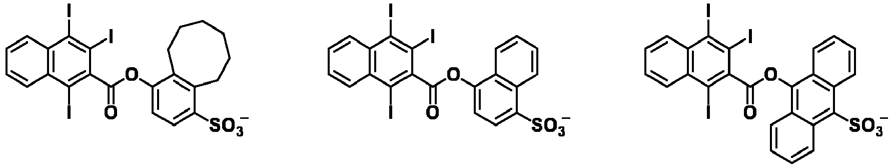

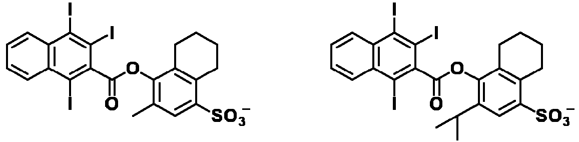

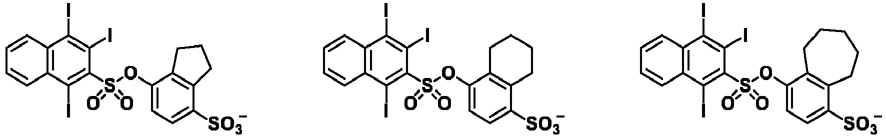

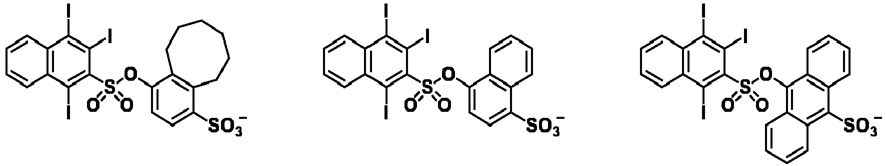

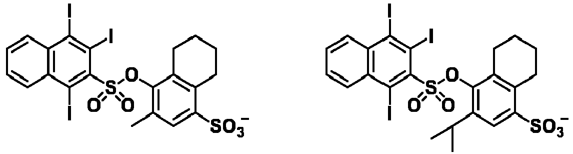

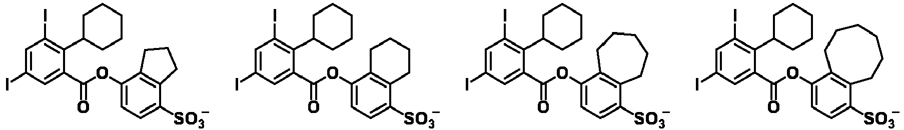

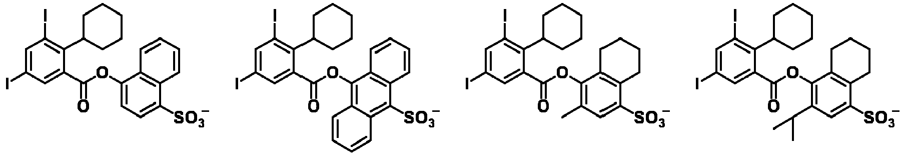

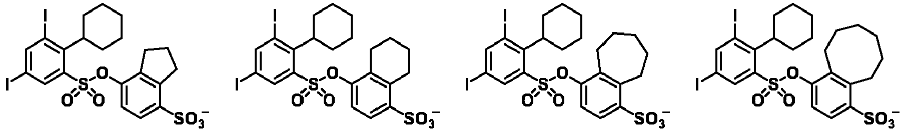

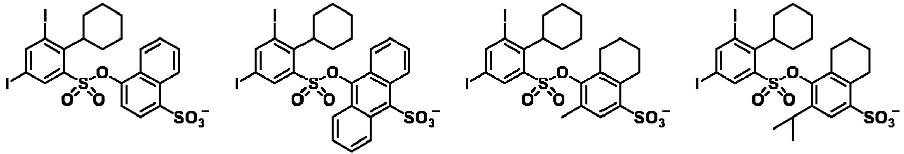

When the negative resist composition contains the acid generator (B), an appropriate amount of the acid generator used is 1 to 30 parts, more preferably 2 to 20 parts by weight per 80 parts by weight of the base polymer (A). In the embodiment wherein the base polymer contains repeat units A6 to A13, that is, in the case of polymer-bound acid generator, the addition of acid generator (B) may be omitted. The acid generator may be used alone or in admixture.

### (C) Crosslinker

The chemically amplified negative resist composition may comprise a crosslinker as component (C). Suitable crosslinkers which can be used herein include epoxy compounds, melamine compounds, guanamine compounds, glycoluril compounds and urea compounds having substituted thereon at least one group selected from among methylol, alkoxymethyl and acyloxymethyl groups, isocyanate compounds, azide compounds, and compounds having a double bond such as an alkenyloxy group. These compounds may be used as an additive or introduced into a polymer side chain as a pendant. Hydroxy-containing compounds may also be used as the crosslinker.

Of the foregoing crosslinkers, examples of suitable epoxy compounds include tris(2,3-epoxypropyl) isocyanurate, trimethylolmethane triglycidyl ether, trimethylolpropane triglycidyl ether, and triethylolethane triglycidyl ether.

Examples of the melamine compound include hexamethylol melamine, hexamethoxymethyl melamine, hexamethylol melamine compounds having 1 to 6 methylol groups methoxymethylated and mixtures thereof, hexamethoxyethyl melamine, hexaacyloxymethyl melamine, hexamethylol melamine compounds having 1 to 6 methylol groups acyloxymethylated and mixtures thereof.

Examples of the guanamine compound include tetramethylol guanamine, tetramethoxymethyl guanamine, tetramethylol guanamine compounds having 1 to 4 methylol groups methoxymethylated and mixtures thereof, tetramethoxyethyl guanamine, tetraacyloxyguanamine, tetramethylol guanamine compounds having 1 to 4 methylol groups acyloxymethylated and mixtures thereof.

Examples of the glycoluril compound include tetramethylol glycoluril, tetramethoxyglycoluril, tetramethoxymethyl glycoluril, tetramethylol glycoluril compounds having 1 to 4 methylol groups methoxymethylated and mixtures thereof, tetramethylol glycoluril compounds having 1 to 4 methylol groups acyloxymethylated and mixtures thereof.

Examples of the urea compound include tetramethylol urea, tetramethoxymethyl urea, tetramethylol urea compounds having 1 to 4 methylol groups methoxymethylated and mixtures thereof, and tetramethoxyethyl urea.

Suitable isocyanate compounds include tolylene diisocyanate, diphenylmethane diisocyanate, hexamethylene diisocyanate and cyclohexane diisocyanate.

Suitable azide compounds include 1,1'-biphenyl-4,4'-bisazide, 4,4'-methylidenebisazide, and 4,4'-oxybisazide.

Examples of the alkenyloxy-containing compound include ethylene glycol divinyl ether, triethylene glycol divinyl ether, 1,2-propanediol divinyl ether, 1,4-butanediol divinyl ether, tetramethylene glycol divinyl ether, neopentyl glycol divinyl ether, trimethylol propane trivinyl ether, hexanediol divinyl ether, 1,4-cyclohexanediol divinyl ether, pentaerythritol trivinyl ether, pentaerythritol tetravinyl ether, sorbitol tetravinyl ether, sorbitol pentavinyl ether, and trimethylol propane trivinyl ether.

An appropriate amount of the crosslinker (C) used is 0.1 to 50 parts, and more preferably 1 to 30 parts by weight per 80 parts by weight of the base polymer (A). As long as the amount of the crosslinker is in the range, the risk of resolution being reduced by forming bridges between pattern features is mitigated. The crosslinkers may be used alone or in admixture.

### (D) Fluorinated polymer

The negative resist composition may further comprise a fluorinated polymer for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. The fluorinated polymer contains repeat units of at least one type selected from repeat units having the formula (D1), repeat units having the formula (D2), repeat units having the formula (D3), and repeat units having the formula (D4), and may contain repeat units of at least one type selected from repeat units having the formula (D5) and repeat units having the formula (D6). It is noted that repeat units having formulae (D1), (D2), (D3), (D4), (D5), and (D6) are also referred to as repeat units D1, D2, D3, D4, D5, and D6, respectively, hereinafter. Since the fluorinated polymer also has a surfactant function, it can prevent insoluble residues from re-depositing onto the substrate during the development step and is thus effective for preventing development defects.

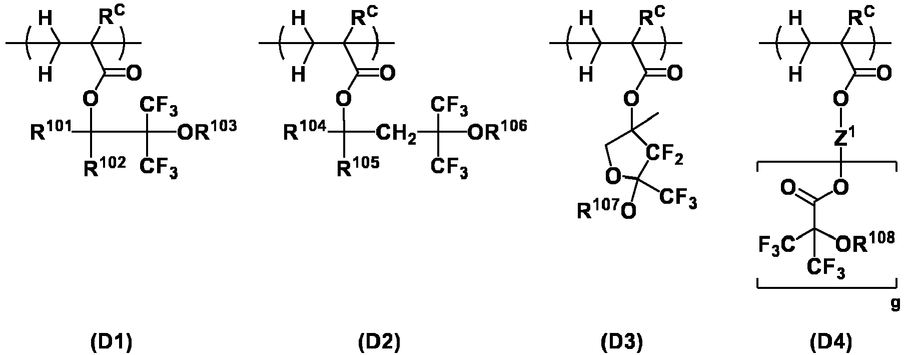

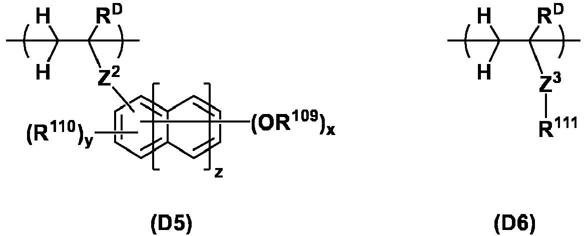

In formulae (D1) to (D6), x is an integer of 1 to 3, y is an integer satisfying: 0 ≤ y ≤ 5+2z-x, z is 0 or 1, and g is an integer of 1 to 3. R^{C} is each independently hydrogen, fluorine, methyl or trifluoromethyl. R^{D} is each independently hydrogen or methyl. R¹⁰¹, R¹⁰², R¹⁰⁴ and R¹⁰⁵ are each independently hydrogen or a C₁-C₁₀ saturated hydrocarbyl group. R¹⁰³, R¹⁰⁶, R¹⁰⁷ and R¹⁰⁸ are each independently hydrogen, a C₁-C₁₅ hydrocarbyl group or fluorinated hydrocarbyl group, or an acid labile group, with the proviso that an ether bond or carbonyl moiety may intervene in a carbon-carbon bond in the hydrocarbyl groups or fluorinated hydrocarbyl groups represented by R¹⁰³, R¹⁰⁶, R¹⁰⁷ and R¹⁰⁸. R¹⁰⁹ is hydrogen or a C₁-C₅ straight or branched hydrocarbyl group in which a heteroatom-containing moiety may intervene in a carbon-carbon bond. R¹¹⁰ is a C₁-C₅ straight or branched hydrocarbyl group in which a heteroatom-containing moiety may intervene in a carbon-carbon bond. R¹¹¹ is a C₁-C₂₀ saturated hydrocarbyl group in which at least one hydrogen is substituted by fluorine and some constituent -CH₂- may be replaced by an ester bond or ether bond. Z¹ is a C₁-C₂₀ (g+1)-valent hydrocarbon group or C₁-C₂₀ (g+1)-valent fluorinated hydrocarbon group. Z² is a single bond, *-C(=O)-O- or *-C(=O)-NH-. Z³ is a single bond, -O-, *-C(=O)-O-Z³¹-Z³²- or *-C(=O)-NH-Z³¹-Z³²-. Z³¹ is a single bond or a C₁-C₁₀ saturated hydrocarbylene group, and Z³² is a single bond, ester bond, ether bond or sulfonamide bond. The asterisk (*) designates a point of attachment to the carbon atom in the backbone.

In formulae (D1) and (D2), the C₁-C₁₀ saturated hydrocarbyl group represented by R¹⁰¹, R¹⁰², R¹⁰⁴ and R¹⁰⁵ may be straight, branched or cyclic and examples thereof include C₁-C₁₀ alkyl groups such as methyl, ethyl, n-propyl, isopropyl, n-butyl, isobutyl, sec-butyl, tert-butyl, n-pentyl, n-hexyl, n-heptyl, n-octyl, n-nonyl, and n-decyl, and C₃-C₁₀ cyclic saturated hydrocarbyl groups such as cyclopropyl, cyclobutyl, cyclopentyl, cyclohexyl, adamantyl, and norbornyl. Inter alia, C₁-C₆ saturated hydrocarbyl groups are preferred.

In formulae (D1) to (D4), the C₁-C₁₅ hydrocarbyl group represented by R¹⁰³, R¹⁰⁶, R¹⁰⁷ and R¹⁰⁸ may be straight, branched or cyclic and examples thereof include C₁-C₁₅ alkyl, C₂-C₁₅ alkenyl and C₂-C₁₅ alkynyl groups, with the alkyl groups being preferred. Suitable alkyl groups include methyl, ethyl, n-propyl, isopropyl, n-butyl, isobutyl, sec-butyl, n-pentyl, n-hexyl, n-heptyl, n-octyl, n-nonyl, n-decyl, n-undecyl, n-dodecyl, n-tridecyl, n-tetradecyl and n-pentadecyl. The fluorinated hydrocarbyl groups correspond to the foregoing hydrocarbyl groups in which some or all carbon-bonded hydrogen atoms are substituted by fluorine atoms.

In formula (D4), examples of the C₁-C₂₀ (g+1)-valent hydrocarbon group Z¹ include the foregoing C₁-C₂₀ alkyl groups and C₃-C₂₀ cyclic saturated hydrocarbyl groups, with g number of hydrogen atoms being eliminated. Examples of the C₁-C₂₀ (g+1)-valent fluorinated hydrocarbon group Z¹ include the foregoing (g+1)-valent hydrocarbon groups in which at least one hydrogen atom is substituted by fluorine.

Examples of the repeat units D1 to D4 are given below, but not limited thereto. Herein R^{C} is as defined above.

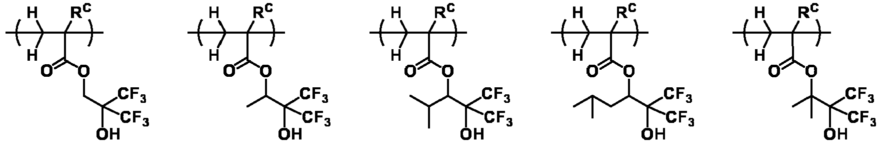

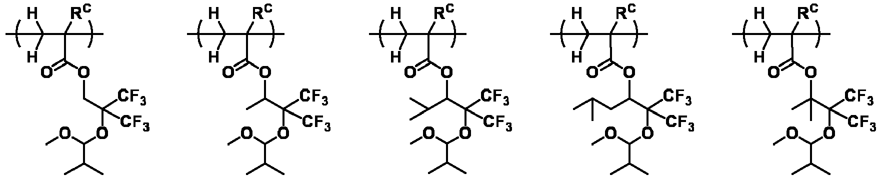

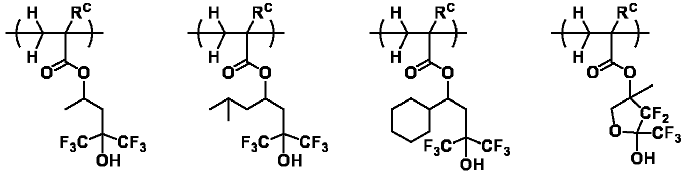

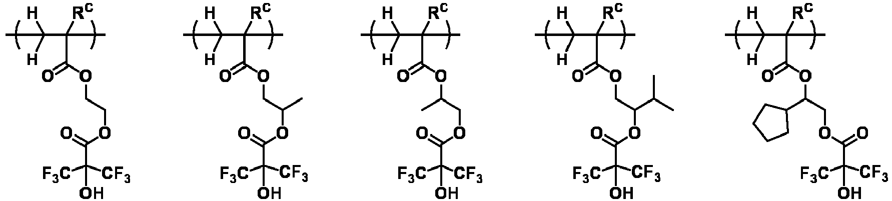

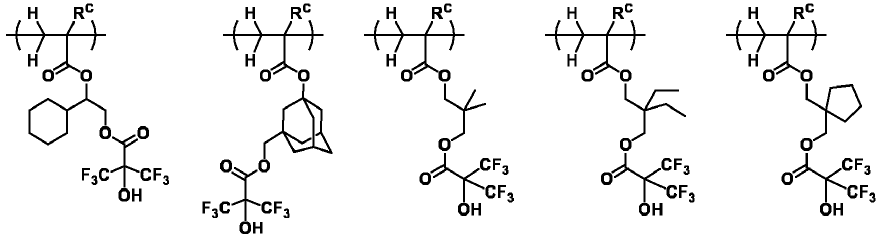

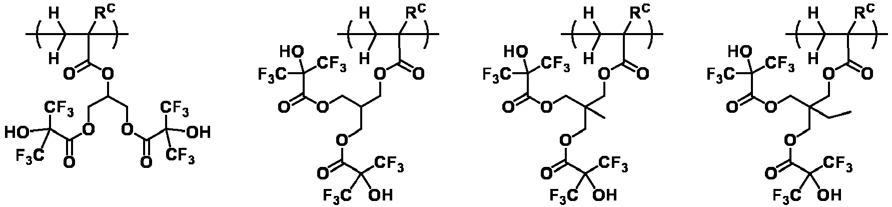

In formula (D5), examples of the C₁-C₅ hydrocarbyl groups R¹⁰⁹ and R¹¹⁰ include alkyl, alkenyl and alkynyl groups, with the alkyl groups being preferred. Suitable alkyl groups include methyl, ethyl, n-propyl, isopropyl, n-butyl, isobutyl, sec-butyl, and n-pentyl. In these groups, a moiety containing a heteroatom such as oxygen, sulfur or nitrogen may intervene in a carbon-carbon bond.

In formula (D5), -OR¹⁰⁹ is preferably a hydrophilic group. In this case, R¹⁰⁹ is preferably hydrogen or a C₁-C₅ alkyl group in which oxygen intervenes in a carbon-carbon bond.

In formula (D5), Z² is preferably *-C(=O)-O- or *-C(=O)-NH-. Also preferably R^{D} is methyl. The inclusion of carbonyl in Z² enhances the ability to trap the acid originating from the anti-charging film. A polymer wherein R^{D} is methyl is a robust polymer having a high glass transition temperature (Tg) which is effective for suppressing acid diffusion. As a result, the resist film is improved in stability with time, and neither resolution nor pattern profile is degraded.

Examples of the repeat unit D5 are given below, but not limited thereto. Herein R^{D} is as defined above.

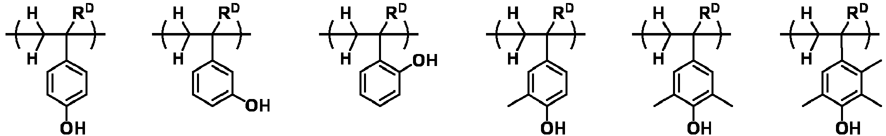

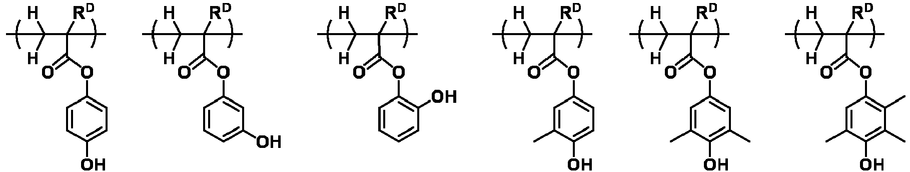

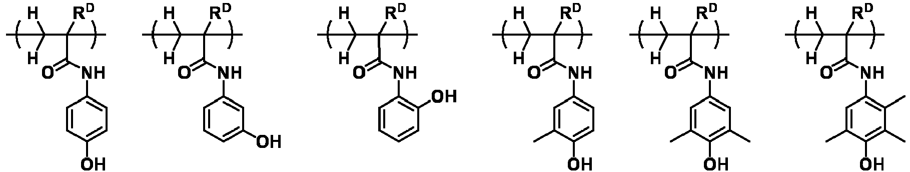

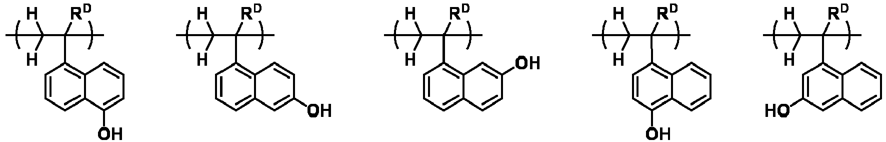

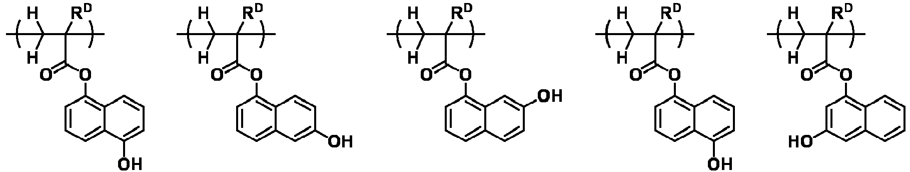

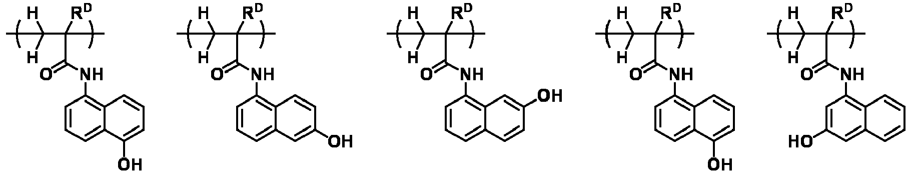

In formula (D6), the C₁-C₁₀ saturated hydrocarbylene group Z³ may be straight, branched or cyclic and examples thereof include methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, and 1,1-dimethylethane-1,2-diyl.

The C₁-C₂₀ saturated hydrocarbyl group having at least one hydrogen substituted by fluorine, represented by R¹¹¹, may be straight, branched or cyclic and examples thereof include C₁-C₂₀ alkyl groups and C₃-C₂₀ cyclic saturated hydrocarbyl groups in which at least one hydrogen is substituted by fluorine.

Examples of the repeat unit D6 are given below, but not limited thereto. Herein R^{D} is as defined above.

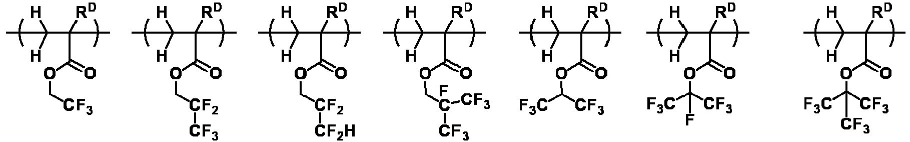

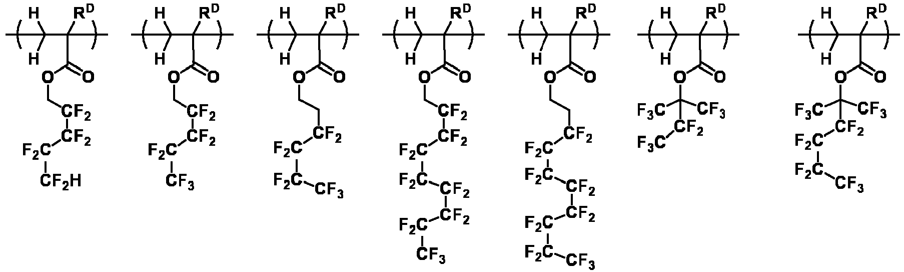

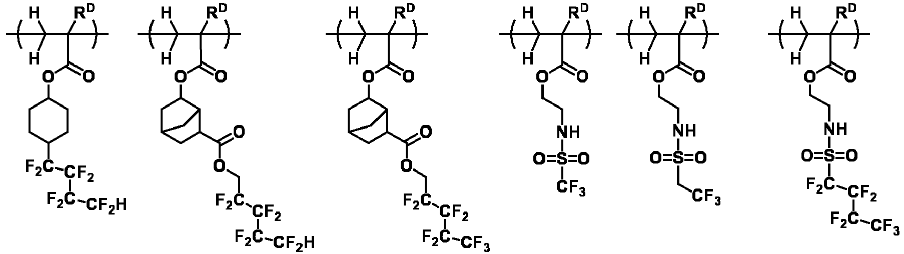

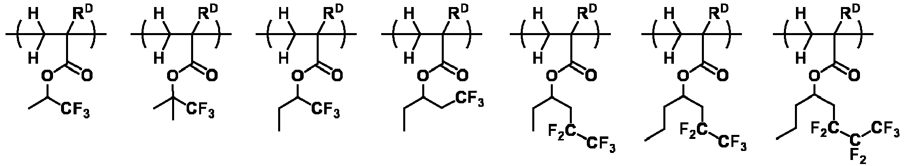

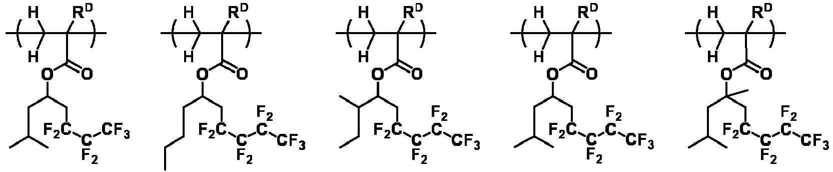

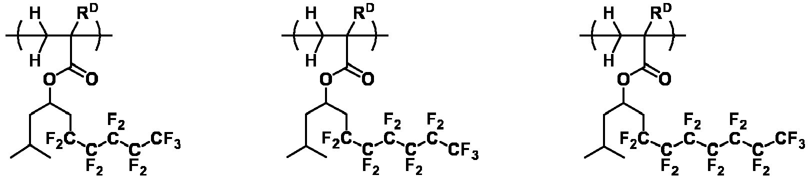

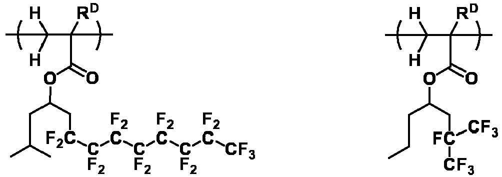

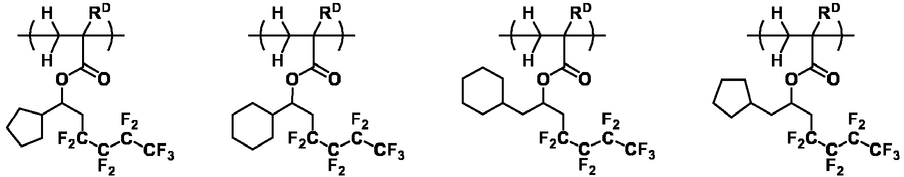

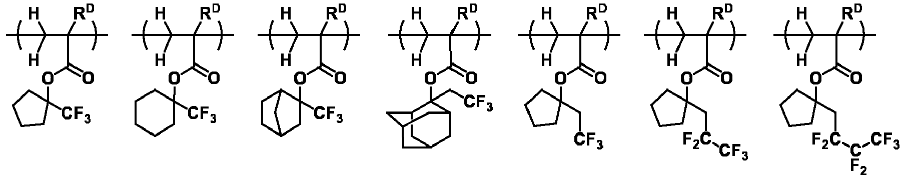

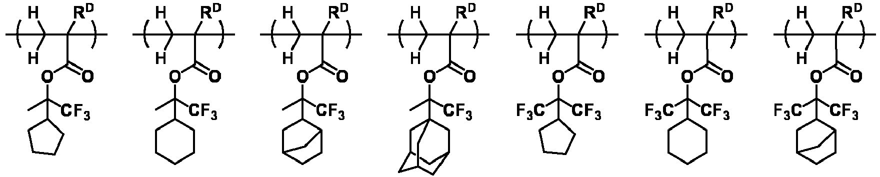

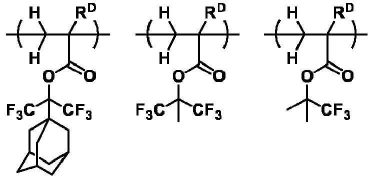

The repeat units D1 to D4 are preferably incorporated in an amount of 15 to 95 mol%, more preferably 20 to 85 mol% based on the overall repeat units of the fluorinated polymer. The repeat unit D5 and/or D6 is preferably incorporated in an amount of 5 to 85 mol%, more preferably 15 to 80 mol% based on the overall repeat units of the fluorinated polymer. Each of repeat units D1 to D6 may be used alone or in admixture.

The fluorinated polymer may comprise additional repeat units as well as the repeat units D1 to D6. Suitable additional repeat units include those described in USP 9,091,918 (JP-A 2014-177407, paragraphs [0046]-[0078]). When the fluorinated polymer comprises additional repeat units, their content is preferably up to 50 mol% based on the overall repeat units.

The fluorinated polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630.

The fluorinated polymer should preferably have a Mw of 2,000 to 50,000, and more preferably 3,000 to 20,000. A fluorinated polymer with a Mw of less than 2,000 helps acid diffusion, degrading resolution and detracting from age stability. A polymer with too high Mw has a reduced solubility in solvent, with a risk of leaving coating defects. The fluorinated polymer preferably has a dispersity (Mw/Mn) of 1.0 to 2.2, more preferably 1.0 to 1.7.

In the negative resist composition, the fluorinated polymer (D) is preferably used in an amount of 0.01 to 30 parts, more preferably 0.1 to 20 parts, even more preferably 0.5 to 10 parts by weight per 80 parts by weight of the base polymer (A). The fluorinated polymer may be used alone or in admixture.

### (E) Quencher

The chemically amplified negative resist composition preferably comprises a quencher as component (E). As used herein, the quencher refers to a compound capable of trapping an acid generated from the acid generator upon exposure to prevent the acid from diffusing into the unexposed region, thus enabling to form the desired pattern.

The quencher is typically selected from conventional basic compounds. Conventional basic compounds include primary, secondary, and tertiary aliphatic amines, mixed amines, aromatic amines, heterocyclic amines, nitrogen-containing compounds with carboxy group, nitrogen-containing compounds with sulfonyl group, nitrogen-containing compounds with hydroxy group, nitrogen-containing compounds with hydroxyphenyl group, alcoholic nitrogen-containing compounds, amide derivatives, imide derivatives, and carbamate derivatives. Also included are primary, secondary, and tertiary amine compounds, specifically amine compounds having a hydroxy, ether bond, ester bond, lactone ring, cyano, or sulfonate ester group as described in JP-A 2008-111103, paragraphs [0146]-[0164], and compounds having a carbamate group as described in JP 3790649. Inter alia, tris[2-(methoxymethoxy)ethyl]amine, tris[2-(methoxymethoxy)ethyl]amine-N-oxide, dibutylaminobenzoic acid, morpholine derivatives, and imidazole derivatives are preferred. Addition of a basic compound may be effective for further suppressing the diffusion rate of acid in the resist film or correcting the pattern profile.

Onium salts such as sulfonium, iodonium and ammonium salts of carboxylic acids which are not fluorinated at α-position as described in USP 8,795,942 (JP-A 2008-158339) may also be used as the quencher. While an α-fluorinated sulfonic acid, imide acid, and methide acid are necessary to deprotect the acid labile group, an α-non-fluorinated carboxylic acid is released by salt exchange with an α-non-fluorinated onium salt. The α-non-fluorinated carboxylic acid functions as a quencher because it does not induce substantial deprotection reaction.

Examples of the onium salt of α-non-fluorinated carboxylic acid include compounds having the formula (E1).

R²⁰¹-CO₂⁻ Mq_{A}⁺ (E1)

In formula (E1), R²⁰¹ is hydrogen or a C₁-C₄₀ hydrocarbyl group which may contain a heteroatom, exclusive of the hydrocarbyl group in which the hydrogen bonded to the carbon atom at α-position of the carboxy group is substituted by fluorine or fluoroalkyl.

The hydrocarbyl group R²⁰¹ may be saturated or unsaturated and straight, branched or cyclic. Examples thereof include C₁-C₄₀ alkyl groups such as methyl, ethyl, n-propyl, isopropyl, n-butyl, sec-butyl, tert-butyl, n-pentyl, tert-pentyl, n-hexyl, n-octyl, 2-ethylhexyl, n-nonyl, n-decyl; C₃-C₄₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; C₂-C₄₀ alkenyl groups such as vinyl, allyl, propenyl, butenyl and hexenyl; C₃-C₄₀ cyclic unsaturated aliphatic hydrocarbyl groups such as cyclohexenyl; C₆-C₄₀ aryl groups such as phenyl, naphthyl, alkylphenyl groups (e.g., 2-methylphenyl, 3-methylphenyl, 4-methylphenyl, 4-ethylphenyl, 4-tert-butylphenyl, 4-n-butylphenyl), di- or trialkylphenyl groups (e.g., 2,4-dimethylphenyl and 2,4,6-triisopropylphenyl), alkylnaphthyl groups (e.g., methylnaphthyl and ethylnaphthyl), dialkylnaphthyl groups (e.g., dimethylnaphthyl and diethylnaphthyl); and C₇-C₄₀ aralkyl groups such as benzyl, 1-phenylethyl and 2-phenylethyl.

In the hydrocarbyl groups, some or all hydrogen may be substituted by a moiety containing a heteroatom such as oxygen, sulfur, nitrogen or halogen, and some -CH₂-may be replaced by a moiety containing a heteroatom such as oxygen, sulfur or nitrogen, so that the group may contain a hydroxy moiety, cyano moiety, carbonyl moiety, ether bond, thioether bond, ester bond, sulfonate ester bond, carbonate bond, lactone ring, sultone ring, carboxylic anhydride (-C(=O)-O-C(=O)-), or haloalkyl moiety. Suitable heteroatom-containing hydrocarbyl groups include heteroaryl groups such as thienyl; alkoxyphenyl groups such as 4-hydroxyphenyl, 4-methoxyphenyl, 3-methoxyphenyl, 2-methoxyphenyl, 4-ethoxyphenyl, 4-tert-butoxyphenyl, 3-tert-butoxyphenyl; alkoxynaphthyl groups such as methoxynaphthyl, ethoxynaphthyl, n-propoxynaphthyl and n-butoxynaphthyl; dialkoxynaphthyl groups such as dimethoxynaphthyl and diethoxynaphthyl; and aryloxoalkyl groups, typically 2-aryl-2-oxoethyl groups such as 2-phenyl-2-oxoethyl, 2-(1-naphthyl)-2-oxoethyl and 2-(2-naphthyl)-2-oxoethyl.

In formula (E1), Mq_{A}⁺ is an onium cation. The onium cation is preferably selected from sulfonium, iodonium and ammonium cations, more preferably sulfonium and iodonium cations. Exemplary sulfonium cations are as exemplified above for the sulfonium cation in repeat units A7 to A9. Exemplary iodonium cations are as exemplified above for the iodonium cation in repeat units A11 to A13.

Examples of the anion in the onium salt having formula (E1) are shown below, but not limited thereto.

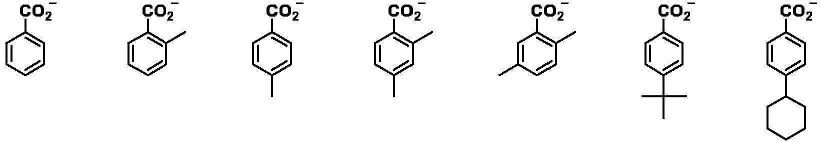

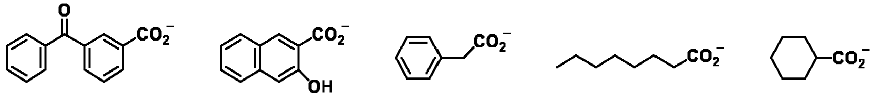

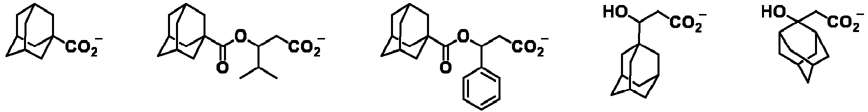

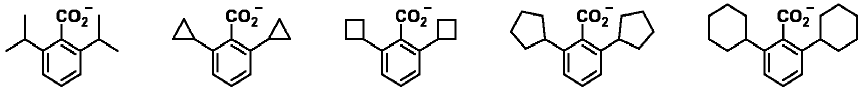

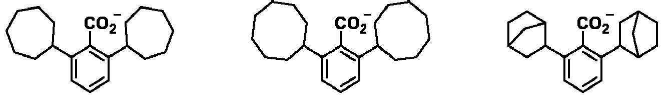

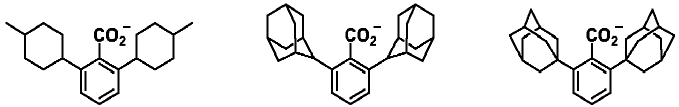

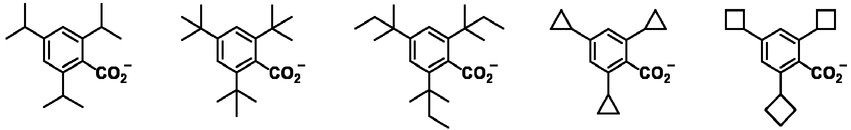

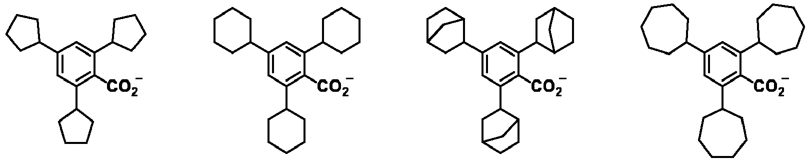

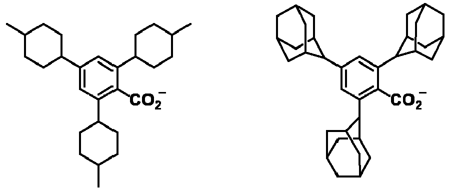

A sulfonium salt of iodized benzene ring-containing carboxylic acid having the formula (E2) is also useful as the quencher.

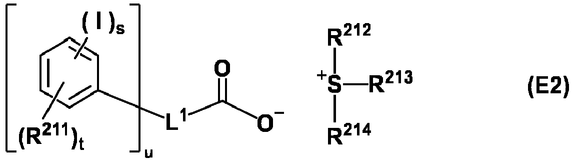

In formula (E2), s is an integer of 1 to 5, t is an integer of 0 to 3, s+t is from 1 to 5, and u is an integer of 1 to 3.

In formula (E2), R²¹¹ is hydroxy, fluorine, chlorine, bromine, amino, nitro, cyano, or a C₁-C₆ saturated hydrocarbyl, C₁-C₆ saturated hydrocarbyloxy, C₂-C₆ saturated hydrocarbylcarbonyloxy or C₁-C₄ saturated hydrocarbylsulfonyloxy group, in which some or all hydrogen may be substituted by halogen, or -N(R^{211A})-C(=O)-R^{211B}, or -N(R^{211A})-C(=O)-O-R^{211B}. R^{211A} is hydrogen or a C₁-C₆ saturated hydrocarbyl group. R^{211B} is a C₁-C₆ saturated hydrocarbyl or C₂-C₈ unsaturated aliphatic hydrocarbyl group. A plurality of R²¹¹ may be the same or different when t and/or u is 2 or 3.

In formula (E2), L¹ is a single bond, or a C₁-C₂₀ (u+1)-valent linking group which may contain at least one moiety selected from ether bond, carbonyl moiety, ester bond, amide bond, sultone ring, lactam ring, carbonate bond, halogen, hydroxy moiety, and carboxy moiety. The saturated hydrocarbyl, saturated hydrocarbyloxy, saturated hydrocarbylcarbonyloxy, and saturated hydrocarbylsulfonyloxy groups may be straight, branched or cyclic.

In formula (E2), R²¹², R²¹³ and R²¹⁴ are each independently halogen, or a C₁-C₂₀ hydrocarbyl group which may contain a heteroatom. The hydrocarbyl group may be saturated or unsaturated and straight, branched or cyclic. Examples thereof include C₁-C₂₀ alkyl, C₂-C₂₀ alkenyl, C₆-C₂₀ aryl, and C₇-C₂₀ aralkyl groups. In these groups, some or all hydrogen may be substituted by hydroxy, carboxy, halogen, oxo, cyano, nitro, sultone ring, sulfo, or sulfonium salt-containing moiety, or some -CH₂- may be replaced by an ether bond, ester bond, carbonyl moiety, amide bond, carbonate bond or sulfonate ester bond. Also, R²¹² and R²¹³ may bond together to form a ring with the sulfur atom to which they are attached.

Examples of the compound having formula (E2) include those described in USP 10,295,904 (JP-A 2017-219836). These compounds exert a sensitizing effect due to remarkable absorption and an acid diffusion-controlling effect.

A nitrogen-containing carboxylic acid salt compound having the formula (E3) is also useful as the quencher.

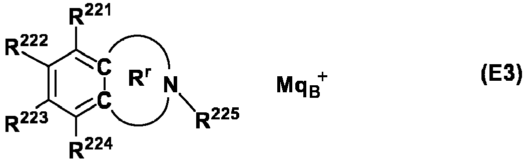

In formula (E3), R²²¹ to R²²⁴ are each independently hydrogen, -L²-CO₂⁻, or a C₁-C₂₀ hydrocarbyl group which may contain a heteroatom. R²²¹ and R²²², R²²² and R²²³, or R²²³ and R²²⁴ may bond together to form a ring with the carbon atom to which they are attached. L² is a single bond or a C₁-C₂₀ hydrocarbylene group which may contain a heteroatom. R^{22s} is hydrogen or a C₁-C₂₀ hydrocarbyl group which may contain a heteroatom.

In formula (E3), the ring R^{r} is a C₂-C₆ ring containing the carbon and nitrogen atoms in the formula, in which some or all of the carbon-bonded hydrogen atoms may be substituted by a C₁-C₂₀ hydrocarbyl group or -L²-CO₂⁻ and in which some carbon may be replaced by sulfur, oxygen or nitrogen. The ring may be alicyclic or aromatic and is preferably a 5- or 6-membered ring. Suitable rings include pyridine, pyrrole, pyrrolidine, piperidine, pyrazole, imidazoline, pyridazine, pyrimidine, pyrazine, imidazoline, oxazole, thiazole, morpholine, thiazine, and triazole rings.

The carboxylic onium salt having formula (E3) has at least one -L²-CO₂⁻. That is, at least one of R²²¹ to R²²⁴ is -L²-CO₂⁻, and/or at least one of hydrogen atoms bonded to carbon atoms in the ring R^{r} is substituted by -L²-CO₂⁻.

In formula (E3), Mq_{B}⁺ is a sulfonium, iodonium or ammonium cation, with the sulfonium cation being preferred. Examples of the sulfonium cation are as exemplified above for the sulfonium cation in repeat units A7 to A9.

Examples of the anion in the compound having formula (E3) are shown below, but not limited thereto.

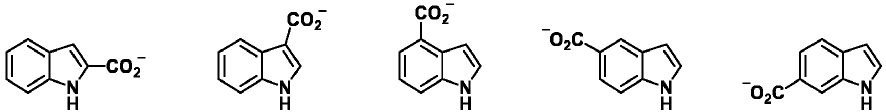

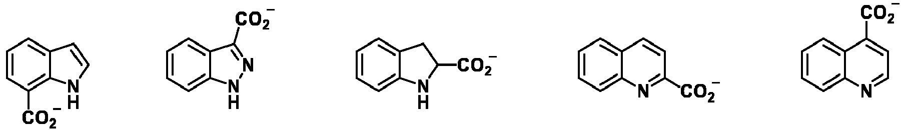

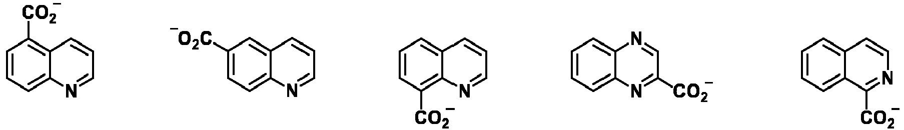

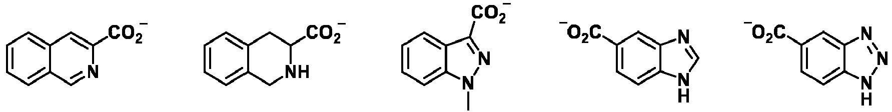

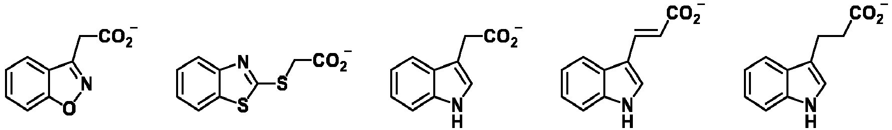

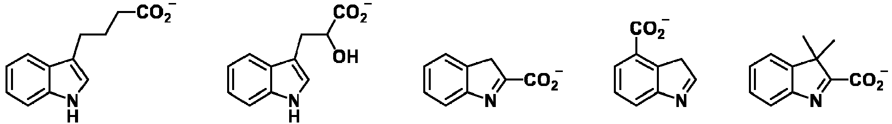

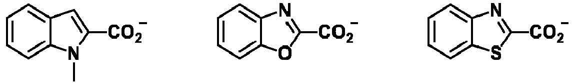

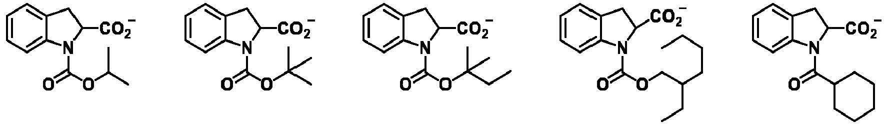

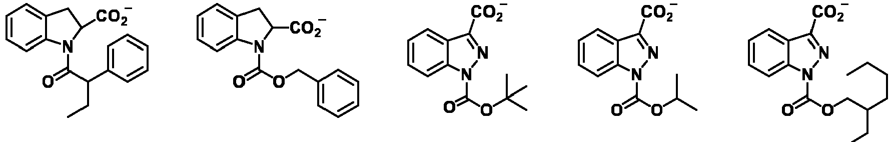

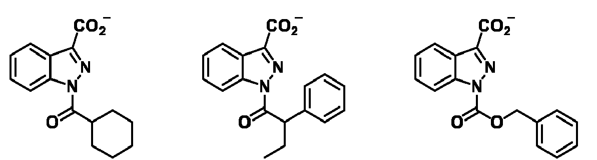

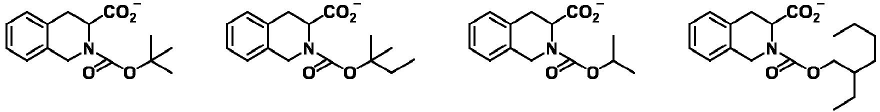

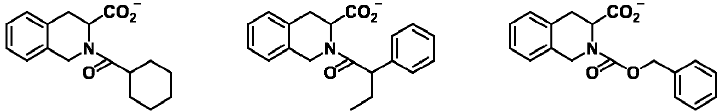

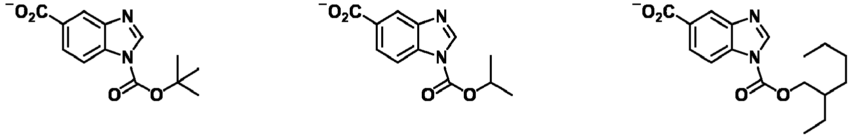

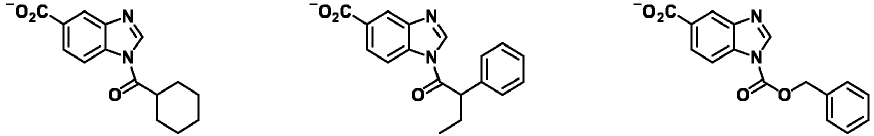

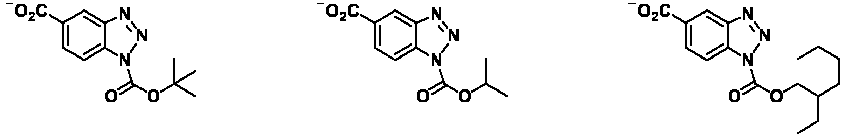

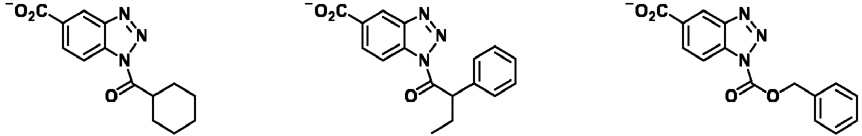

Weak acid betaine compounds are also useful as the quencher. Non-limiting examples thereof are shown below.

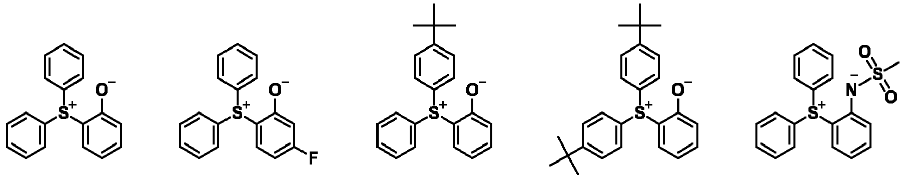

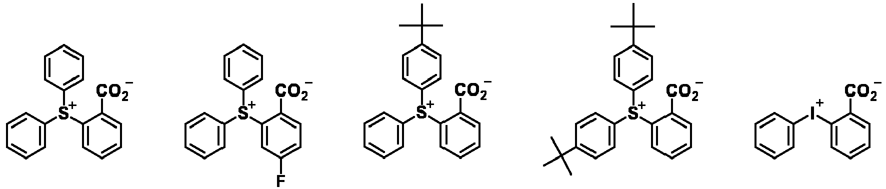

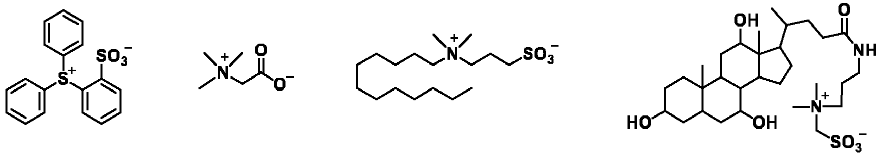

Also useful are quenchers of polymer type as described in USP 7,598,016 (JP-A 2008-239918). The polymeric quencher segregates at the resist surface after coating and thus enhances the rectangularity of resist pattern. When a protective film is applied as is often the case in the immersion lithography, the polymeric quencher is also effective for preventing a film thickness loss of resist pattern or rounding of pattern top.

When used, the quencher (E) is preferably added in an amount of 0 to 50 parts, more preferably 0.1 to 40 parts by weight per 80 parts by weight of the base polymer (A). The quencher may be used alone or in admixture.

When the chemically amplified negative resist composition contains both the PAG (B) and the quencher (E), the weight ratio of the PAG to the quencher, (B)/(E) is preferably less than 6/1, more preferably less than 5/1, even more preferably less than 4/1. As long as the weight ratio of the PAG to the quencher is in the range, the resist composition is able to fully suppress acid diffusion, leading to improved resolution and dimensional uniformity.

### (F) Organic solvent

The resist composition may further contain an organic solvent as component (F). The organic solvent used herein is not particularly limited as long as resist components are soluble therein. Suitable organic solvents include ketones such as cyclohexanone, cyclopentanone, methyl-2-n-pentyl ketone, and 2-heptanone; alcohols such as 3-methoxybutanol, 3-methyl-3-methoxybutanol, 1-methoxy-2-propanol, 1-ethoxy-2-propanol, and diacetone alcohol (DAA); ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate (EL), ethyl pyruvate, butyl acetate, methyl 3-methoxypropionate, ethyl 3-ethoxypropionate, t-butyl acetate, t-butyl propionate, and propylene glycol mono-t-butyl ether acetate; and lactones such as γ-butyrolactone (GBL), and mixtures thereof. Of these organic solvents, 1-ethoxy-2-propanol, PGME, PGMEA, cyclohexanone, EL, GBL, and mixtures thereof are preferred.

When the resist composition contains an organic solvent (F), the organic solvent is preferably used in an amount of 200 to 10,000 parts, more preferably 400 to 6,000 parts by weight per 80 parts by weight of the base polymer (A). The organic solvent may be used alone or in admixture.

### (G) Surfactant

The negative resist composition may contain any conventional surfactants for facilitating to coat the composition to the substrate. Exemplary surfactants include PF-636 (Omnova Solutions Inc.) and FC-4430 (3M) as well as a number of known surfactants as described in JP-A 2004-115630. Any suitable one may be chosen therefrom. The amount of the surfactant (G) added is preferably 0 to 5 parts by weight per 80 parts by weight of the base polymer (A). The surfactant may be used alone or in admixture.

### Process

A further embodiment of the invention is a resist pattern forming process comprising the steps of applying the chemically amplified negative resist composition defined above onto a substrate to form a resist film thereon, exposing the resist film patternwise to high-energy radiation, and developing the resist film in an alkaline developer to form a resist pattern.

Pattern formation using the negative resist composition of the invention may be performed by well-known lithography processes. In general, the resist composition is first applied onto a substrate for IC fabrication (e.g., Si, SiO, SiO₂, SiN, SiON, TiN, WSi, BPSG, SOG, organic antireflective coating, etc.) or a substrate for mask circuit fabrication (e.g., Cr, CrO, CrON, MoSi₂, Si, SiO, SiO₂, SiON, SiONC, CoTa, NiTa, TaBN, SnO₂, etc.) by a suitable coating technique such as spin coating. The coating is prebaked on a hotplate preferably at a temperature of 60 to 150°C for 1 to 20 minutes, more preferably at 80 to 140°C for 1 to 10 minutes to form a resist film of 0.03 to 2 µm thick.

Then the resist film is exposed patternwise to high-energy radiation such as UV, deep-UV, excimer laser (KrF, ArF), EUV, x-ray, γ-ray or synchrotron radiation or EB. The resist composition of the invention is especially effective in the EUV or EB lithography.

On use of UV, deep-UV, EUV, excimer laser, x-ray, γ-ray or synchrotron radiation as the high-energy radiation, the resist film is exposed through a mask having a desired pattern, preferably in a dose of 1 to 500 mJ/cm², more preferably 10 to 400 mJ/cm².

On use of EB, a pattern may be written directly in a dose of preferably 1 to 500 µC/cm², more preferably 10 to 400 µC/cm².

The exposure may be performed by conventional lithography whereas the immersion lithography of holding a liquid, typically water between the mask and the resist film may be employed if desired. In the case of immersion lithography, a protective film which is insoluble in water may be used.

The resist film is then baked (PEB) on a hotplate preferably at 60 to 150°C for 1 to 20 minutes, more preferably at 80 to 140°C for 1 to 10 minutes.

Thereafter, the resist film is developed with a developer in the form of an aqueous base solution, for example, 0.1 to 5 wt%, preferably 2 to 3 wt% aqueous solution of tetramethylammonium hydroxide (TMAH) preferably for 0.1 to 3 minutes, more preferably 0.5 to 2 minutes by conventional techniques such as dip, puddle and spray techniques. In this way, a desired resist pattern is formed on the substrate.

From the chemically amplified negative resist composition, a pattern with a high resolution and minimal LER can be formed. The resist composition is effectively

applicable to a substrate, specifically a substrate having a surface layer of material to which a resist film is less adherent and which is likely to invite pattern stripping or pattern collapse. Typical is a substrate having sputter deposited on its outermost surface metallic chromium or a chromium compound containing at least one light element selected from oxygen, nitrogen and carbon or a substrate having an outermost surface layer of SiO, SiOₓ, or a tantalum, molybdenum, cobalt, nickel, tungsten or tin compound. The negative resist composition is useful in forming a pattern on such a substrate, typically a photomask blank, which may be either of transmission or reflection type.

The mask blank of transmission type is typically a photomask blank having a light-shielding film of chromium-based material. It may be either a photomask blank for binary masks or a photomask blank for phase shift masks. In the case of the binary mask-forming photomask blank, the light-shielding film may include an antireflection layer of chromium-based material and a light-shielding layer. In one example, the antireflection layer on the surface layer side is entirely composed of a chromium-based material. In an alternative example, only a surface side portion of the antireflection layer on the surface layer side is composed of a chromium-based material and the remaining portion is composed of a silicon compound-based material which may contain a transition metal. In the case of the phase shift mask-forming photomask blank, it may include a phase shift film and a chromium-based light-shielding film thereon.

Photomask blanks having an outermost layer of chromium base material are well known as described in JP-A 2008-026500 and JP-A 2007-302873 and the references cited therein. Although the detail description is omitted herein, the following layer construction may be employed when a light-shielding film including an antireflective layer and a light-shielding layer is composed of chromium base materials.

In the example where a light-shielding film including an antireflective layer and a light-shielding layer is composed of chromium base materials, layers may be stacked in the order of an antireflective layer and a light-shielding layer from the outer surface side, or layers may be stacked in the order of an antireflective layer, a light-shielding layer, and an antireflective layer from the outer surface side. Each of the antireflective layer and the light-shielding layer may be composed of multiple sub-layers. When the sub-layers have different compositions, the composition may be graded discontinuously or continuously from sub-layer to sub-layer. The chromium base material used herein may be metallic chromium or a material consisting of metallic chromium and a light element such as oxygen, nitrogen or carbon. Examples used herein include metallic chromium, chromium oxide, chromium nitride, chromium carbide, chromium oxynitride, chromium oxycarbide, chromium nitride carbide, and chromium oxide nitride carbide.

The mask blank of reflection type includes a substrate, a multilayer reflective film formed on one major surface (front surface) of the substrate, for example, a multilayer reflective film of reflecting exposure radiation such as EUV radiation, and an absorber film formed on the multilayer reflective film, for example, an absorber film of absorbing exposure radiation such as EUV radiation to reduce reflectivity. From the reflection type mask blank (reflection type mask blank for EUV lithography), a reflection type mask (reflection type mask for EUV lithography) having an absorber pattern (patterned absorber film) formed by patterning the absorber film is produced. The EUV radiation used in the EUV lithography has a wavelength of 13 to 14 nm, typically about 13.5 nm.

The multilayer reflective film is preferably formed contiguous to one major surface of a substrate. An underlay film may be disposed between the substrate and the multilayer reflective film as long as the benefits of the invention are not lost. The absorber film may be formed contiguous to the multilayer reflective film while a protective film (protective film for the multilayer reflective film) may be disposed between the multilayer reflective film and the absorber film, preferably contiguous to the multilayer reflective film, more preferably contiguous to the multilayer reflective film and the absorber film. The protective film is used for protecting the multilayer reflective film in a cleaning, tailoring or otherwise processing step. Also preferably, the protective film has an additional function of protecting the multilayer reflective film or preventing the multilayer reflective film from oxidation during the step of patterning the absorber film by etching. Besides, an electroconductive film, which is used for electrostatic chucking of the reflection type mask to an exposure tool, may be disposed below the other major surface (back side surface) which is opposed to the one major surface of the substrate, preferably contiguous to the other major surface. It is provided herein that a substrate has one major surface which is a front or upper side surface and another major surface which is a back or lower side surface. The terms "front and back" sides or "upper and lower" sides are used for the sake of convenience. One or another major surface may be either of the two major surfaces (film-bearing surfaces) of a substrate, and in this sense, front and back or upper and lower are exchangeable. Specifically, the multilayer reflective film may be formed by any of the methods of JP-A 2021-139970 and the references cited therein.

The resist pattern forming process is successful in forming patterns of rectangular profile having a high resolution, reduced LER, improved fidelity, and improved dose margin even on a substrate (typically mask blank of transmission or reflection type) whose outermost surface is made of a material tending to affect resist pattern profile such as a chromium, silicon or tantalum-containing material.

### EXAMPLES

Examples of the invention are given below by way of illustration and not by way of limitation. All parts (pbw) are by weight. For copolymers, the compositional ratio is a molar ratio and Mw is determined by GPC versus polystyrene standards using DMF solvent. IR spectroscopy was measured by NICOLET 6700 (Thermo Fisher Scientific Inc.) and ¹H-NMR spectroscopy by ECA-500 (JEOL Ltd.).

### [1] Synthesis of monomers

### Example 1-1

### Synthesis of Monomer A-1

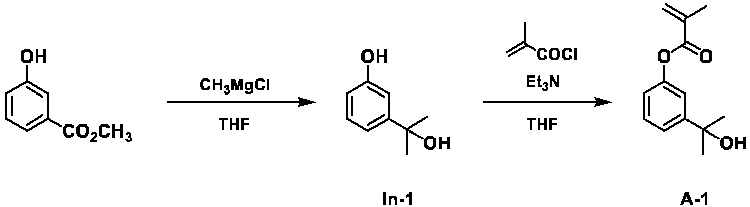

### (1) Synthesis of Intermediate In-1

A reactor under nitrogen atmosphere was charged with methylmagnesium chloride (3.0M, THF solution, 1,200 mL) and THF (1,200 mL). The reactor was heated until the internal temperature reached 50°C. A solution of methyl 3-hydroxybenzoate (152.2 g) in THF (400 mL) was added dropwise to the reactor while the internal temperature of the reactor was maintained below 60°C. Thereafter, the solution was aged for 3 hours while keeping the reactor temperature at 60°C. After aging, the reaction solution was cooled in an ice bath and a solution of ammonium chloride (360 g), 20 wt% hydrochloric acid (360 g), and water (1,800 g) was added dropwise to the reaction solution to quench the reaction. Ethyl acetate (2,000 mL) was added to the reaction solution for extracting the desired compound. This was followed by standard aqueous workup, solvent distillation, and recrystallization from hexane, obtaining Intermediate In-1 as white crystals (amount 149.1 g, yield 98%).

### (2) Synthesis of Monomer A-1

In a reactor under nitrogen atmosphere, Intermediate In-1 (66.5 g) and triethylamine (66.3 g) were dissolved in THF (200 mL). Then methacrylic chloride (50.2 g) was added dropwise to the reactor while the internal temperature of the reactor was maintained below 30°C. Thereafter, the solution was aged at room temperature for 2 hours. After aging, the reaction solution was cooled in an ice bath and a saturated aqueous solution of sodium bicarbonate (270 mL) was added dropwise to quench the reaction. Ethyl acetate (270 mL) was added to the reaction solution for extracting the desired compound. This was followed by standard aqueous workup, solvent distillation, and purification by silica gel column chromatography, obtaining Monomer A-1 as colorless oily matter (amount 85.4 g, yield 89%).

Monomer A-1 was analyzed by IR and ¹H-NMR spectroscopy.
IR (D-ATR): v = 3435, 2976, 2929, 1734, 1637, 1609, 1587, 1485, 1433, 1402, 1378, 1365, 1323, 1297, 1256, 1187, 1166, 1131, 1002, 951, 912, 889, 834, 808, 785, 699, 642, 562, 478 cm⁻¹
¹H-NMR (600 MHz in DMSO-d₆):
   δ = 7.33 (2H, m), 7.22 (1H, m), 6.98 (1H, m), 6.26 (1H, d), 5.88 (1H, d), 5.09 (1H, s), 2.00 (3H, s), 1.41 (6H, s) ppm

### Examples 1-2 to 1-9

### Synthesis of Monomers A-2 to A-9

Monomers A-2 to A-9 shown below were similarly synthesized using the corresponding reactants.

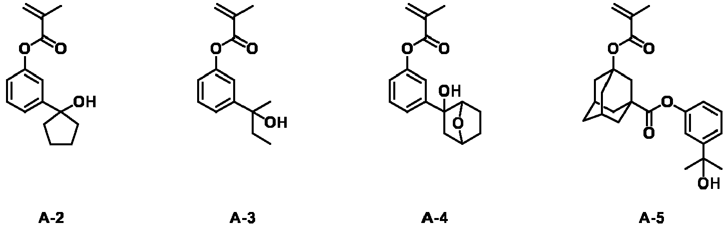

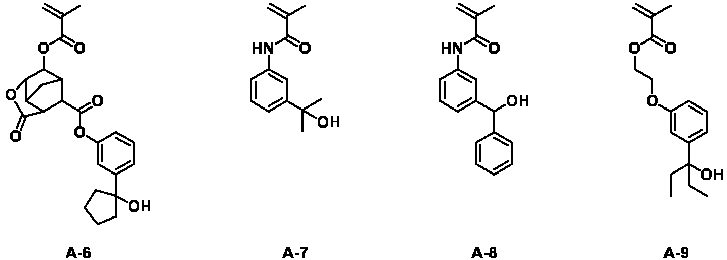

### Comparative Examples 1-1 to 1-7

### Synthesis of Comparative Monomers AX-1 to AX-7

Comparative Monomers AX-1 to AX-7 were synthesized as a comparative monomer for unit A, using the corresponding reactants.

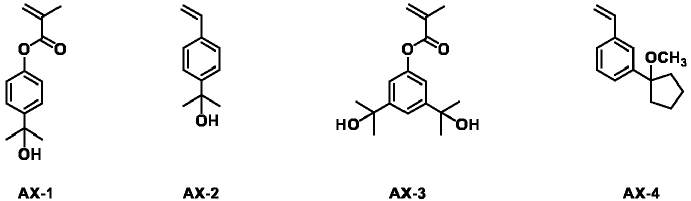

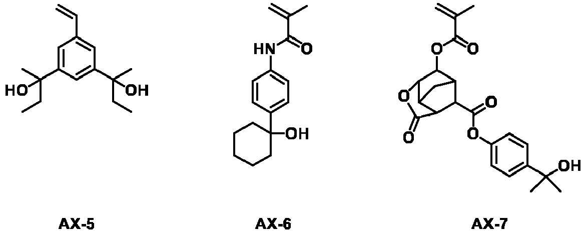

### [2] Synthesis of polymers

A variety of monomers including Monomers A-1 to A-9 and Comparative Monomers AX-1 to AX-7 were used in the synthesis of polymers. The other monomers used herein are shown below.

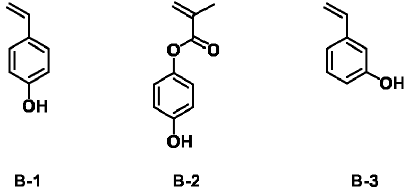

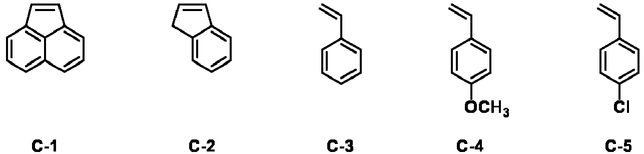

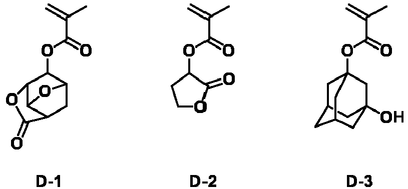

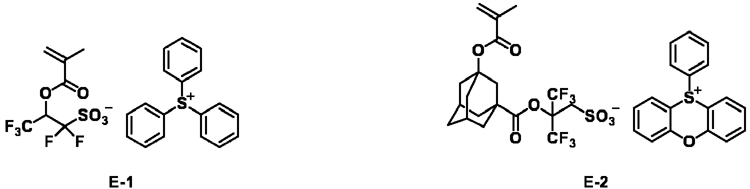

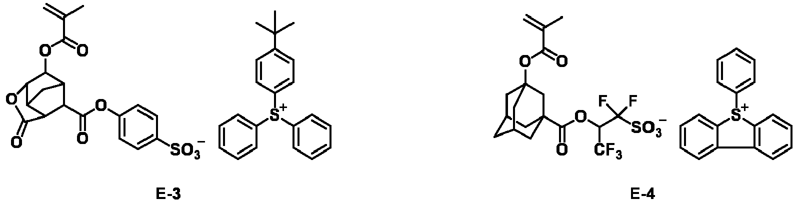

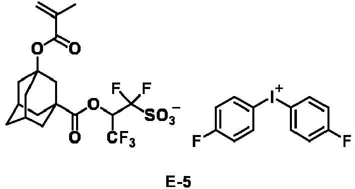

### Example 2-1

### Synthesis of Polymer P-1

A 200-mL dropping funnel under nitrogen blanket was charged with 24.8 g of 4-acetoxystyrene, 7.8 g of acenaphthalene, 67.4 g of 3-(1-hydroxy-1-methylethyl)phenyl 2-methyl-2-propionate, 9.8 g of dimethyl 2,2'-azobis(2-methylpropionate) (trade name V601, FUJIFILM Wako Pure Chemical Corp.), and 112 g of methyl ethyl ketone (MEK) solvent to form a solution A. A 500-mL flask under nitrogen blanket was charged with 38 g of MEK, which was heated at 80°C. While the temperature was maintained, solution A was added dropwise to the flask over 4 hours. At the end of dropwise addition, the solution was continuously stirred for 18 hours while the temperature of 80°C was maintained during polymerization. The polymerization solution was cooled to room temperature and added dropwise to 1,400 g of hexane, whereupon a solid precipitated. The solid precipitate was filtered and washed twice with 280 g of hexane, after which it was dissolved in 400 g of THF in a 1-L flask under nitrogen blanket. 29.4 g of ethanol amine was added to the solution, which was stirred at 60°C for 3 hours. The reaction solution was concentrated under reduced pressure. The concentrate was dissolved in a mixture of 300 g of ethyl acetate and 90 g of water. The solution was transferred to a separatory funnel, to which 29 g of acetic acid was added, followed by separatory operation. After the lower layer was removed, 90 g of water and 39 g of pyridine were added to the organic layer, followed by separatory operation again. After the lower layer was removed, 90 g of water was added to the organic layer, followed by water washing and separatory operation. The water washing and separatory operation was repeated 5 times in total. The organic layer after separation was concentrated and dissolved in 150 g of acetone. The acetone solution was added dropwise to 3,000 g of water whereupon crystals precipitated. The precipitate was filtered, washed with water, and suction filtered over 2 hours. The filter cake was dissolved in 150 g of acetone again. The acetone solution was added dropwise to 3,000 g of water whereupon crystals precipitated. The precipitate was filtered, washed with water and dried, obtaining 58.0 g of the target polymer P-1 as white solid.

Polymer P-1 was analyzed by ¹³C-NMR and ¹H-NMR spectroscopy to determine a compositional ratio of repeat units and by GPC to determine Mw and Mw/Mn, with the results shown below.

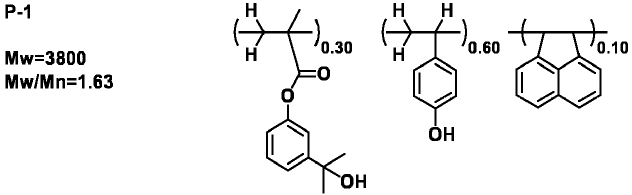

### Examples 2-2 to 2-31 and Comparative Examples 2-1 to 2-20

### Synthesis of Polymers P-2 to P-31 and Comparative Polymers CP-1 to CP-20

Polymers P-2 to P-31 and Comparative Polymers CP-1 to CP-20 shown in Tables 1 to 3 were synthesized by the same procedure as in Example 2-1 except that the type and amount of monomers were changed.

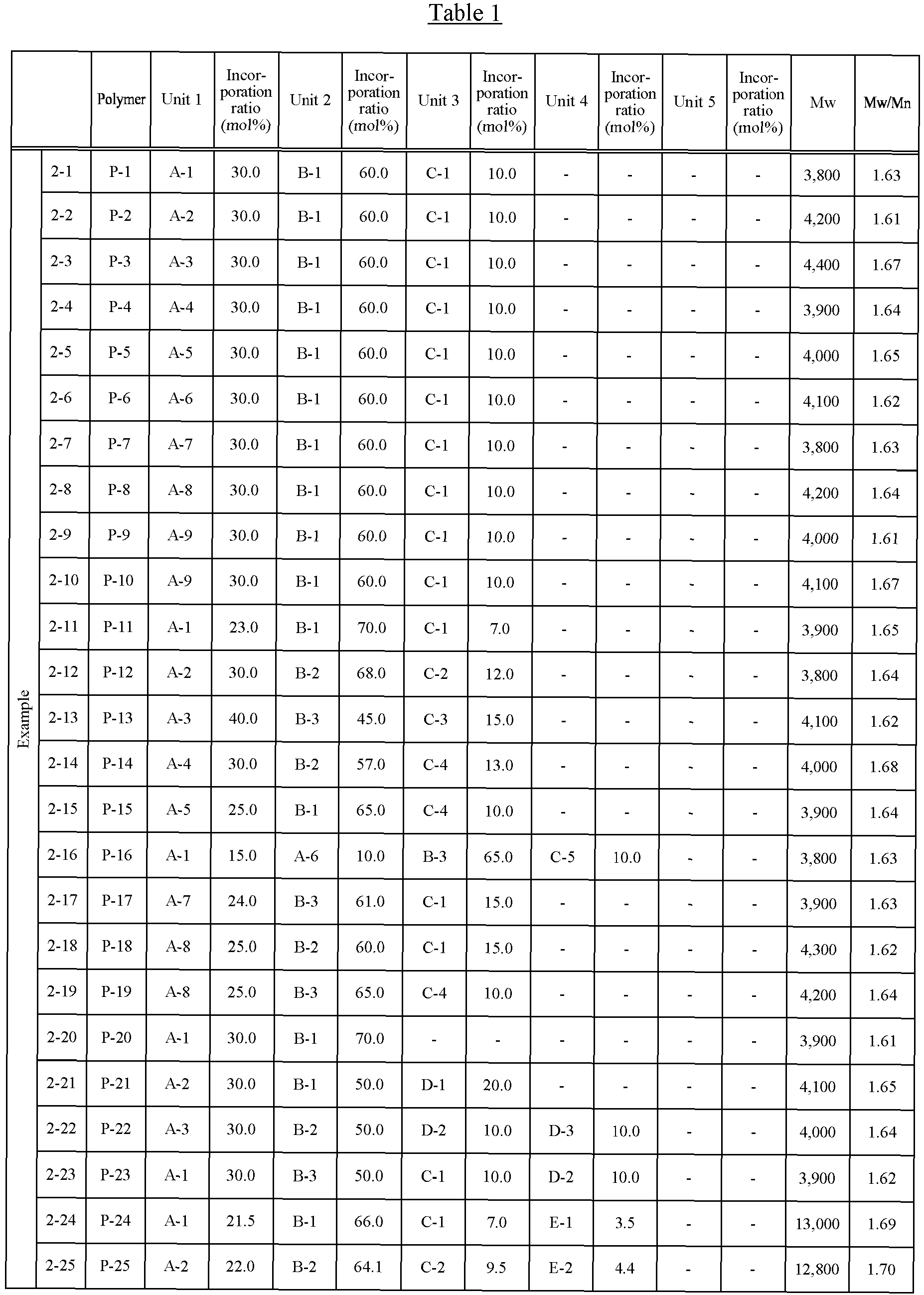

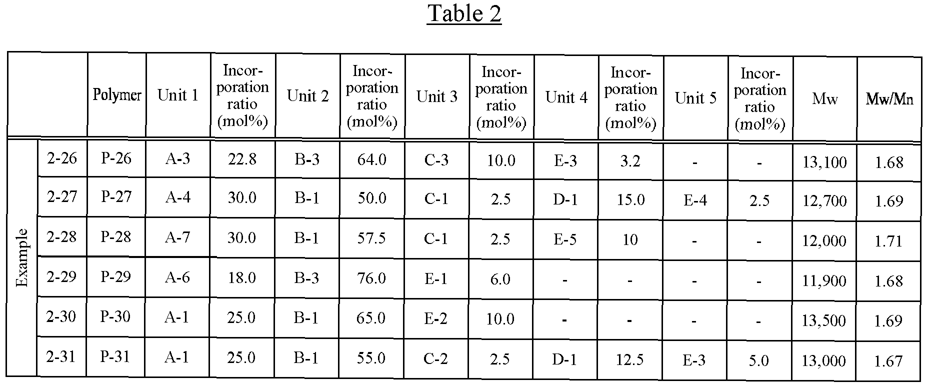

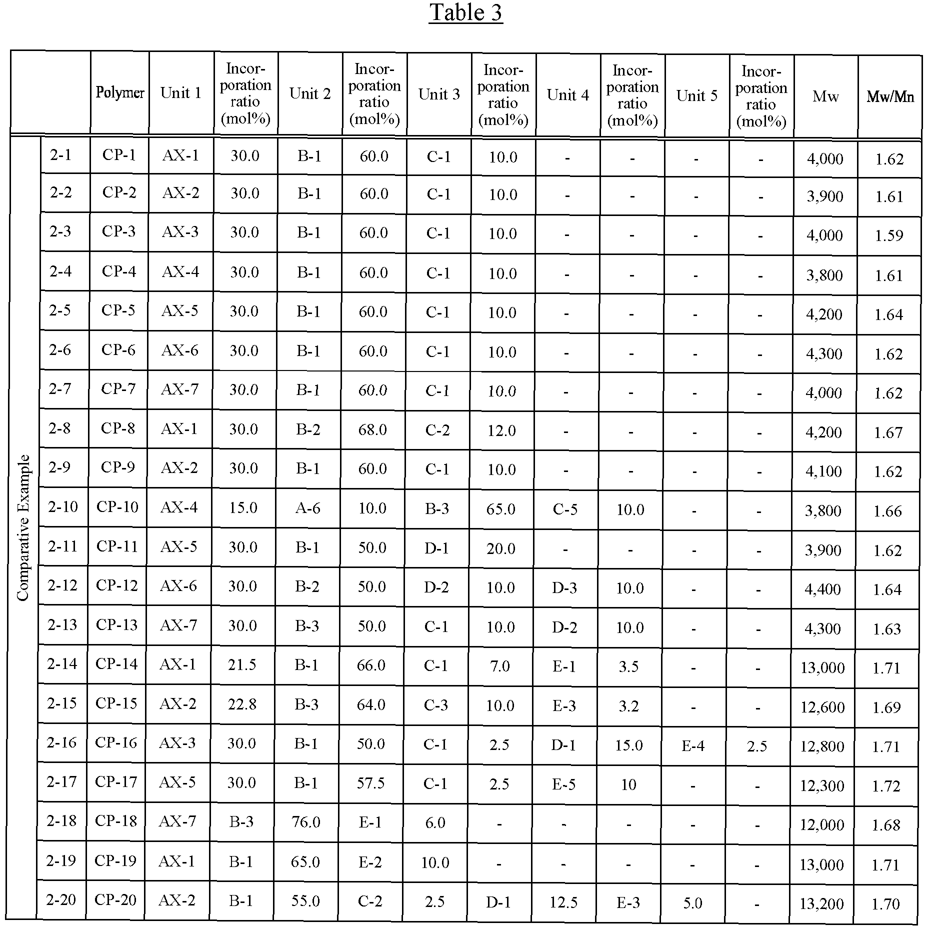

### [3] Preparation of chemically amplified negative resist compositions

### Examples 3-1 to 3-40 and Comparative Examples 3-1 to 3-25

A chemically amplified negative resist composition (R-1 to R-40, CR-1 to CR-25) in solution form was prepared by dissolving selected components in an organic solvent in accordance with the recipe shown in Tables 4 to 6, and filtering the solution through a UPE filter and/or nylon filter with a pore size of 10 nm, 5 nm, 3 nm or 1 nm. The organic solvent was a mixture of 790 pbw of PGMEA, 1,580 pbw of EL, and 1,580 pbw of PGME. Some resist compositions contained a fluorinated polymer (Polymers FP-1 to FP-5) as additive, tetramethoxymethyl glycoluril (TMGU) as crosslinker, and PF-636 (Omnova Solutions, Inc.) as surfactant.

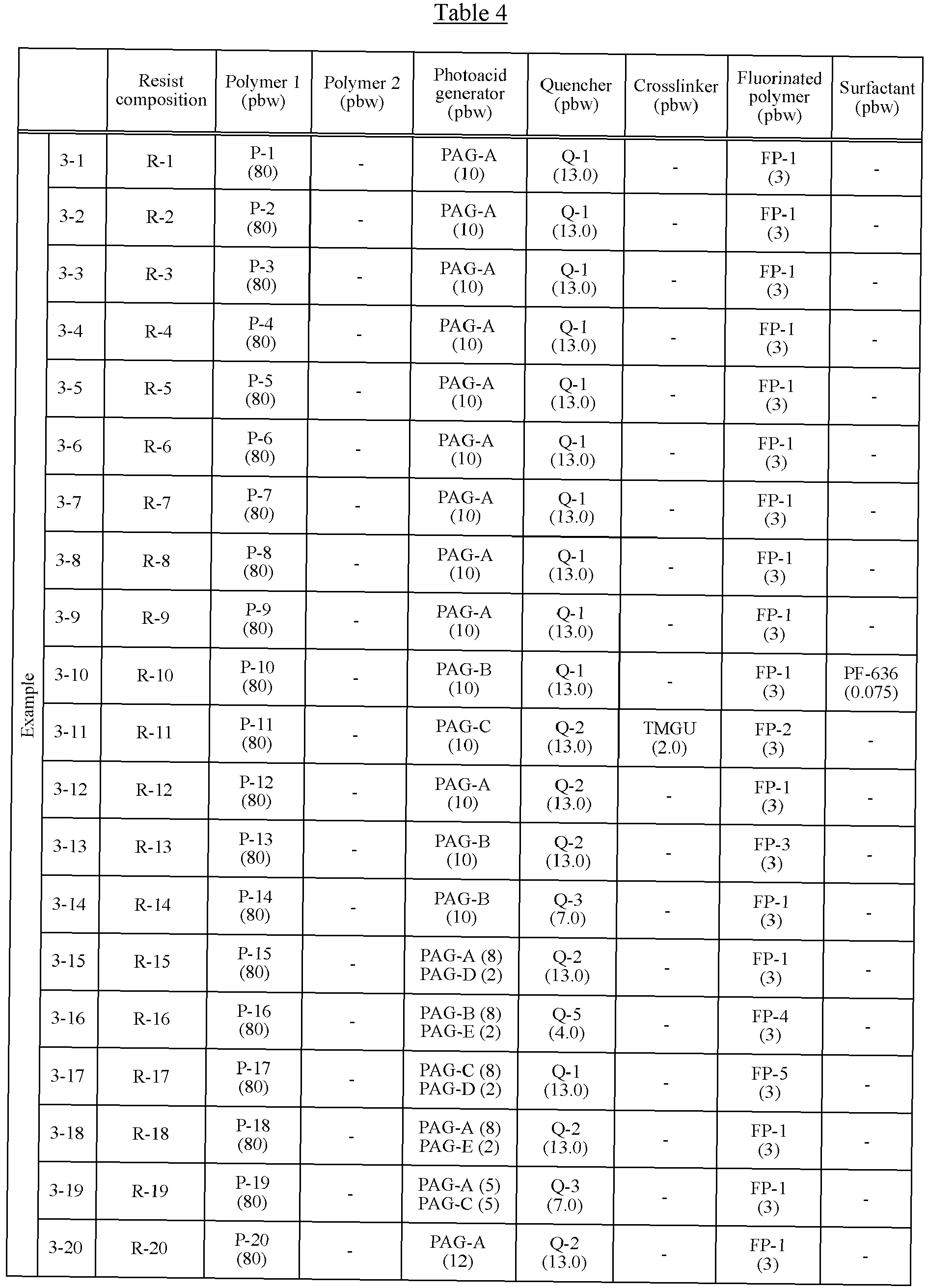

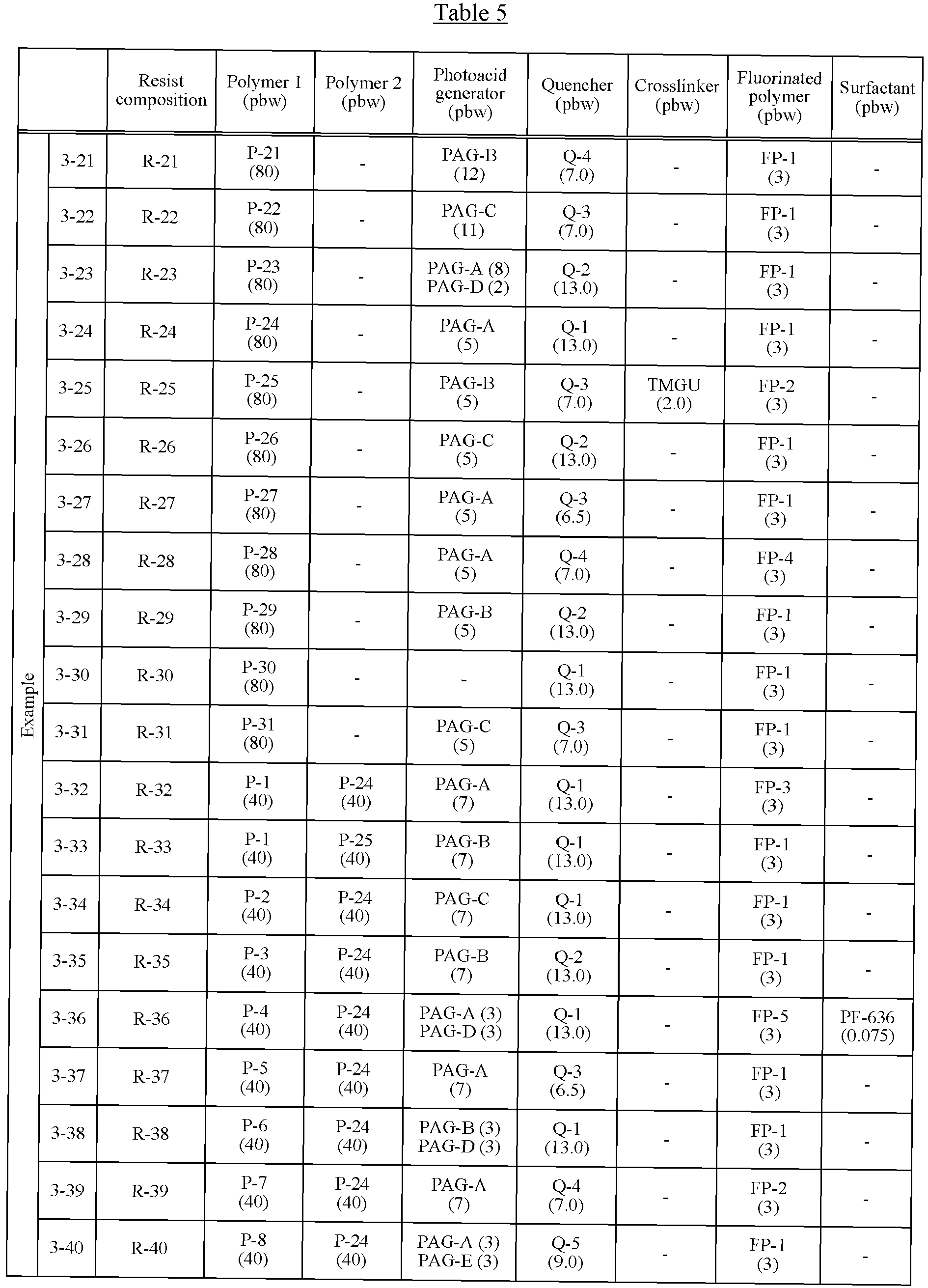

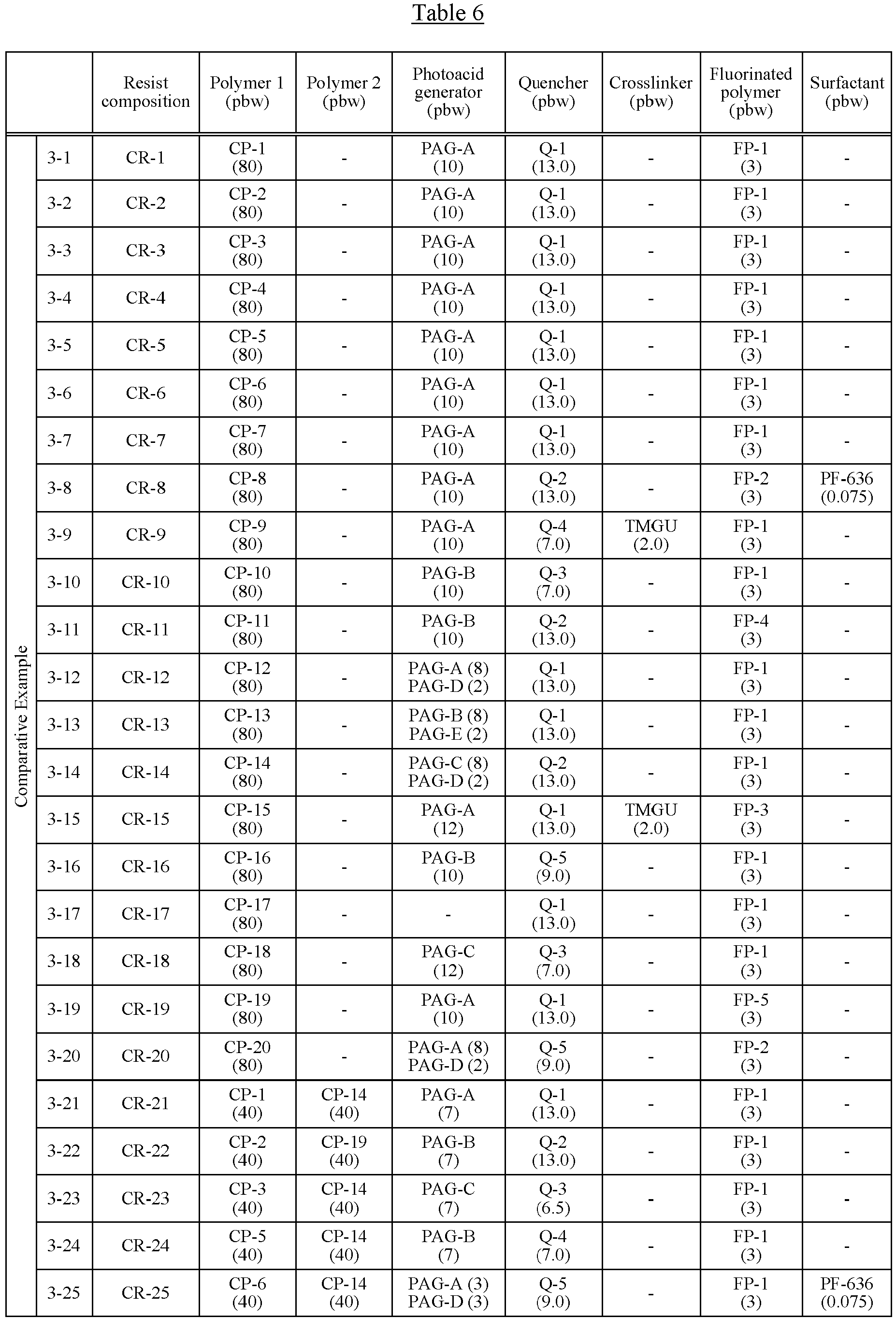

The photoacid generators PAG-A to PAG-E, quenchers Q-1 to Q-5, and fluorinated polymers FP-1 to FP-5 in the resist compositions are identified below.

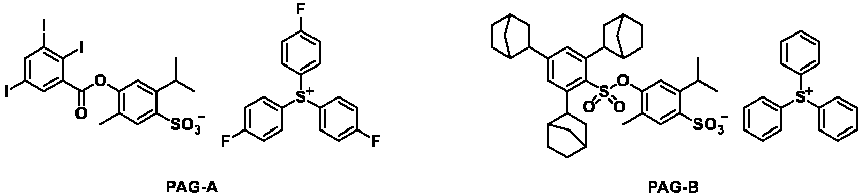

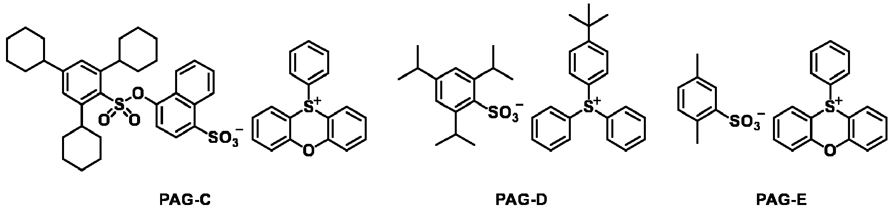

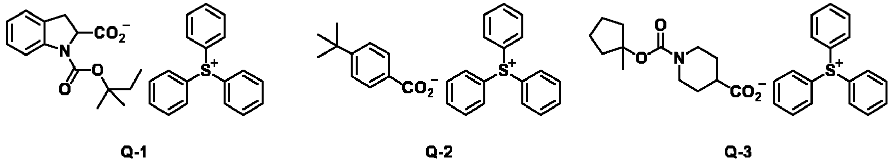

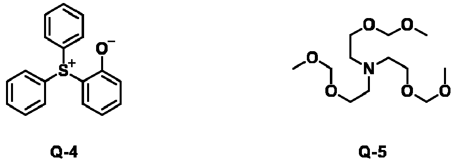

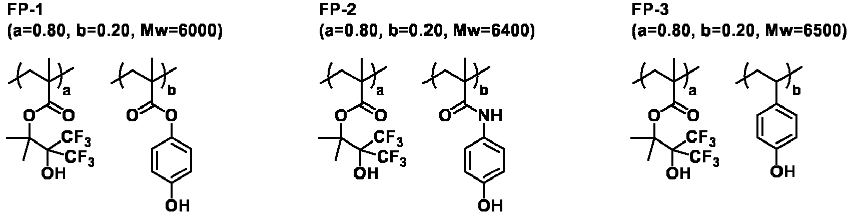

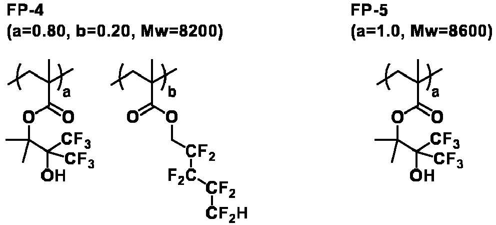

### [4] EB lithography test

### Examples 4-1 to 4-40 and Comparative Examples 4-1 to 4-25

Using a coater/developer system ACT-M (Tokyo Electron Ltd.), each of the negative resist compositions (R-1 to R-40 and CR-1 to CR-25) was spin coated onto a photomask blank of 152 mm squares, which had a silicon oxide film at the outermost surface and had been vapor primed with hexamethyldisilazane, and prebaked on a hot plate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

The coated mask blanks were exposed to electron beam using an EB writer system EBM-5000Plus (NuFlare Technology Inc., accelerating voltage 50 kV), then baked (PEB) at 120°C for 600 seconds, and developed in a 2.38 wt% TMAH aqueous solution to form negative patterns.

The patterned mask blank was observed under a top-down scanning electron microscope (TD-SEM). The optimum exposure (Eop) was defined as the exposure dose (µC/cm²) which provided a 1:1 resolution at the top and bottom of a 200-nm 1:1 line-and-space (LS) pattern. The maximum resolution of the resist was defined as the minimum line width of a LS pattern that could be resolved at the optimum exposure. For each of the edges of 32 lines of the 200-nm LS pattern, edge detection was carried out at 80 points, from which a 3-fold value (3σ) of the standard deviation (σ) or variation was determined and reported as LER (nm). It was judged by visual observation whether or not the pattern profile was rectangular. For the evaluation of pattern fidelity, when a square dot pattern of size 120 nm and density 36% was placed, an area loss (%) at one corner of the square dot was computed. A smaller value indicates that the dot profile is more rectangular. The results are shown in Tables 7 and 8.

**Table 7**

| | | Resist composition | Eop (µC/cm²) | Maximum resolution (nm) | LER (nm) | Pattern profile | Area loss (%) |
|---|---|---|---|---|---|---|---|
| | 4-1 | R-1 | 100 | 35 | 4.3 | rectangular | 8 |
| | 4-2 | R-2 | 95 | 35 | 4.3 | rectangular | 8 |
| | 4-3 | R-3 | 105 | 36 | 4.4 | rectangular | 8 |
| | 4-4 | R-4 | 100 | 35 | 4.2 | rectangular | 8 |
| | 4-5 | R-5 | 105 | 35 | 4.4 | rectangular | 8 |
| | 4-6 | R-6 | 100 | 34 | 4.5 | rectangular | 8 |
| | 4-7 | R-7 | 110 | 35 | 4.3 | rectangular | 8 |
| | 4-8 | R-8 | 100 | 35 | 4.3 | rectangular | 9 |
| | 4-9 | R-9 | 100 | 36 | 4.3 | rectangular | 8 |
| | 4-10 | R-10 | 105 | 35 | 4.4 | rectangular | 8 |
| | 4-11 | R-11 | 95 | 35 | 4.5 | rectangular | 8 |
| | 4-12 | R-12 | 100 | 35 | 4.2 | rectangular | 8 |
| | 4-13 | R-13 | 110 | 36 | 4.6 | rectangular | 8 |
| | 4-14 | R-14 | 100 | 35 | 4.4 | rectangular | 8 |
| | 4-15 | R-15 | 105 | 35 | 4.7 | rectangular | 8 |
| | 4-16 | R-16 | 100 | 35 | 4.5 | rectangular | 8 |
| | 4-17 | R-17 | 105 | 36 | 4.4 | rectangular | 8 |
| | 4-18 | R-18 | 100 | 35 | 4.2 | rectangular | 9 |
| | 4-19 | R-19 | 100 | 35 | 4.5 | rectangular | 8 |
| Example | 4-20 | R-20 | 105 | 35 | 4.5 | rectangular | 8 |
| | 4-21 | R-21 | 100 | 36 | 4.3 | rectangular | 8 |
| | 4-22 | R-22 | 100 | 35 | 4.3 | rectangular | 8 |
| | 4-23 | R-23 | 105 | 35 | 4.6 | rectangular | 9 |
| | 4-24 | R-24 | 100 | 35 | 4.3 | rectangular | 8 |
| | 4-25 | R-25 | 110 | 35 | 4.2 | rectangular | 8 |
| | 4-26 | R-26 | 100 | 36 | 4.4 | rectangular | 8 |
| | 4-27 | R-27 | 95 | 35 | 4.3 | rectangular | 9 |
| | 4-28 | R-28 | 100 | 35 | 4.3 | rectangular | 8 |
| | 4-29 | R-29 | 110 | 35 | 4.4 | rectangular | 8 |
| | 4-30 | R-30 | 100 | 35 | 4.3 | rectangular | 8 |
| | 4-31 | R-31 | 95 | 34 | 4.3 | rectangular | 8 |
| | 4-32 | R-32 | 100 | 35 | 4.4 | rectangular | 8 |
| | 4-33 | R-33 | 100 | 35 | 4.2 | rectangular | 8 |
| | 4-34 | R-34 | 95 | 36 | 4.5 | rectangular | 9 |
| | 4-35 | R-35 | 95 | 35 | 4.2 | rectangular | 8 |
| | 4-36 | R-36 | 100 | 35 | 4.5 | rectangular | 8 |
| | 4-37 | R-37 | 105 | 36 | 4.3 | rectangular | 8 |
| | 4-38 | R-38 | 110 | 35 | 4.4 | rectangular | 8 |
| | 4-39 | R-39 | 95 | 35 | 4.5 | rectangular | 9 |
| | 4-40 | R-40 | 100 | 34 | 4.3 | rectangular | 8 |

**Table 8**

| | | Resist composition | Eop (µC/cm²) | Maximum resolution (nm) | LER (nm) | Pattern profile | Area loss (%) |
|---|---|---|---|---|---|---|---|
| | 4-1 | CR-1 | 100 | 42 | 5.2 | rectangular | 13 |
| | 4-2 | CR-2 | 95 | 40 | 5.3 | footing | 14 |
| | 4-3 | CR-3 | 105 | 44 | 5.4 | footing | 15 |
| | 4-4 | CR-4 | 100 | 42 | 5.5 | footing | 14 |
| | 4-5 | CR-5 | 105 | 44 | 5.2 | footing | 14 |
| | 4-6 | CR-6 | 100 | 44 | 5.4 | rectangular | 15 |
| | 4-7 | CR-7 | 110 | 44 | 5.3 | footing | 14 |
| | 4-8 | CR-8 | 100 | 40 | 5 | footing | 13 |
| | 4-9 | CR-9 | 100 | 42 | 5.2 | footing | 15 |
| | 4-10 | CR-10 | 95 | 40 | 5.1 | footing | 14 |
| | 4-11 | CR-11 | 59 | 42 | 4.9 | footing | 13 |
| | 4-12 | CR-12 | 100 | 44 | 5 | footing | 14 |
| Comparative Example | 4-13 | CR-13 | 105 | 46 | 5.1 | footing | 14 |
| | 4-14 | CR-14 | 110 | 42 | 5.2 | rectangular | 15 |
| | 4-15 | CR-15 | 105 | 42 | 4.9 | footing | 14 |
| | 4-16 | CR-16 | 110 | 44 | 4.8 | footing | 14 |
| | 4-17 | CR-17 | 100 | 48 | 5 | footing | 14 |
| | 4-18 | CR-18 | 95 | 46 | 5.1 | footing | 13 |
| | 4-19 | CR-19 | 100 | 44 | 5.3 | footing | 14 |
| | 4-20 | CR-20 | 100 | 42 | 5.5 | footing | 15 |
| | 4-21 | CR-21 | 95 | 40 | 5.3 | footing | 14 |
| | 4-22 | CR-22 | 105 | 48 | 5.2 | footing | 14 |
| | 4-23 | CR-23 | 100 | 44 | 5.4 | footing | 13 |
| | 4-24 | CR-24 | 100 | 42 | 5.1 | footing | 14 |
| | 4-25 | CR-25 | 95 | 42 | 5.2 | footing | 14 |

As demonstrated above, the chemically amplified negative resist compositions within the scope of the invention exhibit a very high resolution and form patterns with satisfactory LER, profile and fidelity. The resist pattern forming process using the negative resist composition is useful in photolithography for the fabrication of semiconductor devices and the processing of photomask blanks of transmission or reflection type.

## Claims

1. A monomer having the formula (A1): 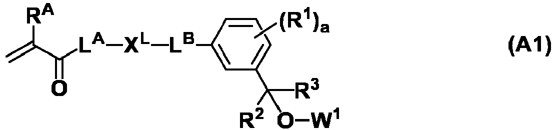 wherein a is an integer of 0 to 4,
R^{A} is hydrogen, fluorine, methyl or trifluoromethyl,
L^{A} is -O- or -NH-,
L^{B} is a single bond, ether bond, ester bond or amide bond,
X^{L} is a single bond or a C₁-C₄₀ hydrocarbylene group which may contain a heteroatom,
R¹ is halogen or a C₁-C₂₀ hydrocarbyl group which may contain a heteroatom,
R² and R³ are each independently hydrogen, a C₁-C₁₅ saturated hydrocarbyl group or C₆-C₁₅ aryl group, the hydrocarbyl group may be substituted with a hydroxy moiety or C₁-C₆ saturated hydrocarbyloxy moiety, the aryl group may have a substituent, with the proviso that R² and R³ are not hydrogen at the same time, and R² and R³ may bond together to form a ring with the carbon atom to which they are attached, some constituent -CH₂- in the ring may be replaced by -O- or -S-, and
W¹ is hydrogen, a C₁-C₁₀ aliphatic hydrocarbyl group, C₂-C₁₀ aliphatic hydrocarbylcarbonyl group, or C₆-C₁₅ aryl group, the aryl group may have a substituent.

2. A polymer comprising repeat units derived from the monomer of claim 1.

3. The polymer of claim 2, further comprising repeat units having the formula (A2): 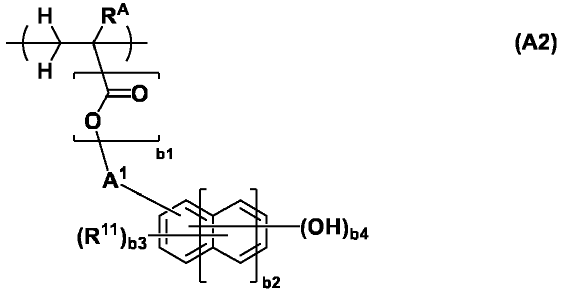 wherein b1 is 0 or 1, b2 is an integer of 0 to 2, b3 is an integer satisfying 0 ≤ b3 ≤ 5+2(b2)-b4, b4 is an integer of 1 to 3,
R^{A} is hydrogen, fluorine, methyl or trifluoromethyl,
R¹¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, and
A¹ is a single bond or C₁-C₁₀ saturated hydrocarbylene group in which some constituent -CH₂- may be replaced by -O-.

4. The polymer of claim 2 or 3, further comprising repeat units of at least one type selected from repeat units having the formula (A3), repeat units having the formula (A4), and repeat units having the formula (A5): 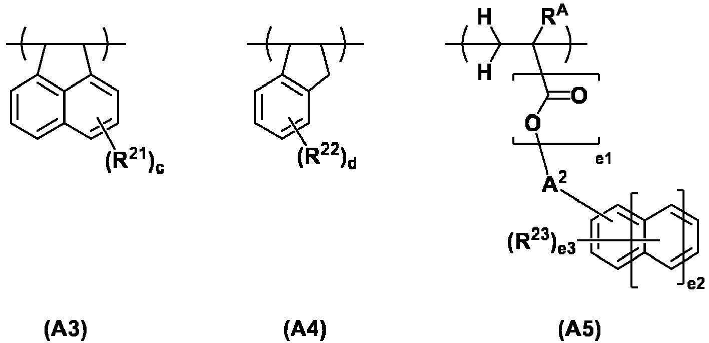 wherein c and d are each independently an integer of 0 to 4, e1 is 0 or 1, e2 is an integer of 0 to 2, e3 is an integer of 0 to 5,
R^{A} is hydrogen, fluorine, methyl or trifluoromethyl,
R²¹ and R²² are each independently hydroxy, halogen, an optionally halogenated C₁-C₈ saturated hydrocarbyl group, optionally halogenated C₁-C₈ saturated hydrocarbyloxy group, or optionally halogenated C₂-C₈ saturated hydrocarbylcarbonyloxy group,
R²³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro, cyano, C₁-C₂₀ saturated hydrocarbyl sulfinyl group, or C₁-C₂₀ saturated hydrocarbyl sulfonyl group,
A² is a single bond or C₁-C₁₀ saturated hydrocarbylene group in which some constituent -CH₂- may be replaced by -O-.

5. The polymer of any one of claims 2 to 4, further comprising repeat units of at least one type selected from repeat units having the formulae (A6) to (A13): 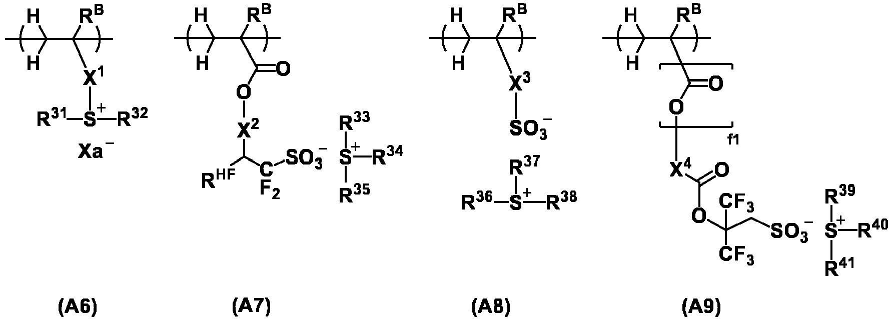 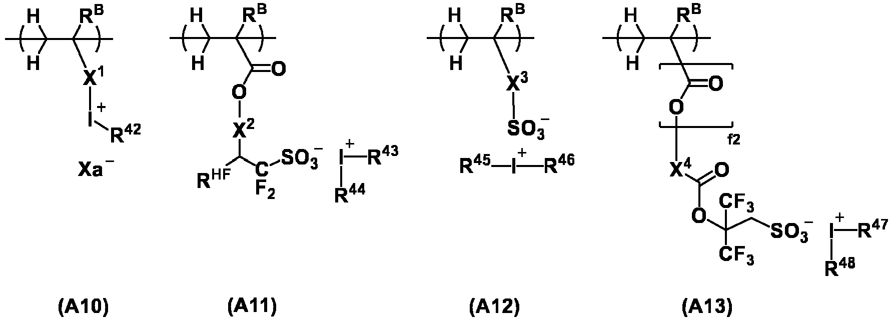 wherein R^{B} is hydrogen or methyl,
X¹ is each independently a single bond, C₁-C₆ aliphatic hydrocarbylene group, phenylene, naphthylene, or C₇-C₁₈ group obtained by combining the foregoing, -O-X¹¹-, -C(=O)-O-X¹¹-, or -C(=O)-NH-X¹¹-, X¹¹ is a C₁-C₆ aliphatic hydrocarbylene group, phenylene, naphthylene, or C₇-C₁₈ group obtained by combining the foregoing, which may contain a carbonyl moiety, ester bond, ether bond or hydroxy moiety,
X² is each independently a single bond or -X²¹-C(=O)-O-, X²¹ is a C₁-C₂₀ hydrocarbylene group which may contain a heteroatom,
X³ is each independently a single bond, methylene, ethylene, phenylene, fluorinated phenylene, trifluoromethyl-substituted phenylene, -O-X³¹-, -C(=O)-O-X³¹-, or -C(=O)-NH-X³¹-, X³¹ is a C₁-C₆ aliphatic hydrocarbylene group, phenylene, fluorinated phenylene, trifluoromethyl-substituted phenylene, or C₇-C₂₀ group obtained by combining the foregoing, which may contain a carbonyl moiety, ester bond, ether bond or hydroxy moiety,
X⁴ is each independently a single bond or C₁-C₃₀ hydrocarbylene group which may contain a heteroatom,
f1 and f2 are each independently 0 or 1, f1 and f2 are 0 when X⁴ is a single bond,
R³¹ to R⁴⁸ are each independently halogen, or C₁-C₂₀ hydrocarbyl group which may contain a heteroatom, R³¹ and R³² may bond together to form a ring with the sulfur atom to which they are attached, R³³ and R³⁴, R³⁶ and R³⁷, or R³⁹ and R⁴⁰ may bond together to form a ring with the sulfur atom to which they are attached,
R^{HF} is hydrogen or trifluoromethyl, and
Xa⁻ is a non-nucleophilic counter ion.

6. A chemically amplified negative resist composition comprising (A) a base polymer containing the polymer of any one of claims 2 to 5.

7. The negative resist composition of claim 6 wherein repeat units having an aromatic skeleton account for at least 60 mol% of the overall repeat units of the polymer in the base polymer.

8. The negative resist composition of claim 6 or 7, further comprising (B) an acid generator.

9. The negative resist composition of any one of claims 6 to 8, further comprising (C) a crosslinker.

10. The negative resist composition of any one of claims 6 to 8, which is free of a crosslinker.

11. The negative resist composition of any one of claims 6 to 10, further comprising (D) a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6): 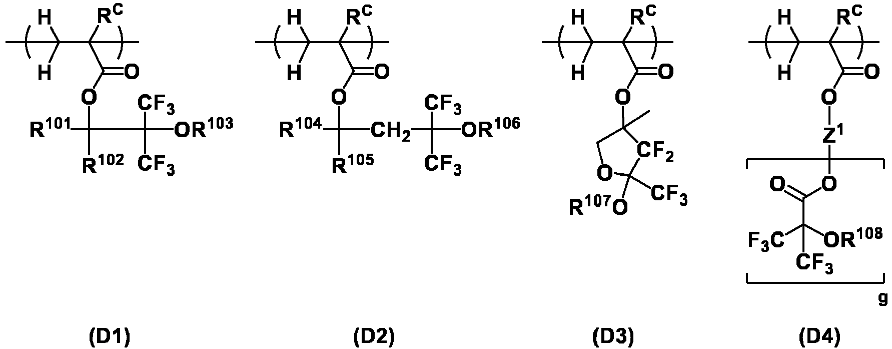 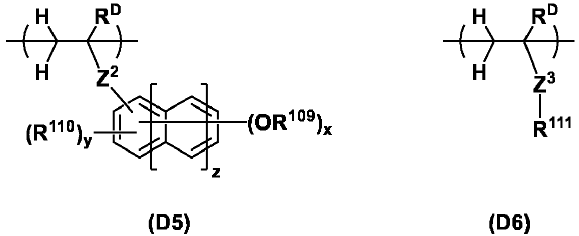 wherein x is an integer of 1 to 3, y is an integer satisfying 0 ≤ y ≤ 5+2z-x, z is 0 or 1, g is an integer of 1 to 3,
R^{C} is each independently hydrogen, fluorine, methyl or trifluoromethyl,
R^{D} is each independently hydrogen or methyl,
R¹⁰¹, R¹⁰², R¹⁰⁴ and R¹⁰⁵ are each independently hydrogen or a C₁-C₁₀ saturated hydrocarbyl group,
R¹⁰³, R¹⁰⁶, R¹⁰⁷ and R¹⁰⁸ are each independently hydrogen, a C₁-C₁₅ hydrocarbyl group, C₁-C₁₅ fluorinated hydrocarbyl group, or acid labile group, and when R¹⁰³, R¹⁰⁶, R¹⁰⁷ and R¹⁰⁸ each are a hydrocarbyl or fluorinated hydrocarbyl group, an ether bond or carbonyl moiety may intervene in a carbon-carbon bond,
R¹⁰⁹ is hydrogen or a C₁-C₅ straight or branched hydrocarbyl group in which a heteroatom-containing moiety may intervene in a carbon-carbon bond,
R¹¹⁰ is a C₁-C₅ straight or branched hydrocarbyl group in which a heteroatom-containing moiety may intervene in a carbon-carbon bond,
R¹¹¹ is a C₁-C₂₀ saturated hydrocarbyl group in which at least one hydrogen is substituted by fluorine, and in which some constituent -CH₂- may be replaced by an ester bond or ether bond,
Z¹ is a C₁-C₂₀ (g+1)-valent hydrocarbon group or C₁-C₂₀ (g+1)-valent fluorinated hydrocarbon group,
Z² is a single bond, *-C(=O)-O- or *-C(=O)-NH-,
Z³ is a single bond, -O-, *-C(=O)-O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.

12. The negative resist composition of any one of claims 6 to 11, further comprising (E) a quencher.

13. The negative resist composition of any one of claims 6 to 12, further comprising (F) an organic solvent.

14. A resist pattern forming process comprising the steps of:
applying the chemically amplified negative resist composition of any one of claims 6 to 13 onto a substrate to form a resist film thereon,
exposing the resist film patternwise to high-energy radiation, and
developing the exposed resist film in an alkaline developer.

15. A mask blank of transmission or reflection type which is coated with the chemically amplified negative resist composition of any one of claims 6 to 13.
